(19) Europäisches Patentamt
European Patent Office
Office européen des brevets

(11) EP 1 571 154 A1

(12) # EUROPEAN PATENT APPLICATION

(43) Date of publication:
07.09.2005 Bulletin 2005/36

(21) Application number: 04004904.1

(22) Date of filing: 03.03.2004

(51) Int Cl.[7]: C07K 5/02, C07D 413/12, C07D 409/12, C07D 295/104, C07D 419/12, A61K 31/381, A61K 31/5377, A61K 31/4965, A61K 31/4178, A61K 31/54

(84) Designated Contracting States:
AT BE BG CH CY CZ DE DK EE ES FI FR GB GR HU IE IT LI LU MC NL PL PT RO SE SI SK TR
Designated Extension States:
AL LT LV MK

(71) Applicant: Aventis Pharma Deutschland GmbH
65929 Frankfurt am Main (DE)

(72) Inventors:
- Urmann, Matthias, Dr.
65760 Eschborn (DE)
- Nazaré, Marc, Dr.
65510 Idstein (DE)
- Wehner, Volkmar, Dr.
97657 Sandberg (DE)
- Matter, Hans, Dr.
63505 Langenselbold (DE)
- Bauer, Armin, Dr.
65843 Sulzbach/Ts. (DE)
- Wagner, Michael, Dr.
64665 Alsbach (DE)

(54) **Beta-aminoacid-derivatives as factor Xa inhibitors**

(57) The present invention relates to compounds of the formula I,

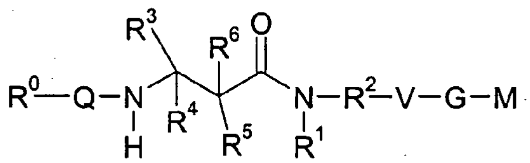

(I)

in which $R^0$ ; $R^1$ ; $R^2$ ; $R^3$ ; $R^4$; $R^5$, $R^6$, Q; V, G and M have the meanings indicated in the claims. The compounds of the formula I are valuable pharmacologically active compounds. They exhibit a strong antithrombotic effect and are suitable, for example, for the therapy and prophylaxis of cardiovascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended. The invention furthermore relates to processes for the preparation of compounds of the formula I, their use, in particular as active ingredients in pharmaceuticals, and pharmaceutical preparations comprising them.

EP 1 571 154 A1

Printed by Jouve, 75001 PARIS (FR)

## Description

The present invention relates to compounds of the formula I,

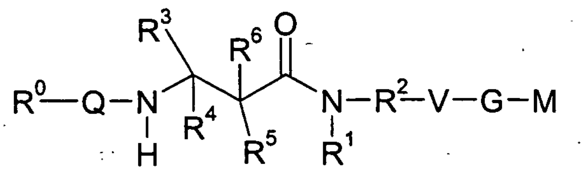

(I)

in which $R^0$ ; $R^1$ ; $R^2$ ; $R^3$ ; $R^4$; $R^5$, $R^6$, Q; V, G and M have the meanings indicated below. The compounds of the formula I are valuable pharmacologically active compounds. They exhibit a strong anti-thrombotic effect and are suitable, for example, for the therapy and prophylaxis of cardio-vascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended. The invention furthermore relates to processes for the preparation of compounds of the formula I, their use, in particular as active ingredients in pharmaceuticals, and pharmaceutical preparations comprising them.

Normal haemeostasis is the result of a complex balance between the processes of clot initiation, formation and clot dissolution. The complex interactions between blood cells, specific plasma proteins and the vascular surface, maintain the fluidity of blood unless injury and blood loss occurs (EP-A-987274). Many significant disease states are related to abnormal haemeostasis. For example, local thrombus formation due to rupture of atheroslerotic plaque is a major cause of acute myocardial infarction and unstable angina. Treatment of an occlusive coronary thrombus by either thrombolytic therapy or percutaneous angioplasty may be accompanied by acute thrombolytic reclosure of the affected vessel.

There continues to be a need for safe and effective therapeutic anticoagulants to limit or prevent thrombus formation. It is most desirable to develop agents that inhibit coagulation without directly inhibiting thrombin but by inhibiting other steps in the coagulation cascade like factor Xa and/or factor VIIa adivity. It is now believed that inhibitors of factor Xa carry a lower bleeding risk than thrombin inhibitors (A. E. P. Adang & J. B. M. Rewinkel, Drugs of the Future 2000, 25, 369-383).

Low molecular weight, factor Xa-specific blood clotting inhibitors that are effective but do not cause unwanted side effects have been described, for example, in WO-A-95/29189.

However, besides being an effective factor Xa-specific blood clotting inhibitor, it is desirable that such inhibitors also have further advantageous properties, for instance stability in plasma and liver and selectivity versus other serine proteases whose inhibition is not intended, such as thrombin. There is an ongoing need for further low molecular weight factor Xa specific blood clotting inhibitors, which are effective and have the above advantages as well.

Specific inhibition of the factor VIIa/tissue factor catalytic complex using monoclonal antibodies (WO-A-92/06711) or a protein such as chloromethyl ketone inactivated factor VIIa (WO-A-96/12800, WO-A-97/47651) is an extremely effective means of controlling thrombus formation caused by acute arterial injury or the thrombotic complications related to bacterial septicemia. There is also experimental evidence suggesting that inhibition of factor VIIa/tissue factor activity inhibits restenosis following balloon angioplasty. Bleeding studies have been conducted in baboons and indicate that inhibition of the factor VIIa/tissue factor complex has the widest safety window with respect to therapeutic effectiveness and bleeding risk of any anticoagulant approach tested including thrombin, platelet and factor Xa inhibition. Certain inhibitors of factor VIIa have already been described. EP-A-987274, for example discloses compounds containing a tripeptide unit, which inhibit factor VIIa. However, the property profile of these compounds is still not ideal, and there is an ongoing need for further low molecular weight factor VIIa inhibitory blood clotting inhibitors

The present invention satisfies the above needs by providing novel compounds of the formula I, which exhibit better factor Xa and/or factor VIIa inhibitory activity and are favorable agents with high bioavailability.

Thus, the present invention relates to compounds of the formula I,

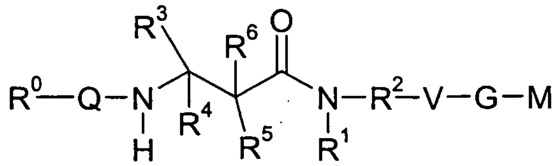

(I)

wherein

$R^0$ is 1) a monocyclic or bicyclic 4- to 15-membered heterocyclyl, selected out of the group benzimidazolyl, 1,3-benzodioxolyl, benzofuranyl, benzoxazolyl, benzothiazolyl, benzothienyl, cinnolinyl, chromanyl, indazolyl, indolyl, imidazolyl, isochromanyl, isoindolyl, isoquinolyl, phthalazinyl, pteridinyl, purinyl, pyridyl, pyrimidinyl, pyridazinyl, pyrazolyl, pyrrolyl, pyrazinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, thiazolyl, thiadiazolyl, thienyl or triazolyl, wherein said heterocyclyl is unsubstituted or mono-, di- or trisubstituted independently of one another by R8, or
2) a monocyclic or bicyclic 4- to 15-membered heterocyclyl, containing one, two, three or four heteroatoms chosen from nitrogen, sulfur or oxygen,
wherein said heterocyclyl is unsubstituted or mono-, di- or trisubstituted independently of one another by R8,
3) a monocyclic or bicyclic 6- to 14-membered aryl, wherein aryl is mono-, di- or trisubstituted independently of one another by R8,

R8 is 1) halogen,
2) $-NO_2$,
3) -CN,
4) $-C(O)-NH_2$,
5) -OH,
6) $-NH_2$,
7) $-O-CF_3$,
8) $-O-(C_1-C_8)$-alkyl, wherein alkyl is unsubstituted or mono-, di- or trisubstituted independently of one another by halogen, $NH_2$, -OH or a methoxy residue,
9) $-(C_1-C_8)$-alkyl, wherein alkyl is unsubstituted or mono-, di- or trisubstituted independently of one another by halogen, $NH_2$, -OH or a methoxy residue,
10) $-SO_2-CH_3$ or
11) $-SO_2-CF_3$,

provided that R8 is at least one halogen, $-C(O)-NH_2$ or $-O-(C_1-C_8)$-alkyl residue,

Q is a direct bond, $-(C_0-C_2)$-alkylene-C(O)-$(C_0-C_2)$-alkyl, $-(C_1-C_6)$-alkylene, $-(C_0-C_3)$-alkylene-$S(O)_2$- or $-(C_0-C_2)$-alkylene-$NR^{10}$-C(O)-O-$(C_0-C_2)$-alkylene, wherein $R^{10}$ is as defined below, and wherein the alkylene residues are unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or

oxygen; $-(C_1-C_3)$-perfluoroalkylene, $-(C_1-C_3)$-alkylene-S(O)$-(C_1-C_4)$-alkyl, $-(C_1-C_3)$-alkylene-$S(O)_2-(C_1-C_3)$-alkyl, $-(C_1-C_3)$-alkylene-$S(O)_2-N(R^{4'})-R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a 3- to 7-membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =0, $-(C_1-C_8)$-alkyl, $-(C_1-C_4)$-alkoxy, $-NO_2$, $-(C_0-C_4)$-alkyl-C(O)-O-$R^{18}$, -CN, $-(C_0-C_4)$-alkyl-$N(R^{18})-R^{21}$, $-(C_0-C_4)$-alkyl-O-$R^{18}$, $-(C_0-C_4)$-alkyl-het, $-(C_0-C_8)$-alkyl-$SO_2-(C_1-C_4)$-alkyl, $-(C_0-C_8)$-alkyl-$SO_2-(C_1-C_3)$-perfluoroalkyl, $-(C_0-C_8)$-alkyl-$SO_2-N(R^{18})-R^{21}$, -C(O)-NH-$(C_1-C_8)$-alkyl, -C(O)-N-$[(C_1-C_8)\text{-alkyl}]_2$, $-NR^{18}$-C(O)-NH-$(C_1-C_8)$-alkyl, $-C(O)-NH_2$, $-S-R^{18}$, or $-NR^{18}$-C(O)-NH-$[(C_1-C_8)\text{-alkyl}]_2$, wherein $R^{18}$ and $R^{21}$ are independently from each other hydrogen atom, $-(C_1-C_3)$-perfluoroalkyl or $-(C_1-C_6)$-alkyl,

V is 1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_n-$, $-(CH_2)_m-NR^{10}-C(O)-(CH_2)_n-$, $-(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$,

n and m are independently of one another identical or different and are the integers zero, 1, 2, 3, 4, 5 or 6,

M is 1) a 6- to14-membered aryl selected out of the group phenyl,

naphthyl, biphenylyl, 2-biphenylyl, 3-biphenylyl, 4-biphenylyl, anthryl or fluorenyl; wherein aryl is unsubstituted or mono-, di- or trisubstituted independently of one another by R14,

2) a monocyclic or bicyclic 4- to 15-membered heterocyclyl selected out of the group acridinyl, azaindole (1H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo-(1,3,4)oxathiazine, 6H-1,5,2-dithiazinyl, dihydrofuro [2,3-b]-tetrahydrofuranyl, furanyl, furazanyl, imidazolidinyl, imidazolinyl, imidazolyl, H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, odahydroisoquinolinyl, oxadiazolyl, 1 ,2,3-oxadiazolyl, 1,2,4-oxadiazolyl, 1,2,5-oxadiazolyl, 1,3,4-oxadiazolyl, 1,2-oxa-thiepanyl, 1 ,2-oxathiolanyl, 1 , 4-oxazepanyl, 1,4-oxazepinyl, 1,2-oxazinyl, 1 ,3-oxazinyl, 1 ,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1 ,2,5-thiadiazinyl, 1,2,3-thiadiazolyl, 1,2,4-thiadiazolyl, 1 ,2,5-thiadiazolyl, 1 ,3,4-thiadiazolyl, thianthrenyl, 1 ,2-thiazinyl, 1,3-thiazinyl, 1,4-thiazinyl, 1 ,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1 ,2,3-triazolyl, 1,2,4-triazolyl, 1,2,5-triazolyl, 1,3,4-triazolyl and xanthenyl, wherein heterocyclyl is unsubstituted or mono-, di- or trisubstituted independently of one another by R14,

3) a 3- to 7-membered cyclic residue, containing 1, 2, 3 or 4 heteroatoms chosen from nitrogen, sulfur or oxygen, selected out of the group azepine, azetidine, aziridine, azirine, 1,4 diazepane, 1,2-diazepine, 1,3-diazepine, 1,4-diazepine, diaziridine, diazirine, dihydroimidazolone, dioxazole, dioxazine, dioxole, 1,3-dioxolene, 1 ,3-dioxolane, furan, imidazole, imidazoline, imidazolidine, imidazolidinone, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazolone, oxazole, [1,3,4]oxathiazinane 3,3-dioxide, oxaziridine, oxazolidinone, oxetan, oxirane, piperazine, piperidine, pyran, pyrazine, pyrazole, pyrazin-2-one, piperazin-2-one, pyrazoline, pyrazolidine, pyridazine, pyridine, pyridinone, pyrimidine, pyrimidine-2,4-dione, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1 ,3-thiazole, thiazole, thiazolidine, thiazoline, thienyl, thietan, thiomorpholine, thiomorpholine 1,1-dioxide thiopyran, 1,2,3-triazine, 1,2,4-triazine, 1,3,5-triazine, 1,2,3-triazole or 1,2,4-triazole, wherein said cyclic residue is unsubstituted or mono-, di- or trisubstituted independently of one another by R14, wherein R14 is defined above,

$R^3$, $R^4$, $R^5$ or $R^6$ are independent of one another and are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

   a) hydrogen atom,
   b) -($C_1$-$C_4$)-alkyl, wherein alkyl is unsubstituted or mono-, di- or trisubstituted independently of one another by R13, or
   c) phenyl, wherein phenyl is unsubstituted or mono-, di- or trisubstituted independently of one another by R13,
   d) -$CF_3$, or
   e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O) -($C_1$-$C_4$)-alkyl,
18) -C(0)-0-C(R15, R16)-0-C(0)-R17,
19) -($C_0$-$C_2$)alkylene-C(O)-O-($C_2$-$C_4$)-alkylene-O-C(O) -O-($C_1$-$C_6$)-alkyl,
20) -C(0)-0- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cy-

cloalkyl is unsubstituted or mono-, di- or trisubstituted independently of one another by R13,

24) -($C_1$-$C_4$)-alkylene-het, wherein het is unsubstituted or mono-, di- or trisubstituted independently of one another by R13,

25) -($C_0$-$C_4$)-alkylene-O-$CH_2$-($C_1$-$C_3$)-perfluoroalkylene-$CH_2$-O-($C_0$-$C_4$)-alkyl,

26) -$SO_w$-N($R^{11}$)-$R^{13}$, wherein w is 1 or 2,

27) -($C_0$-$C_4$)-alkylene-C(O)-N($R^{11}$)-$R^{13}$,

28) -($C_0$-$C_4$)-alkylene-N($R^{11}$)-$R^{13}$, or

29) a residue from the following list

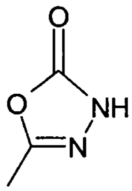
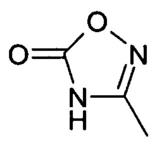
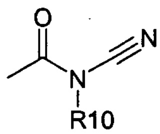
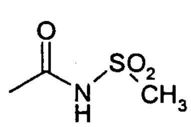
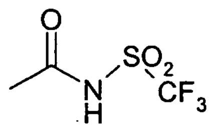

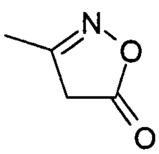
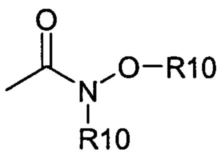
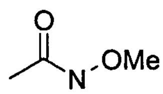
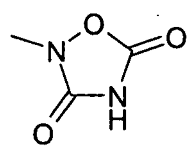
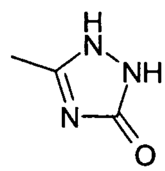

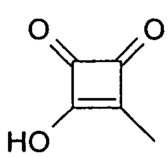
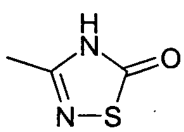
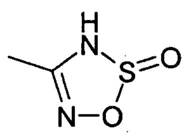
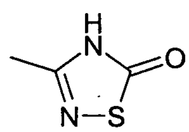
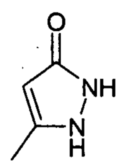
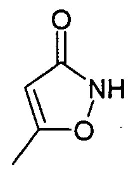

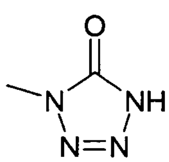
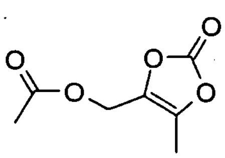
and
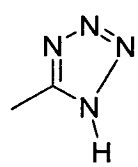

wherein Me is methyl, or

if two -OR19 residues are attached to adjacent atoms they can form together with the atoms which they are attached to a 5- or 6- membered ring, which is unsubstituted or substituted one, two, three or four times by R13,

provided that the residue -NH-$CH_2$($R^4$)-$R^3$ , which is part of formula I, is not one of the following linkage residues -NH-$CH_2$($R^4$)-N- or -N-$CH_2$($R^4$)-O-, wherein R4 is as defined above, or

R3 and R4, or R5 and R6, or R3 and R5 or R4 and R6 together with the carbon atom to which they are bonded can form a 3- to 8-membered ring, containing zero, 1, 2, 3 or 4 heteroatoms chosen from nitrogen, sulfur or oxygen, wherein said ring is unsubstituted or mono-, di- or trisubstituted independently of one another by R13, wherein R13 is defined below, or

R3 and R5 or R4 and R6 together with the carbon atoms to which they are bonded can form a 4-to 8-membered ring, containing zero, 1, 2, 3 or 4 heteroatoms chosen from nitrogen, sulfur or oxygen, wherein said ring is unsubstituted or mono-, di- or trisubstituted independently of one another by R13, wherein R13 is defined below,

R11 and R12 are independently of one another identical or different and are

1) hydrogen atom,
2) $-(C_1-C_6)$-alkyl, wherein alkyl is unsubstituted or mono-, di- or trisubstituted independently of one another by R13,
3) $-(C_0-C_6)$-alkyl-$(C_3-C_8)$-cycloalkyl,
4) $-SO_t-R^{10}$, wherein t is 1 or 2,
5) $-(C_0-C_6)$-alkyl-$(C_6-C_{14})$-aryl, wherein alkyl and aryl independently from one another are unsubstituted or mono-, di- or trisubstituted by R13,
6) $-(C_1-C_3)$-perfluoroalkyl,
7) $-O-R^{17}$, or
8) $-(C_0-C_6)$-alkyl-$(C_4-C_{15})$-heterocyclyl, wherein alkyl and heterocyclyl independently from one another are unsubstituted or mono-, di- or trisubstituted by R13, or

R11 and R12 together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, $-NO_2$, -CN, =0, -OH, $-CF_3$, $-C(O)-O-R^{10}$, $-C(O)-N(R^{10})-R^{20}$, $-N(R^{10})-R^{20}$, $-(C_3-C_8)$-cycloalkyl, $-(C_0-C_3)$-alkylene-$O-R^{10}$, $-Si-(CH_3)_3$, $-N(R^{10})-S(O)_u-R^{10}$, wherein u is 1 or 2, $-S-R^{10}$, $-SO_r-R^{10}$, wherein r is 1 or 2, $-S(O)_v-N(R^{10})-R^{20}$, wherein v is 1 or 2, $-C(O)-R^{10}$, $-(C_1-C_8)$-alkyl, $-(C_1-C_8)$-alkoxy, phenyl, phenyloxy-, $-(C_1-C_3)$-perfluoroalkyl, $-(C_0-C_4)$-alkyl-C(O)-O-C(R15, R16)-O-C(O)-R17, -0-R15, $-NH-C(O)-NH-R^{10}$, $-O-CF_3$, $-(C_0-C_4)$-alkyl-C(O)-O-C(R15, R16)-O-C(O)-O-R17, $-NH-C(O)-O-R^{10}$, or a residue from the following list

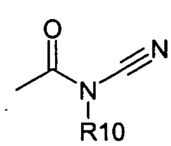
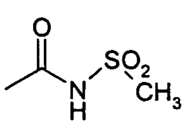
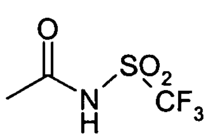
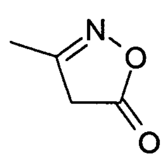
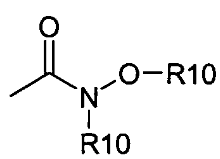

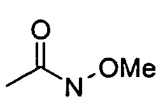
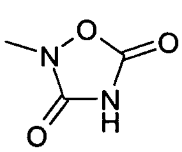
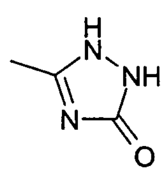
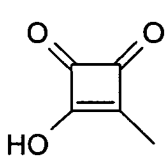
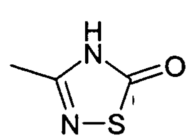
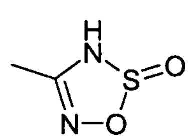

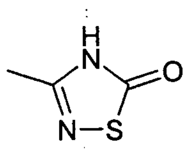 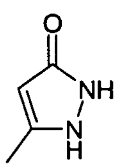 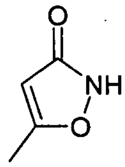 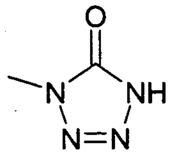 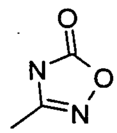 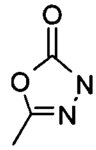 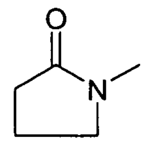

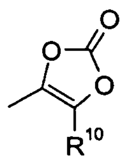 and 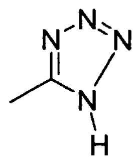 ,

$R^{10}$ and $R^{20}$ are independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

2) The present invention also relates to compounds of the formula I, wherein

$R^0$ is 1) a monocyclic or bicyclic 5- to 15-membered heterocyclyl out of the group benzothienyl, indazolyl, indolyl, imidazolyl, isoindolyl, isoquinolyl, phthalazinyl, pyridyl, pyrimidinyl, pyridazinyl, pyrazolyl, pyrazinyl, quinazolinyl, quinolyl, thiazolyl, thiadiazolyl, thienyl or triazolyl, wherein said heterocyclyl is unsubstituted or mono-, di- or trisubstituted independently of one another by R8, or
2) a 6- to14-membered aryl selected out of the group phenyl, naphthyl, biphenylyl, 2-biphenylyl, 3-biphenylyl, 4-biphenylyl, anthryl or fluorenyl, wherein aryl is mono-, di- or trisubstituted independently of one another by R8,

R8 is 1) halogen,
2) $-NO_2$,
3) -CN,
4) -C(O)-$NH_2$,
5) -OH,
6) $-NH_2$,
7) -O-$CF_3$
8) $-(C_1-C_8)$-alkyl, wherein alkyl is unsubstituted or mono-, di- or trisubstituted independently of one another by halogen, $NH_2$, -OH or a methoxy residue, or
9) -O-$(C_1-C_8)$-alkyl, wherein alkyl is unsubstituted or mono-, di- or trisubstituted independently of one another by halogen, $NH_2$, -OH or a methoxy residue,
10) $-SO_2-CH_3$ or
11) $-SO_2-CF_3$,

provided that R8 is at least one halogen, -C(O)-$NH_2$ or -O-$(C_1-C_8)$-alkyl residue,

Q is a direct bond, $-(C_0-C_2)$-alkylene-C(O)-$(C_0-C_2)$-alkyl, $-(C_1-C_6)$-alkylene, $-(C_0-C_3)$-alkylene-$SO_2$-, $-(C_0-C_2)$-alkylene-$NR^{10}$-C(O)-O-$(C_0-C_2)$-alkylene, wherein $R^{10}$ is as defined below, and wherein the alkylene residues are unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen; $-(C_1-C_3)$-perfluoroalkylene, $-(C_1-C_3)$-alkylene-S(O)-$(C_1-C_4)$-alkyl, $-(C_1-C_3)$-alkylene-S$(O)_2$-$(C_1-C_3)$-alkyl, $-(C_1-C_3)$-alkylene-$S(O)_2$-N($R^{4'}$)-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, dior trisubstituted independently of one another by R14,

R14 is halogen, -OH, =0, $-(C_1-C_8)$-alkyl, $-(C_1-C_4)$-alkoxy, $-NO_2$, $-(C_0-C_4)$-alkyl-C(O)-O-$R^{18}$, -CN, $-(C_0-C_4)$-alkyl-N($R^{18}$)-$R^{21}$, $-(C_0-C_4)$-alkyl-O-$R^{18}$, $-(C_0-C_4)$-alkyl-het, $-(C_0-C_8)$-alkyl-$SO_2$-$(C_1-C_4)$-alkyl, $-(C_0-C_8)$-alkyl-$SO_2$-$(C_1-C_3)$-perfluoroalkyl, $-(C_0-C_8)$-alkyl-$SO_2$-N($R^{18}$)-$R^{21}$, -C(O)-NH-$(C_1-C_8)$-alkyl, -C(O)-N-$[(C_1-C_8)$-alkyl$]_2$, -$NR^{18}$-C(O)-NH-$(C_1-C_8)$-alkyl, -C(O)-$NH_2$, -S-$R^{18}$, or -$NR^{18}$-C(O)-NH-$[(C_1-C_8)$-alkyl$]_2$, wherein $R^{18}$ and $R^{21}$ are independently from each other hydrogen atom, $-(C_1-C_3)$-perfluoroalkyl or $-(C_1-C_6)$-alkyl,

V is 1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m$-$NR^{10}$-$SO_2$-$NR^{10}$-$(CH_2)_n$-, $-(CH_2)_m$-, $-(CH_2)_m$-O-$(CH_2)_n$-, $-(CH_2)_m$-C(O)-$NR^{10}$-$(CH_2)_n$-, $-(CH_2)$-$SO_2$-$(CH_2)_n$-,$-(CH_2)_m$-$NR^{10}$-C(O)-$NR^{10}$-$(CH_2)_n$-, $-(CH_2)_m$-$NR^{10}$-C(O)-$(CH_2)_n$-, $-(CH_2)_m$-C(O)-$(CH_2)_n$-, $-(CH_2)_m$-$SO_2$-$NR^{10}$-$(CH_2)_n$-, $-(CH_2)_m$-$NR^{10}$-$SO_2$-$(CH_2)_n$-, $-(CH_2)_m$-$NR^{10}$-, $-(CH_2)_m$-O-C(O)-$NR^{10}$-$(CH_2)_n$- or $-(CH_2)_m$-$NR^{10}$-C(O)-O-$(CH_2)_n$-,

n and m are independently of one another identical or different and are the integers zero, 1, 2, 3, 4, 5 or 6,

M is 1) a 6- to14-membered aryl selected out of the group phenyl, naphthyl, biphenylyl, 2-biphenylyl, 3-biphenylyl, 4-biphenylyl, anthryl or fluorenyl, wherein aryl is unsubstituted or mono-, di- or trisubstituted independently of one another by R14,
2) a monocyclic or bicyclic 4- to 15-membered heterocyclyl selected out of the group acridinyl, azaindole (1H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo-(1,3,4)oxathiazine, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1 ,2,4-oxadiazolyl, 1,2,5-oxadiazolyl, 1,3,4-oxadiazolyl, 1 ,2-oxa-thiepanyl, 1 ,2-oxathiolanyl, 1 ,4-oxazepanyl, 1,4-oxazepinyl, 1 ,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1 ,2,3-triazinyl, 1 ,2,4-triazinyl, 1 ,3,5-triazinyl, 1,2,3-triazolyl, 1,2,3-triazolyl, 1,2,4-triazolyl, 1,2,5-triazolyl, 1,3,4-triazolyl and xanthenyl, wherein heterocyclyl is unsubstituted or mono-, di- or trisubstituted independently of one another by R14,
3) a 3- to 7-membered cyclic residue, containing 1, 2, 3 or 4 heteroatoms chosen from nitrogen, sulfur or oxygen, selected out of the group azepine, azetidine, aziridine, azirine, 1,4 diazepane, 1,2-diazepine, 1,3-diazepine, 1,4-diazepine, diaziridine, diazirine, dihydroimidazolone, dioxazole, dioxazine, dioxole, 1 ,3-dioxolene, 1 ,3-dioxolane, furan, imidazole, imidazoline, imidazolidine, imidazolidinone, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazolone, oxazole, [1,3,4]oxathiazinane 3,3-dioxide, oxaziridine, oxazolidinone, oxetan, oxirane, piperazine, piperidine, pyran, pyrazine, pyrazole, pyrazin-2-one, piperazin-2-one, pyrazoline, pyrazolidine, pyridazine, pyridine, pyridinone, pyrimidine, pyrimidine-2,4-dione, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiomorpholine 1,1-dioxide thiopyran, 1,2,3-triazine, 1,2,4-triazine, 1,3,5-triazine, 1,2,3-triazole or 1,2,4-triazole, wherein said cyclic residue is unsubstituted or mono-, di- or trisubstituted independently of one another by R14, wherein R14 is defined above,

$R^3$, $R^4$, $R^5$ or $R^6$ are independent of one another and are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

a) hydrogen atom,
b) -($C_1$-$C_4$)-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$,
e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,

13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(O)-O-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(O)-O-C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_3$)-alkylene-O-$CH_2$-($C_1$-$C_3$)-perfluoroalkylene-$CH_2$-O-($C_0$-$C_3$)-alkyl, or
26) -$SO_w$-N($R^{11}$)-$R^{13}$, wherein w is 1 or 2,
27) -($C_0$-$C_4$)-alkylene-C(O)-N($R^{11}$)-$R^{13}$ ,
28) -($C_0$-$C_4$)-alkylene-N($R^{11}$)-$R^{13}$, or
29) a residue from the following list

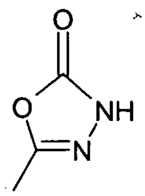
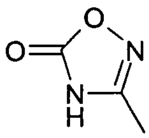
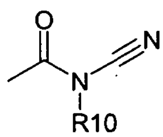
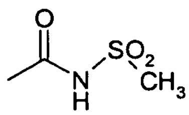
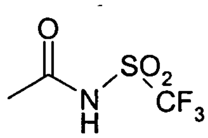

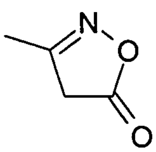
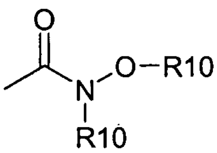
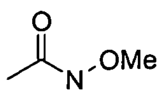
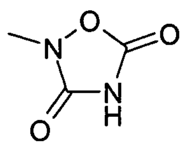
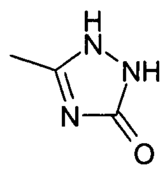

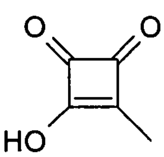
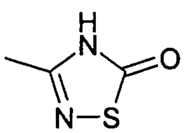
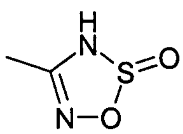
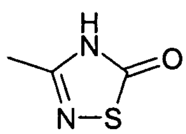
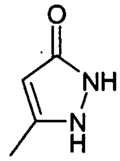
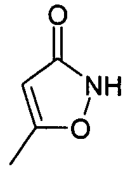

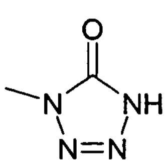
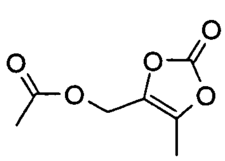
and
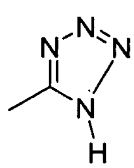

wherein Me is methyl, or if two -OR19 residues are attached to adjacent atoms they can form together with the atoms which they are attached to a 5- or 6- membered ring, which is unsubstituted or substituted one, two, three or four times by R13,

provided that the residue -NH-$CH_2(R^4)$-$R^3$ , which is part of formula I, is not one of the following linkage residues -NH-$CH_2(R^4)$-N- or -N-$CH_2(R^4)$-O-, wherein R4 is as defined above, or

R3 and R4 or R5 and R6 together with the carbon atom to which they are bonded can form a 3-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or

R3 and R5 or R4 and R6 together with the carbon atoms to which they are bonded can form a 4-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =0, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -($C_1$-$C_3$)-perfluoroalkyl, -($C_0$-$C_4$)-alkyl-C(O)-O-C(R15, R16)-0-C(0)-R17, -O-R15, -NH-C(O)-NH-$R^{10}$, -O-$CF_3$, -($C_0$-$C_4$)-alkyl-C(O)-O-C(R15, R16)-0-C(0)-0-R17, -NH-C(O)-O-$R^{10}$, or a residue from the following list

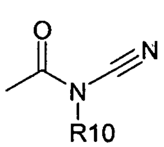 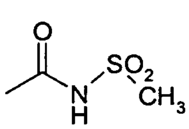 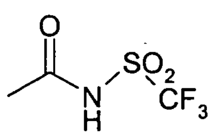 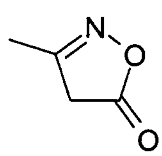 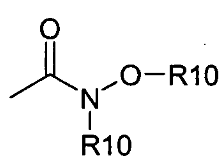

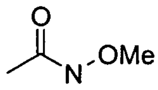 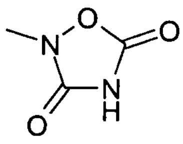 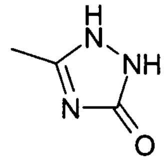 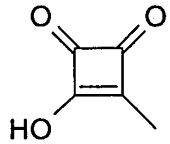 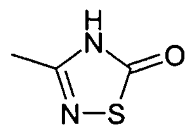 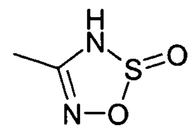

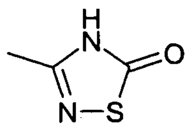 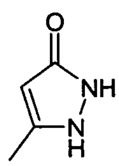 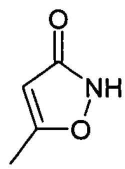 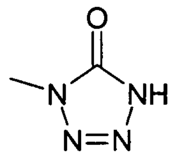 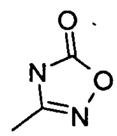 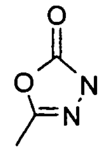 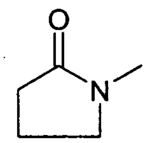

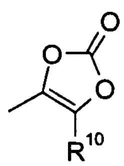 and 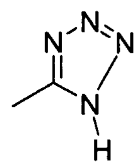 ,

| | |
|---|---|
| $R^{10}$ and $R^{20}$ | are independently of one another hydrogen, $-(C_1-C_6)$-alkyl or $-(C_1-C_3)$-perfluoroalkyl, |
| R15and R16 | are independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and |
| R17 | is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$, |

in all its stereoisomeric forms and mixtures thereof in any ratio, and its physiologically tolerable salts.

3) Thus, the present invention relates to compounds of the formula I, wherein

$R^0$ is 1) phenyl or naphtyl, wherein phenyl or naphtyl is mono-, di- or trisubstituted independently of one another by R8,

2) a monocyclic or bicyclic 4- to 15-membered heterocyclyl out of the group benzimidazolyl, 1,3-benzodioxolyl, benzofuranyl, benzoxazolyl, benzothiazolyl, benzothienyl, cinnolinyl, chromanyl, indazolyl, indolyl, isochromanyl, isoindolyl, isoquinolinyl, imidazolyl phenylpyridyl, phthalazinyl, pteridinyl, purinyl, pyridinyl, pyridoimidazolyl, pyridopyrimidinyl, pyridothienyl, pyrimidinyl, quinazolinyl, quinolyl, quinoxalinyl or 1,4,5,6-tetrahydropyridazinyl, thiadiazolyl, thiazolyl, thienyl, wherein said heterocyclyl is unsubstituted or mono-, di- or trisubstituted independently of one another by R8, or

3) a heterocyclyl, wherein heterocyclyl is selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1 , 3,4-oxadiazolyl, 1 ,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl,

pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, 1,4,5,6-tetrahydro-pyridazinyl, tetrahydropyridinyl, tetrahydrothiophenyl, tetrazinyl, tetrazolyl, 6H-1 ,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1 ,4-thiazinyl, 1 ,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1 ,2,3-triazinyl, 1 ,2,4-triazinyl, 1,3,5-triazinyl, 1,2,3-triazolyl, 1,2,4-triazolyl, 1,2,5-triazolyl, 1,3,4-triazolyl and xanthenyl,

wherein said heterocyclyl is unsubstituted or mono-, di- or trisubstituted independently of one another by R8

R8 is
1) halogen,
2) $-NO_2$,
3) -CN,
4) $-C(O)-NH_2$,
5) -OH,
6) $-NH_2$,
7) $-O-CF_3$,
8) $-(C_1-C_8)$-alkyl, wherein alkyl is unsubstituted or mono-, di- or trisubstituted independently of one another by halogen, $NH_2$, -OH or a methoxy residue,
9) $-O-(C_1-C_8)$-alkyl, wherein alkyl is unsubstituted or mono-, di- or trisubstituted independently of one another by halogen, $NH_2$, -OH or a methoxy residue,
10) $-SO_2-CH_3$ or
11) $-SO_2-CF_3$,

provided that R8 is at least one halogen, $-C(O)-NH_2$ or $-O-(C_1-C_8)$-alkyl residue,

Q is $-(C_0-C_2)$-alkylene-C(O)-$(C_0-C_2)$-alkylene- or $-SO_2-$, wherein the alkylene residue is unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH;

$R^1$ is hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, an aryl out of the group phenyl, naphthyl, biphenylyl, anthryl or fluorenyl, wherein aryl is mono-, di- or trisubstituted independently of one another by R8, a monocyclic or bicyclic 4- to 15-membered heterocyclyl,which is as defined above; $-(C_1-C_3)$-perfluoroalkylene, $-(C_1-C_3)$-alkylene-S(O)$-(C_1-C_4)$-alkyl, $-(C_1-C_3)$-alkylene-$S(O)_2$-$(C_1-C_3)$-alkyl, $-(C_1-C_3)$-alkylene-$S(O)_2$-N($R^{4'}$)-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a residue selected out of the group azepine, azetidine, aziridine, azirine, 1,4-diazapane, 1,2-diazepine, 1,3-diazepine, 1,4-diazepine, diaziridine, diazirine, dioxazole, dioxazine, dioxole, 1,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1 ,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, 2,3 dihydroindole, imidazole, imidazoline, imidazolidine, indole, isothiazole, isothiazolidine, isothiazoline, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, piperazine, piperidine, pyrazine, pyrazole, pyrazoline, pyrazolidine, pyrrole, pyrrolidine, pyrrolidinone, pyrroline, tetrahydropyridine, thiazolidine, thiazoline or thiomorpholine, wherein said cyclic group is unsubstituted or mono-, di- or trisubstituted independently of one another by R14,

R14 is halogen, -OH, =0, -($C_1$-$C_8$)-alkyl, -($C_1$-$C_4$)-alkoxy, -$NO_2$, -($C_0$-$C_4$)-alkyl-C(O)-O-$R^{18}$, -CN, -($C_0$-$C_4$)-alkyl-N($R^{18}$)-$R^{21}$, -($C_0$-$C_4$)-alkyl-O-$R^{18}$, -($C_0$-$C_4$)-alkyl-het, wherein het is a residue selected from azetidine, azetidinone, piperidine, piperazine, pyridine, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, -($C_0$-$C_8$)-alkyl-$SO_2$-($C_1$-$C_4$)-alkyl, -($C_0$-$C_8$)-alkyl-$SO_2$-($C_1$-$C_3$)-perfluoroalkyl, -($C_0$-$C_8$)-alkyl-$SO_2$-N($R^{18}$)-$R^{21}$, -C(O)-NH-($C_1$-$C_8$)-alkyl, -C(O)-N-[($C_1$-$C_8$)-alkyl$]_2$, -$NR^{18}$-C(O)-NH-($C_1$-$C_8$)-alkyl, -C(O)-$NH_2$, -S-$R^{18}$, or -$NR^{18}$-C(O)-NH-[($C_1$-$C_8$)-alkyl$]_2$, wherein $R^{18}$ and $R^{21}$ are independently from each other hydrogen atom, -($C_1$-$C_3$)-perfluoroalkyl or -($C_1$-$C_6$)-alkyl,

V is 1) an aryl out of the group phenyl or naphthyl, wherein aryl is mono-, di- or trisubstituted independently of one another by R14, or

2) a heterocyclyl out of the group acridinyl, azaindole (1H-pyrrolopyridine), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 1,4-diazepane, 4,5-dihydrooxa-zolinyl, dioxazolyl, dioxazinyl, 1 ,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1 ,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1 ,2,5-thiadiazinyl, 1 ,2,3-thiadiazolyl, 1,2,4-thiadiazolyl, 1 ,2,5-thiadiazolyl, 1,3,4-thiadiazolyl, thianthrenyl, 1,2-thiazinyl, 1 ,3-thiazinyl, 1 , 4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1,2,3-triazinyl, 1,2,3-triazolyl, 1,2,3-triazolyl, 1,2,4-triazolyl, 1,2,5-triazolyl, 1,3,4-triazolyl and xanthenyl, wherein said heterocyclyl is unsubstituted or mono-, di- or trisubstituted independently of one another by R14,

G is a direct bond, -$(CH_2)_m$-$NR^{10}$-$SO_2$-$NR^{10}$-$(CH_2)_n$-, -$(CH_2)_m$-, -$(CH_2)_m$-C(O)-$NR^{10}$-$(CH_2)_n$-, -$(CH_2)$-$SO_2$-$(CH_2)_n$-,-$(CH_2)_m$-$NR^{10}$-C(O)-$NR^{10}$-$(CH_2)_n$-, -$(CH_2)_m$-$NR^{10}$-C(O)-$(CH_2)_n$-, -$(CH_2)_m$-C(O)-$(CH_2)_n$-, -$(CH_2)_m$-$SO_2$-$NR^{10}$-$(CH_2)_n$-, -$(CH_2)_m$-$NR^{10}$-$SO_2$-$(CH_2)_n$-, n and m are independently of one another identical or different and are the integers zero, 1, 2, 3, 4, 5 or 6,

M is heterocyclyl, wherein heterocyclyl is a residue out of the group which can be derived from azepane, azepine, 1,4-diazepane, 1,2-diazepine, 1,3-diazepine, 1,4-diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3-triazine, 1,2,4-triazine, 1,3,5-triazine, 1,2,3-triazole or 1,2,4-triazole, wherein said heterocyclyl is un-substituted or mono-, di- or trisubstituted independently of one another by R14,

$R^3$, $R^4$, $R^5$ or $R^6$ are independent of one another are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

    a) hydrogen atom,
    b) -($C_1$-$C_4$)-alkyl, wherein alkyl is unsubstituted or mono-, di- or trisubstituted independently of one another by R13, or
    c) phenyl, wherein phenyl is unsubstituted or mono-, di- or trisubstituted independently of one another by R13,
    d) -$CF_3$,
    e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(0)-0-C(R15, R16)-O-C(O)-R17,
19) -($C_0$-$C_2$)alkylene-C(O)-O-($C_2$-$C_4$)-alkylene-O-C(O)-O-($C_1$-$C_6$)-alkyl,
20) -C(O)-O-C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_3$)-alkylene-O-$CH_2$-($C_1$-$C_3$)-perfluoroalkylene-$CH_2$-O-($C_0$-$C_3$)-alkyl,
26) -$SO_W$-N($R^{11}$)-$R^{13}$, wherein w is 1 or 2,
27) -($C_0$-$C_4$)-alkylene-C(O)-N($R^{11}$)-$R^{13}$,
28) -($C_0$-$C_4$)-alkylene-N($R^{11}$)-$R^{13}$, or
29) a residue from the following list

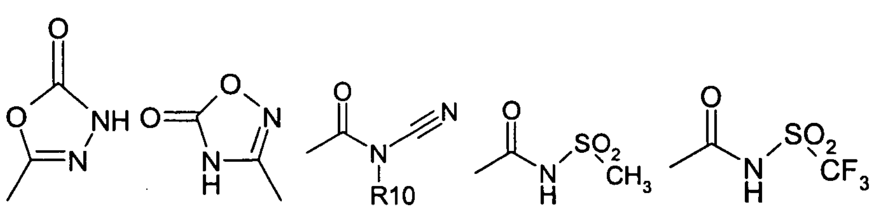

.

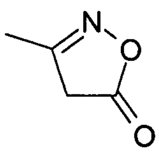 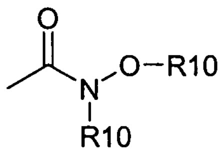 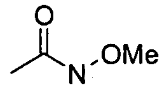 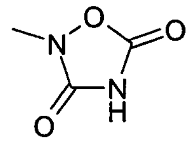 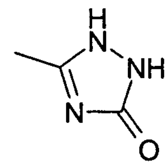

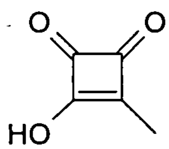 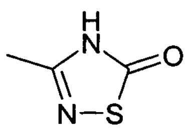 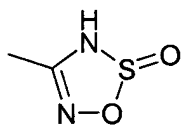 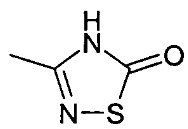 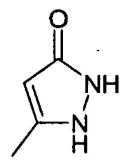 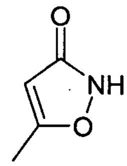

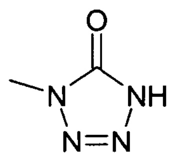 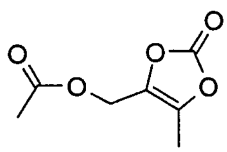 and 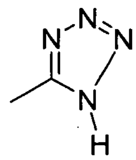

wherein Me is methyl, or if two -OR19 residues are attached to adjacent atoms they can form together with the atoms which they are attached to a 5- or 6- membered ring, which is unsubstituted or substituted one, two, three or four times by R13,

provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or

R3 and R4 or R5 and R6 together with the carbon atom to which they are bonded can form a 3-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or

R3 and R5 or R4 and R6 together with the carbon atoms to which they are bonded can form a 4-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11 and R12 are independently of one another identical or different and are

1) hydrogen atom,
2) $-(C_1-C_6)$-alkyl, wherein alkyl is unsubstituted or mono-, di- or trisubstituted independently of one another by R13,
3) $-(C_0-C_6)$-alkyl-$(C_3-C_8)$-cycloalkyl,
4) $-SO_t-R^{10}$, wherein t is 1 or 2,
5) $-(C_0-C_6)$-alkyl-$(C_6-C_{14})$-aryl, wherein alkyl and aryl independently from one another are unsubstituted or mono-, di- or trisubstituted by R13,

6) -($C_1$-$C_3$)-perfluoroalkyl,

7) -O-$R^{17}$, or

8) -($C_0$-$C_6$)-alkyl-($C_4$-$C_{15}$)-heterocyclyl, wherein alkyl and heterocyclyl are as defined above and are independently from one another unsubstituted or mono-, di- or trisubstituted by R13, or

R11 and R12 together with the nitrogen atom to which they are bonded form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R13 is halogen, -$NO_2$, -CN, =0, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -O-$CF_3$, -($C_0$-$C_4$)-alkyl-C(O)-O-C(R15, R16)-0-C(0)-R17, -($C_1$-$C_4$)-alkoxy-phenyl, -($C_0$-$C_4$)-alkyl-C(O)-O-C(R15, R16)-O-C(O)-O-R17, -($C_1$-$C_3$)-perfluoroalkyl, -O-R15, -NH-C(O)-NH-$R^{10}$, -NH-C(O)-O-$R^{10}$, or a residue from the following list

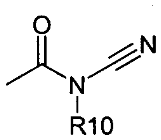
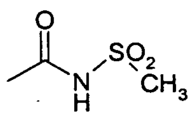
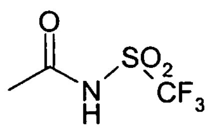
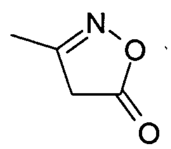
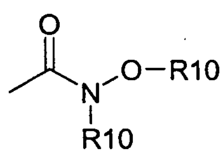

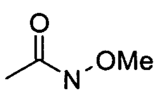
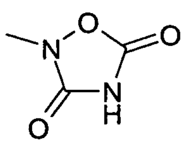
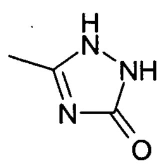
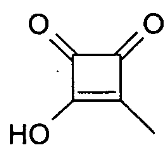
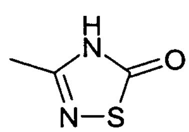
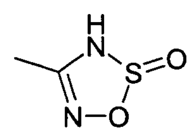

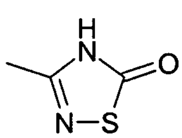
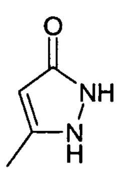
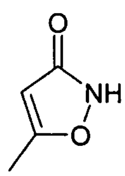
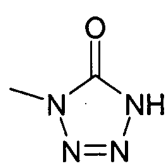
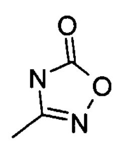
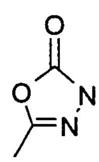
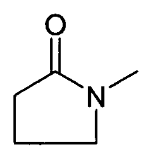

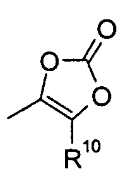
and
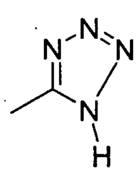
,

$R^{10}$ and $R^{20}$ are independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are independently of one another hydrogen, -($C_1$-$C_6$)-alkyl, cyclopropyl, cyclobutyl, cyclopentyl or cyclohexyl, wherein each ring is unsubstituted or substituted one to three

times by $R^{10}$, and

R17 is -$(C_1-C_6)$-alkyl, -$(C_1-C_6)$-alkyl-OH, -$(C_1-C_6)$-alkyl-O-$(C_1-C_6)$-alkyl, -$(C_3-C_8)$-cycloalkyl, -$(C_1-C_6)$-alkyl-O-$(C_1-C_8)$-alkyl-$(C_3-C_8)$-cycloalkyl, -$(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

4) The present invention also relates to the compounds of the formula I, wherein

$R^0$ is 1) a monocyclic or bicyclic 6- to 14-membered aryl out of the group naphthyl or phenyl, wherein aryl is mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, imidazolyl phenylpyridyl, phthalazinyl, pteridinyl, purinyl, pyridinyl, pyridoimidazolyl, pyridopyrimidinyl, pyridothienyl, pyrimidinyl, quinazolinyl, quinolyl, quinoxalinyl or 1,4,5,6-tetrahydro-pyridazinyl, thienyl, thiazolyl, thiadiazolyl wherein said heterocyclyl is unsubstituted or mono-, di- or trisubstituted independently of one another by R8, or
3) a heterocyclyl out of the group azabenzimidazolyl, benzimidazolyl, 1,3-benzodioxolyl, benzofuranyl, benzothiazolyl, benzothiophenyl, benzoxazolyl, chromanyl, cinnolinyl, 2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, thiadiazolyl, 2-thienyl or 3-thienyl, wherein said heterocyclyl is unsubstituted or mono-, di- or trisubstituted independently of one another by R8,

R8 is 1. fluorine, chlorine or bromine,
2. -$NO_2$,
3. -CN,
4. -C(O)-$NH_2$,
5. -OH,
6. -$NH_2$,
7. -$OCF_3$
8. -$(C_1-C_8)$-alkyl, wherein alkyl is unsubstituted or mono-, di- or trisubstituted independently of one another by halogen, $NH_2$, -OH or a methoxy residue, or
9. -O-$(C_1-C_8)$-alkyl, wherein alkyl is unsubstituted or mono-, di- or trisubstituted independently of one another by halogen, $NH_2$, -OH or a methoxy residue,
10. -$SO_2CH_3$ or
11. -$SO_2CF_3$,

provided that R8 is at least one halogen, -C(O)-$NH_2$ or -O-$(C_1-C_8)$-alkyl residue,

Q is a -$(C_0-C_2)$-alkylene-C(O)-$(C_0-C_2)$-alkylene-, -$SO_2$-, wherein the alkylene residue is unsubstituted or mono-, di- or trisubstituted independently of one another by halogen, -$NH_2$ or -OH; or -$(C_3-C_6)$-cycloalkylen, wherein cycloalkylen is unsubstituted or mono-, di- or trisubstituted independently of one another by halogen, -$NH_2$ or -OH;

$R^1$ is a hydrogen atom, -$(C_1-C_4)$-alkyl, wherein alkyl is unsubstituted or substituted one to three times by R13; -$(C_1-C_3)$-alkylene-C(O)-NH-$R^0$, -$(C_1-C_3)$-alkylene-C(O)-O-R15, -$(C_1-C_3)$-perfluoroalkylene, -$(C_1-C_3)$-alkylene-S(O)-$(C_1-C_4)$-alkyl, -$(C_1-C_3)$-alkylene-$S(O)_2$-$(C_1-C_3)$-alkyl, -$(C_1-C_3)$-alkylene-$S(O)_2$-N($R^{4'}$)-$R^{5'}$, -$(C_1-C_3)$-alkylene-O-$(C_1-C_4)$-alkyl, -$(C_0-C_3)$-alkylene-$(C_3-C_8)$-cycloalkyl, or -$(C_0-C_3)$-alkylene-het, wherein het is a residue selected out of the group azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole,

thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or -($C_1$-$C_4$)-alkyl,

$R^2$ is a direct bond or -($C_1$-$C_4$)-alkylene,

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, 2,3 dihydroindole, imidazole, imidazoline, imidazolidine, indole, isothiazole, isothiazolidine, isothiazoline, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, piperazine, piperidine, pyrazine, pyrazole, pyrazoline, pyrazolidine, pyrrole, pyrrolidine, pyrrolidinone, pyrroline, tetrahydropyridine, thiazolidine, thiazoline or thiomorpholine, wherein said cyclic group is unsubstituted or mono-, di- or trisubstituted independently of one another by R14,

$R^3$, $R^4$,$R^5$ or $R^6$, are independent of one another are identical or different and are

1) hydrogen atom,
2) fluorine, chlorine or bromine,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

   a) hydrogen atom,
   b) -($C_1$-$C_4$)-alkyl, wherein alkyl is unsubstituted or mono-, di- or trisubstituted independently of one another by R13, or
   c) phenyl, wherein phenyl is unsubstituted or mono-, di- or trisubstituted independently of one another by R13,
   d) -$CF_3$,
   e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(0)-0-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(0)-0- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_3$)-alkylene-O-$CH_2$-($C_1$-$C_3$)-perfluoroalkylene-$CH_2$-O-($C_0$-$C_3$)-alkyl, or

26) a residue from the following list

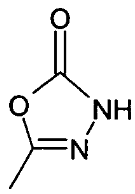
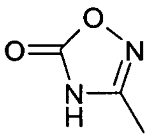
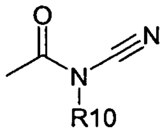
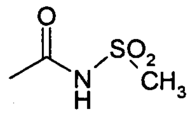
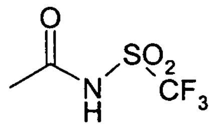
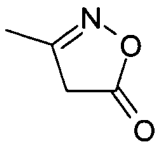
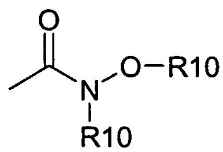
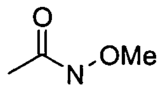
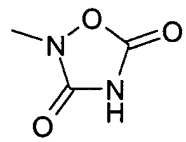
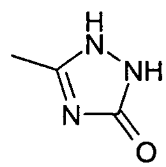
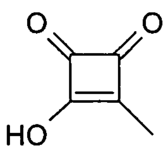
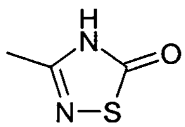
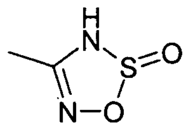
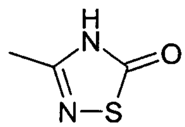
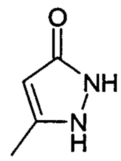
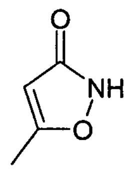
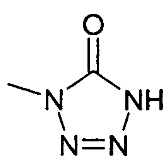
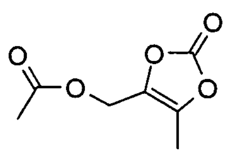
and
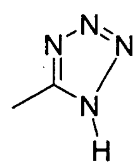

wherein Me is methyl, or

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or

R3 and R4 or R5 and R6 together with the carbon atom to which they are bonded can form a 3-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or

R3 and R5 or R4 and R6 together with the carbon atoms to which they are bonded can form a 4-to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one,

oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11 and R12 together with the nitrogen atom to which they are bonded can form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R14 is fluorine, chlorine, bromine, -OH, =O, $-(C_1-C_8)$-alkyl, $-(C_1-C_4)$-alkoxy, -NO2, -C(O)-OH, -CN,$-NH_2$, $-C(O)-O-(C_1-C_4)$-alkyl, $-(C_0-C_8)$-alkyl-$SO_2$-$(C_1-C_4)$-alkyl, $-(C_0-C_8)$-alkyl-$SO_2$-$(C_1-C_3)$-perfluoroalkyl, $-(C_0-C_8)$-alkyl-$SO_2$-$N(R^{18})$-$R^{21}$, $-C(O)-NH-(C_1-C_8)$-alkyl, $-C(O)-N-[(C_1-C_8)\text{-alkyl}]_2$, $-NR^{18}-C(O)-NH-(C_1-C_8)$-alkyl, $-C(O)-NH_2$, $-S-R^{18}$, or $-NR^{18}-C(O)-NH-[(C_1-C_8)\text{-alkyl}]_2$,

wherein $R^{18}$ and $R^{21}$ are independently from each other hydrogen atom, $-(C_1-C_3)$-perfluoroalkyl or $-(C_1-C_6)$-alkyl,

V is 1) a het residue out of the group azaindole (1H-pyrrolopyridine), azepine, azetidine, aziridine, azirine, 1,4-diazepane, 1,2-diazepine, 1,3-diazepine, 1,4-diazepine, diaziridine, diazirine, dioxazole, dioxazine, dioxole, 1 ,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine, thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, which is as defined above and wherein het is unsubstituted or mono-, di-or trisubstituted independently of one another by R14, or

2) phenyl, wherein phenyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_n-$, $-(CH_2)_m-NR^{10}-C(O)-(CH_2)_n-$, $-(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$, n and m are independently of one another identical or different and are the integers zero, 1, 2, 3, 4, 5 or 6,

M is 1) phenyl or naphthyl, wherein phenyl or naphthyl are unsubstituted or mono-, di-or trisubstituted independently of one another by R14,

2) heterocyclyl, wherein heterocyclyl is a residue out of the group which can be derived from azepane, azepine, 1,4-diazepane, 1,2-diazepine, 1,3-diazepine, 1,4-diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,
4) -O-$R^{17}$, or
5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

R11 and R12 together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, which is unsubstituted or mono-, di- or trisubstituted independently of one another by R13,

R13 is fluorine, chlorine, bromine, iodine, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_0$-$C_3$)-alkylene-O-$R^{10}$, -Si-$(CH_3)_3$, -N($R^{10}$)-S$(O)_2$-$R^{10}$, -S-$R^{10}$, -$SO_2$-$R^{10}$, -S$(O)_2$-N($R^{10}$)-$R^{20}$, -C(O)-$R^{10}$, -($C_1$-$C_8$)-alkyl, -($C_1$-$C_8$)-alkoxy, phenyl, phenyloxy-, -O-$CF_3$, -($C_1$-$C_3$)-perfluoroalkyl, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -O-R15, -NH-C(O)-NH-$R^{10}$, -NH-C(O)-O-$R^{10}$, or a residue from the following list

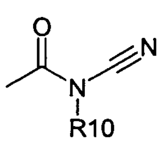 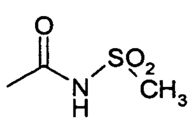 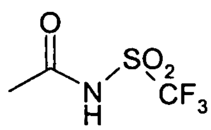 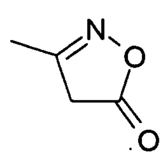 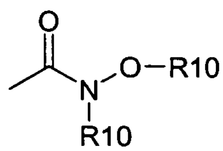

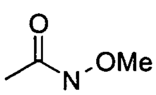 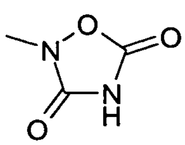 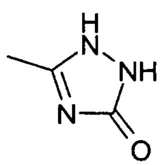 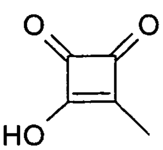 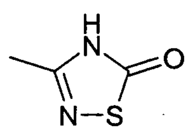 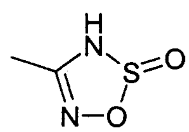

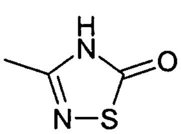 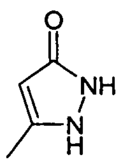 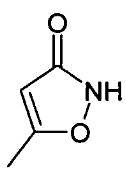 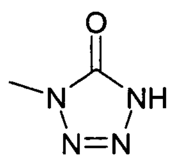 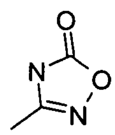 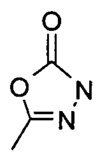 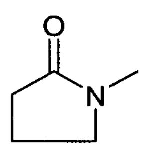

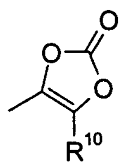 and 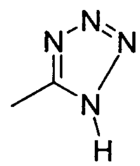 ,

| | |
|---|---|
| $R^{10}$ and $R^{20}$ are | independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl, |
| R15 and R16 are | independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and |
| R17 is | -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$, |

in all its stereoisomeric forms and mixtures thereof in any ratio, and its physiologically tolerable salts.

5) The present invention also relates to the compounds of the formula I, wherein

R0 is
1) phenyl, wherein phenyl is unsubstituted or mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or
3) a heterocyclyl out of the group imidazolyl, pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, pyrazolyl, pyrrolyl, wherein said heterocyclyl is unsubstituted or mono-, di- or trisubstituted independently of one another by R8,

R8 is
1. F, Cl, Br or J,
2. -C(O)-$NH_2$,
3. -($C_1$-$C_4$)-alkyl, wherein alkyl is unsubstituted or mono-, di- or trisubstituted independently of one another by halogen, -OH or a methoxy residue, or
4. -O-($C_1$-$C_4$)-alkyl, wherein alkyl is unsubstituted or mono-, di- or trisubstituted independently of one another by halogen or a methoxy residue,

provided that R8 is at least one F, Cl, Br, J, -C(O)-$NH_2$ or -O-($C_1$-$C_4$)-alkyl residue,

| | |
|---|---|
| Q is | a -($C_0$-$C_2$)-alkylene-C(O)-($C_0$-$C_2$)-alkylene-, -$SO_2$-, |
| $R^1$ is | hydrogen atom, -($C_1$-$C_2$)-alkyl, -($C_1$-$C_3$)-alkylene-C(O)-NH-R0, -($C_1$-$C_3$)-perfluoroalkylene, -($C_1$-$C_3$)-alkylene-C(O)-O-$R^{15}$, -($C_1$-$C_3$)-alkylene-S(O)$_2$-($C_1$-$C_3$)-alkyl or -($C_1$-$C_3$)-alkylene-S(O)$_2$-N($R^{4'}$)-$R^{5'}$, wherein $R^{4'}$ and $R^{5'}$ are independent of one another are identical or different and are hydrogen atom or -($C_1$-$C_4$)-alkyl, |
| $R^2$ is | a direct bond or -($C_1$-$C_2$)-alkylene, |
| $R^1$-N-$R^2$-V | can form a 4- to 7- membered cyclic group out of the group azetidine, azetidinone, 2,3 dihydroindole, indole, piperidine, piperazine, pyridine, pyrrole, pyrazole, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14, |
| R14 is | fluorine, chlorine, -OH, =O, -($C_1$-$C_8$)-alkyl, -C(O)-OH, -CN, -$NH_2$, -C(O)-O-($C_1$-$C_4$)-alkyl, -C(O)-NH-($C_1$-$C_8$)-alkyl, -C(O)-N-[($C_1$-$C_8$)-alkyl]$_2$, -C(O)-$NH_2$ or -N($R^{18}$)-$R^{21}$, wherein $R^{18}$ and $R^{21}$ are inde- |

pendently from each other hydrogen atom, -($C_1$-$C_3$)-perfluoroalkyl or -($C_1$-$C_4$)-alkyl,

V is 1) a cyclic residue out of the group containing compounds which are derived from azaindole (1H-pyrrolopyridine), aziridine, azirine, azetidine, azetidinone, 1,4-diazepane, pyrrole, pyrrolidine, pyridonyl, imidazole, pyrazole, 1,2,3-triazole, 1,2,4-triazole, tetrazole, pyridine, pyrimidine, pyridazine, pyrazine, 1,2,3-triazine, 1,2,4-triazine, 1,3,5-triazine, tetrazine, tetrazole, azepine, diazirine, 1,2-diazepine, 1,3-diazepine, 1,4-diazepine, pyridine, pyrazine, pyrimidine, pyridazine, piperidine, piperazine, pyrrolidinone, ketopiperazine, furan, pyran, dioxole, 1,4-oxazepane, oxazole, isoxazole, 2-isoxazoline, isoxazolidine, morpholine, oxirane, oxaziridine, 1,3-dioxolene, 1,3-dioxolane, 1,2-oxazine, 1,3-oxazine, 1,4-oxazine, oxaziridine, thiophene, thiopyran, thietan, thiazole, isothiazole, isothiazoline, isothiazolidine, 1 ,2-oxathiolan, thiadiazole, thiopyran, 1,2-thiazine, 1,3-thiazole, 1,3-thiazine, 1,4-thiazine, thiadiazine or thiomorpholine,

wherein said cyclic residue is unsubstituted or mono-, di- or trisubstituted independently of one another by R14, or

2) phenyl, wherein phenyl is unsubstituted or mono-, di- or trisubstituted independently of one another by R14,

G is a direct bond, -$(CH_2)_m$-, -$(CH_2)_m$-C(O)-$NR^{10}$-$(CH_2)_n$-, -$(CH_2)_m$-C(O)-$(CH_2)_n$- or -$(CH_2)_m$-$NR^{10}$-

n and m are independently of one another identical or different and are the integers zero, 1, 2, 3 or 4,

M is heterocyclyl, wherein heterocyclyl is a residue out of the group which can be derived from azepane, azepine, 1,4-diazepane, 1,2-diazepine, 1,3-diazepine, 1,4-diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]- oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro- pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

provided that M contains or is substituted with at least with one oxo-residue,

$R^3$, $R^4$, $R^5$ or $R^6$ are independent of one another are identical or different and are

1) hydrogen atom,
2) fluorine, chlorine or bromine,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

a) hydrogen atom,
b) -($C_1$-$C_4$)-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$ or
e) -$CHF_2$,

7) -$NO_2$,
8) -CN,
9) -$SO_S$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,

15) $-NR^{10}-SO_2-R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(0)-0-C(R15, R16)-0-C(0)-R17,
19) $-(C_0-C_2)$alkylene-C(O)-O-$(C_2-C_4)$-alkylene-O-C(O)-O-$(C_1-C_6)$-alkyl,
20) -C(0)-0- C(R15, R16)-O-C(O)-O-R17,
21) $-(C_1-C_4)$-alkylene-$(C_6-C_{14})$-aryl, wherein aryl is mono-, di- or trisubstituted independently of one another by R13,
22) $-(C_1-C_4)$-alkylene-$(C_4-C_{15})$-heterocyclyl, wherein heterocyclyl is unsubstituted or mono-, di- or trisubstituted independently of one another by R13
23) $-(C_1-C_4)$-alkylene-$(C_3-C_8)$-cycloalkyl, wherein cycloalkyl is unsubstituted or mono-, di- or trisubstituted independently of one another by R13,
24) $-(C_1-C_4)$-alkylene-het, wherein het is unsubstituted or mono-, di- or trisubstituted independently of one another by R13,
25) $-(C_0-C_3)$-alkylene-O-$CH_2$-$(C_1-C_3)$-perfluoroalkylene-$CH_2$-O-$(C_0-C_3)$-alkyl, or
26) a residue from the following list

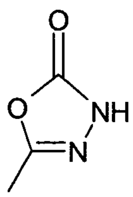
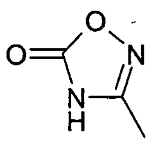
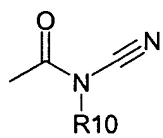
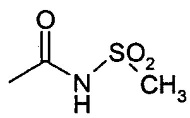
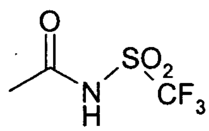

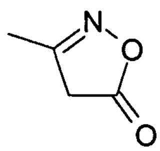
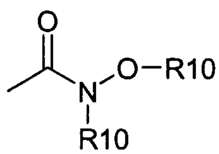
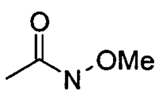
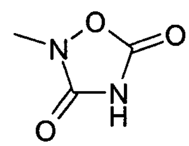
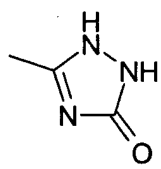

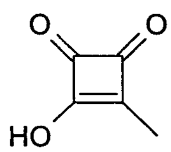
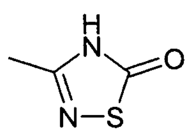
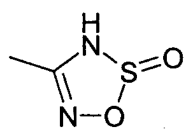
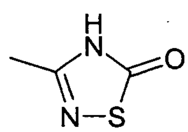
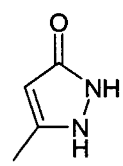

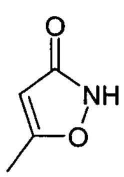
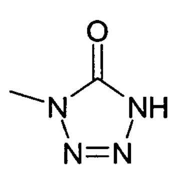
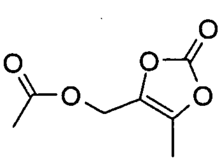
and
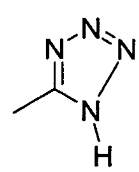

wherein Me is methyl, or

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,
4) -O-$R^{17}$, or
5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

$R^{11}$ and $R^{12}$ together with the nitrogen atom to which they are bonded can form a ring selected out of the group azepine, azetidine, 1,4-diazepane, dioxazole, dioxazine, 1,2- diazepine, 1,3-diazepine, 1,4-diazepine, imidazole, imidazoline, imidazolidine, isothiazole, isothiazolidine, isothiazoline, isoxazole, isoxazoline, isoxazolidine, 2- isoxazoline, ketopiperazine, morpholine, [1,4]-oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_0$-$C_3$)-alkylene-O-$R^{10}$, -Si-($CH_3$)$_3$, -N($R^{10}$)-S(O)$_2$-$R^{10}$, -S-$R^{10}$, -$SO_2$-$R^{10}$, -S(O)$_2$-N($R^{10}$)-$R^{20}$, -C(O)-$R^{10}$, -($C_1$-$C_8$)-alkyl, -($C_1$-$C_8$)-alkoxy, phenyl, phenyloxy-, -O-$CF_3$, -($C_1$-$C_3$)-perfluoroalkyl, -NH-C(O)-NH-$R^{10}$, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -0-R15, -NH-C(O)-O-$R^{10}$, or a residue from the following list

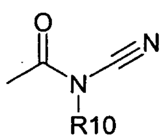 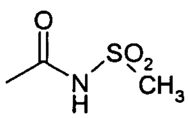 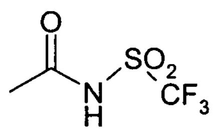 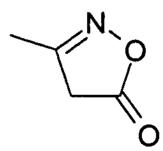 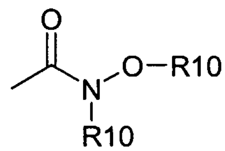

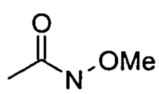 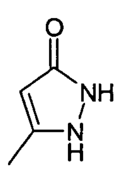 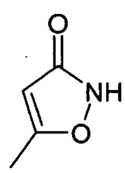 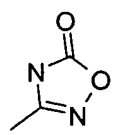 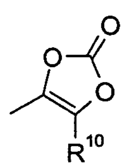 and, 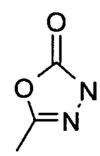

wherein Me is methyl,

$R^{10}$ and $R^{20}$ are independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts.

6) The present invention also relates to the compounds of the formula I, wherein

R0 is a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8,

R8 is 1. F, Cl, Br, J,

2. $-C(O)-NH_2$,
3. $-(C_1-C_4)$-alkyl, wherein alkyl is unsubstituted or mono-, di- or trisubstituted independently of one another by halogen, -OH or a methoxy residue, or
4. $-O-(C_1-C_4)$-alkyl, wherein alkyl is unsubstituted or mono-, di- or trisubstituted independently of one another by halogen or a methoxy residue,

provided that R8 is at least one F, Cl, Br, J, $-C(O)-NH_2$ or $-O-(C_1-C_4)$-alkyl residue,

Q is a $-(C_0-C_2)$-alkylene-$C(O)$-$(C_0-C_2)$-alkylene-, $-SO_2-$,

$R^1$ is hydrogen atom or $-(C_1-C_2)$-alkyl,

$R^2$ is a direct bond or $-(C_1-C_2)$-alkylen, or

$R^1$-N-$R^2$-V can form a 4- to 7- membered cyclic group out of the group azepine, 1,2-diazepine, 1,3-diazepine, 1,4-diazepine, 2,3 dihydroindole, indole, indazole, isothiazole, isoxazole, isoxazolidine, 2-isoxazoline, ketopiperazine, morpholine, oxazole, oxepane, piperidine, piperazine, pyridine, pyrimidine, pyrrole, pyrazole, pyrrolidine, pyrrolidinone, 1,2,3-triazine, 1,2,4-triazine, 1,3,5-triazine, 1,2,3-triazole, 1,2,4-triazole, tetrazine, tetrazole, thiadiazole, thiazole or thiomorpholine, wherein said cyclic group is unsubstituted or mono-, di- or trisubstituted independently of one another by R14,

R14 is fluoro, chlorine, =O, $-(C_1-C_4)$-alkyl or $-NH_2$,

V is 1. a cyclic residue out of the group containing compounds, which are derived from azaindolyl (1H-pyrrolopyridyl), azetidine, azepine, aziridine, azirine, 1,4-diazepane, 1,2-diazepine, 1,3-diazepine, 1,4-diazepine, diazirine, 1,3-dioxolane, dioxazole, furan, imidazole, isoquinoline, isothiazole, isothiazolidine, isothiazoline, isoxazole, 2-isoxazoline, isoxazolidine, ketopiperazine, morpholine, 1,2-oxazine, 1,3-oxazine, 1,4-oxazine, oxazole, 1 ,2-oxathiolan, piperidine, pyran, pyrazine, pyrazole, pyridazine, piperazine, pyridine, pyridone, pyrimidine, pyrrole, pyrrolidine, pyrrolidinone, quinazoline, quinoline, tetrazine, tetrazole, thiadiazine, thiadiazole, 1,2-thiazine, 1,3-thiazine, 1,4-thiazine, 1,3-thiazole, thietan, thiomorpholine, thiophene, thiopyran, 1,2,3-triazine, 1,2,4-triazine, 1,3,5-triazine, 1,2,3-triazole or 1,2,4-triazole, wherein said cyclic residue is unsubstituted or mono-, di- or trisubstituted independently of one another by R14, or
2. phenyl, wherein phenyl is unsubstituted or mono-, di- or trisubstituted independently of one another by R14,

G is a direct bond, $-(CH_2)_m-$, $-(CH_2)_m-C(O)-NR^{10}-(CH_2)_n-$ or $-(CH_2)_m-C(O)-(CH_2)_n-$, n and m are independently of one another identical or different and are the integers zero, 1, 2, 3 or 4,

M is heterocyclyl, wherein heterocyclyl is a residue out of the group which can be derived from azepane, azepine, 1,4-diazepane, 1,2-diazepine, 1,3-diazepine, 1,4-diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

provided that M contains or is substituted with at least with one oxo-residue,

$R^3$, $R^4$, $R^5$ or $R^6$, are independent of one another are identical or different and are

1) hydrogen atom,
2) fluorine, chlorine or bromine,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$ or
e) $CHF_2$,

7) -CN,
8) $-(C_1-C_4)$-alkylene-$(C_4-C_{15})$-heterocyclyl, wherein heterocyclyl is as defined above and is unsubstituted or mono-, di- or trisubstituted independently of one another by R13,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-NR^{10}-SO_2-R^{10}$,
16) $-(C_1-C_4)$-alkylene-het, wherein het is as defined above and is unsubstituted or mono-, di- or trisubstituted independently of one another by R13,
17) $-(C_0-C_2)$alkylene-C(O)-O-$(C_2-C_4)$-alkylene-O-C(O)-$(C_1-C_4)$-alkyl,
18) -C(0)-0-C(R15, R16)-O-C(O)-R17,
19) $-(C_0-C_2)$alkylene-C(O)-O-$(C_2-C_4)$-alkylene-O-C(O)-O-$(C_1-C_6)$-alkyl,
20) -C(0)-0- C(R15, R16)-O-C(O)-O-R17,
21) $-(C_1-C_4)$-alkylene-$(C_6-C_{14})$-aryl, wherein aryl is as defined above and is mono-, di- or trisubstituted independently of one another by R13,
22) $-(C_1-C_4)$-alkylene-$(C_3-C_8)$-cycloalkyl, wherein cycloalkyl is unsubstituted or mono-, di- or trisubstituted independently of one another by R13,
23) $-(C_0-C_3)$-alkylene-O-$CH_2$-$CF_2$-$CH_2$-O-$(C_0-C_3)$-alkyl,
24) $-(C_0-C_3)$-alkylene-O-$CH_2$-$CF_2$-$CF_2$-$CH_2$-O-$(C_0-C_3)$-alkyl,
25) $-(C_0-C_3)$-alkylene-O-$CH_2$-$(C_1-C_3)$-perfluoroalkylene-$CH_2$-OH, or
26) a residue from the following list

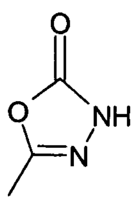 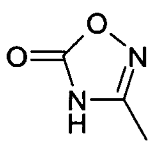 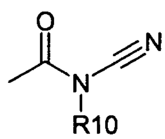 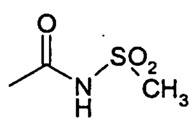 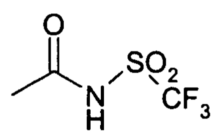

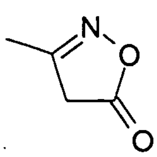 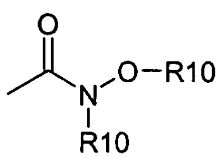 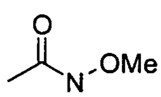 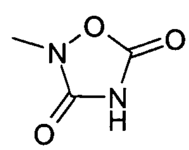 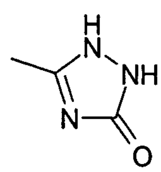

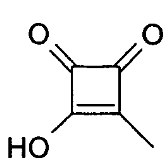 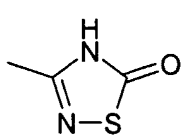 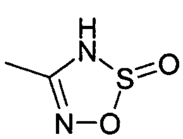 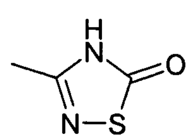 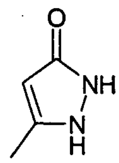 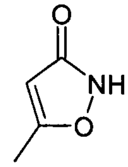

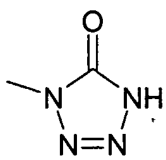
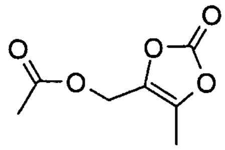
and
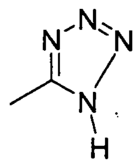
,

wherein Me is methyl, if two -OR19 residues are attached to adjacent atoms they can form together with the atoms which they are attached to a 1,3-dioxole ring or a 2,3-dihydro-[1,4] dioxine ring, which is substituted one, two, three or four times by R13,

provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or

R11 and R12 are independently of one another identical or different and are

1) hydrogen atom,
2) $-(C_1-C_6)$-alkyl, wherein alkyl is unsubstituted or mono-, di- or trisubstituted independently of one another by R13,
3) $-(C_0-C_6)$-alkyl-$(C_6-C_{14})$-aryl, wherein aryl is as defined above and wherein alkyl and aryl are independently from one another unsubstituted or mono-, di- or trisubstituted by R13,
4) $-O-R^{17}$, or
5) $-(C_0-C_6)$-alkyl-$(C_4-C_{15})$-heterocyclyl, wherein alkyl and heterocyclyl is as defined above and independently from one another are unsubstituted or mono-, di- or trisubstituted by R13, or

$R^{11}$ and $R^{12}$ together with the nitrogen atom to which they are bonded can form a ring selected out of the group azepine, azetidine, 1,4-diazepane, dioxazole, dioxazine, 1,2- diazepine, 1,3-diazepine, 1,4-diazepine, imidazole, imidazoline, imidazolidine, isothiazole, isothiazolidine, isothiazoline, isoxazole, isoxazoline, isoxazolidine, 2- isoxazoline, ketopiperazine, morpholine, [1,4]-oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, $-NO_2$, -CN, =O, -OH, $-CF_3$, $-C(O)-O-R^{10}$, $-C(O)-N(R^{10})-R^{20}$, $-N(R^{10})-R^{20}$, $-(C_0-C_3)$-alkylene-$O-R^{10}$, $-Si-(CH_3)_3$, $-N(R^{10})-S(O)_2-R^{10}$, $-S-R^{10}$, $-SO_2-R^{10}$, $-S(O)_2-N(R^{10})-R^{20}$, $-C(O)-R^{10}$, $-(C_1-C_8)$-alkyl, $-(C_1-C_8)$-alkoxy, phenyl, phenyloxy-, $-O-CF_3$, $-(C_1-C_3)$- perfluoroalkyl, $-NH-C(O)-NH-R^{10}$, $-(C_0-C_4)$-alkyl-C(O)-O-C(R15, R16)-O-C(O)-R17, $-(C_1-C_4)$-alkoxy-phenyl, $-(C_0-C_4)$-alkyl-C(O)-O-C(R15, R16)-0-C(0)-0-R17, -O-R15, $-NH-C(O)-O-R^{10}$, or a residue from the following list

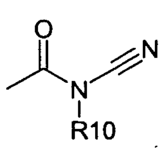
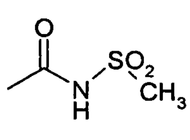
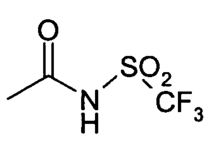
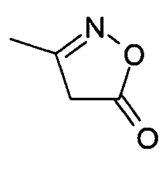
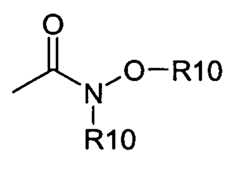

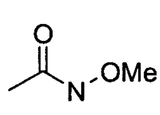
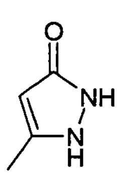
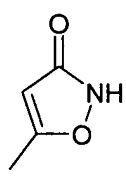
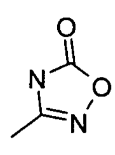
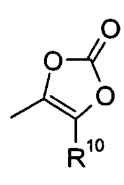
and,
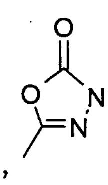

wherein Me is methyl,

$R^{10}$ and $R^{20}$ are independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts.

7) The present invention also relates to the compounds of the formula I, which are
5-Chloro-thiophene-2-carboxylic acid {2-[4-(3-oxo-morpholin-4-yl)-phenylcarbamoyl]-ethyl}-amide,
5-Chloro-thiophene-2-carboxylic acid {2-[4-(2-oxo-2H-pyrazin-1-yl)-phenylcarbamoyl]- ethyl}-amide,
4-Chloro-N-{2-[4-(3-oxo-morpholin-4-yl)-phenylcarbamoyl]-ethyl}-benzamide,
5-Chloro-thiophene-2-carboxylic acid {3-oxo-3-[5-(2-oxo-2H-pyrazin-1-yl)-2,3-dihydro- indol-1-yl]-propyl}-amide,
5-Chloro-thiophene-2-carboxylic acid {3-oxo-3-[5-(2-oxo-2H-pyrazin-1-yl)-indol-1-yl]- propyl}-amide,
5-Chloro-thiophene-2-carboxylic acid {2-[2-fluoro-4-(2-oxo-2H-pyrazin-1-yl)- phenylcarbamoyl]-ethyl}-amide,
5-Chloro-thiophene-2-carboxylic acid {2-[4-(2-dimethylaminomethyl-imidazol-1-yl)-2-fluoro-phenylcarbamoyl]-ethyl}-amide,
3-(5-Chloro-thiophene-2-sulfonylamino)-N-[4-(3-oxo-morpholin-4-yl)-phenyl]- propionamide,
5-Chloro-thiophene-2-carboxylic acid {2-[4-(3,3-dioxo-3$16-[1,3,4]oxathiazinan-4-yl)-phenylcarbamoyl]-ethyl}-amide,
5-Chloro-thiophene-2-carboxylic acid {2,2-difluoro-2-[4-(2-oxo-2H-pyrazin-1-yl)-phenylcarbamoyl]-ethyl}-amide,
5-Chloro-thiophene-2-carboxylic acid {2,2-difluoro-2-[4-(3-oxo-morpholin-4-yl)-phenylcarbamoyl]-ethyl}-amide.

In general, the meaning of any group, residue, heteroatom, number etc., which can occur more than once in the compounds of the formula I, is independent of the meaning of this group, residue, heteroatom, number etc. in any other occurrence. All groups, residues, heteroatoms, numbers etc., which can occur more than once in the compounds of the formula I, can be identical or different.

As used herein, the term alkyl is to be understood in the broadest sense to mean hydrocarbon residues which can be linear, i. e. straight-chain, or branched and which can be acyclic or cyclic residues or comprise any combination of acyclic and cyclic subunits. Further, the term alkyl as used herein expressly includes saturated groups as well as unsaturated groups which latter groups contain one or more, for example one, two or three, double bonds and/or triple bonds, provided that the double bonds are not located within a cyclic alkyl group in such a manner that an aromatic system results. All these statements also apply if an alkyl group occurs as a substituent on another residue, for example in an alkyloxy residue, an alkyloxycarbonyl residue or an arylalkyl residue. Examples of "-($C_1$-$C_8$)-alkyl" or "-($C_1$-$C_8$)-alkylene" are alkyl residues containing 1, 2, 3, 4, 5, 6, 7 or 8 carbon atoms are methyl, methylene, ethyl, ethylene. propyl, propylene, butyl, butylene, pentyl, pentylene, hexyl, heptyl or octyl, the n-isomers of all these residues, isopropyl, isobutyl, 1-methylbutyl, isopentyl, neopentyl, 2,2-dimethylbutyl, 2-methylpentyl, 3-methylpentyl, isohexyl, sec-butyl, tBu, tert-pentyl, sec-butyl, tert-butyl or tert-pentyl. The term "-($C_0$-$C_6$)-alkyl" or "-($C_0$-$C_8$)-alkylene" is a hydrocarbon residues containing 1, 2, 3, 4, 5, 6, 7 or 8 carbon atoms. The term "-$C_0$-alkyl" or "-$C_0$-alkylene" is a covalent bond.

Unsaturated alkyl residues are, for example, alkenyl residues such as vinyl, 1-propenyl, 2-propenyl (= allyl), 2-butenyl, 3-butenyl, 2-methyl-2-butenyl, 3-methyl-2-butenyl, 5-hexenyl or 1,3-pentadienyl, or alkynyl residues such as ethynyl, 1-propynyl, 2-propynyl (= propargyl) or 2-butynyl. Alkyl residues can also be unsaturated when they are substituted.

Examples of -($C_3$-$C_8$)-cycloalkyl cyclic alkyl residues are cycloalkyl residues containing 3, 4, 5, 6, 7 or 8 ring carbon atoms like cyclopropyl, cyclobutyl, cyclopentyl, cyclohexyl, cyloheptyl or cyclooctyl, which can also be substituted and/or unsaturated. Unsaturated cyclic alkyl groups and unsaturated cycloalkyl groups like, for example, cyclopentenyl or cyclohexenyl can be bonded via any carbon atom.

Of course, a cyclic alkyl group has to contain at least three carbon atoms, and an unsaturated alkyl group has to contain at least two carbon atoms. Thus, a group like ($C_1$-$C_8$)-alkyl is to be understood as comprising, among others, saturated acyclic ($C_1$-$C_8$)-alkyl, ($C_3$-$C_6$)-cycloalkyl, and unsaturated ($C_2$-$C_8$)-alkyl like ($C_2$-$C_8$)-alkenyl or ($C_2$-$C_8$)-alkynyl. Similarly, a group like ($C_1$-$C_4$)-alkyl is to be understood as comprising, among others, saturated acyclic ($C_1$-$C_4$)-alkyl, and unsaturated ($C_2$-$C_4$)-alkyl like ($C_2$-$C_4$)-alkenyl or ($C_2$-$C_4$)-alkynyl.

The terms "a monocyclic or bicyclic 6- to 14-membered aryl" or "-($C_6$-$C_{14}$)-aryl" are understood as meaning aromatic hydrocarbon radicals containing from 6 to 14 carbon atoms in the ring. Examples of -($C_6$-$C_{14}$)-aryl radicals are phenyl, naphthyl, for example 1-naphthyl and 2-naphthyl, biphenylyl, for example 2-biphenylyl, 3-biphenylyl and

4-biphenylyl, anthryl or fluorenyl. Biphenylyl radicals, naphthyl radicals and, in particular, phenyl radicals are preferred aryl radicals.

The terms "mono- or bicyclic 4- to 15-membered heterocyclyl" or "-($C_4$-$C_{15}$)-heterocyclyl" refer to heterocycles in which one or more of the 4 to 15 ring carbon atoms are replaced by heteroatoms such as nitrogen, oxygen or sulfur. Examples are acridinyl, azaindole (1 H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4] oxathiazinyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1 , 2,3-oxadiazolyl, 1,2,4-oxadiazolyl, 1,2,5-oxadiazolyl, 1,3,4-oxadiazolyl, 1 ,2-oxa-thiepanyl, 1 ,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1 ,2,3-thiadiazolyl, 1 ,2,4-thiadiazolyl, 1,2,5-thiadiazolyl, 1,3,4-thiadiazolyl, thianthrenyl, 1 ,2-thiazinyl, 1,3-thiazinyl, 1 ,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1 ,2,3-triazinyl, 1,2,4-triazinyl, 1,3,5-triazinyl, 1,2,3-triazolyl, 1,2,3-triazolyl, 1 ,2,4-triazolyl, 1,2,5-triazolyl, 1 ,3,4-triazolyl and xanthenyl.

Preferred are heterocyclyls, such as benzimidazolyl, 1,3-benzodioxolyl, benzofuranyl, benzothiazolyl, benzothiophenyl, benzoxazolyl, chromanyl, cinnolinyl, 2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, 2-thienyl and 3-thienyl.

Also preferred are:

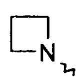 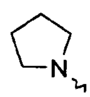 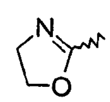 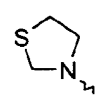 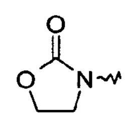 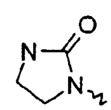

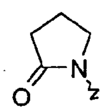 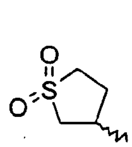 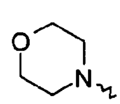 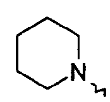 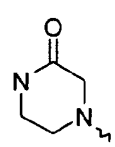 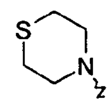

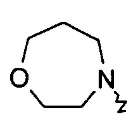 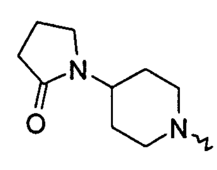 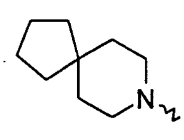

The terms "het" or "a 3- to 7-membered cyclic residue, containing up to 1, 2, 3 or 4 heteroatoms" refer to structures of heterocycles which can be derived from compounds such as azepine, azetidine, aziridine, azirine, 1,4 diazepane, 1,2-diazepine, 1,3-diazepine, 1,4-diazepine, diaziridine, diazirine, dihydroimidazolone, dioxazole, dioxazine, dioxole, 1,3-dioxolene, 1,3-dioxolane, furan, imidazole, imidazoline, imidazolidine, imidazolidinone, isothiazole, isothiazolidine, isothiazoline, isoxazole, isoxazoline, isoxazolidine, 2-isoxazoline, ketomorpholine, ketopiperazine,

morpholine, 1,2-oxa-thiepane, 1,2-oxathiolane, 1,4-oxazepane, 1,2-oxazine, 1,3-oxazine, 1,4-oxazine, oxazolone, oxazole, [1,3,4]oxathiazinane 3,3-dioxide, oxaziridine, oxazolidinone, oxetan, oxirane, piperazine, piperidine, pyran, pyrazine, pyrazole, pyrazin-2-one, piperazin-2-one, pyrazoline, pyrazolidine, pyridazine, pyridine, pyridinone, pyrimidine, pyrimidine-2,4-dione, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1 ,3-thiazole, thiazole, thiazolidine, thiazoline, thienyl, thietan, thiomorpholine, thiomorpholine 1,1-dioxide thiopyran, 1,2,3-triazine, 1,2,4-triazine, 1,3,5-triazine, 1,2,3-triazole or 1,2,4-triazole.

The term " $R^1$-N-$R^2$-V can form a 4- to 8-membered cyclic group " or "$R^{11}$ and $R^{12}$ together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen" refer to structures of heterocycles which can be derived from compounds such as azepane, azepine, azetidine, dioxazole, dioxazine, 1,4-diazepane, 1,2-diazepine, 1,3-diazepine, 1,4-diazepine, 2,3 dihydroindole, imidazole, imidazoline, imidazolidine, indole, isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole.

The term "$R^{15}$ and $R^{16}$ together with the carbon atom to which they are bonded can form a 3-to 6 membered carbocyclic ring" refer to structures, which can be derived from compounds such as cyclopropyl, cyclobutyl, cyclopentyl or cyclohexyl.

The term "R3 and R4 or R5 and R6 together with the carbon atom to which they are bonded can form a 3- to 8-membered ring, containing zero, 1, 2, 3 or 4 heteroatoms chosen from nitrogen, sulfur or oxygen" refers to structures of carbocycles or heterocycles which can be derived from compounds such as azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3] oxazocan-2-one, oxetane, oxocane, oxocan-2-one, piperazine, piperidine, pyran, 5,6,7,8-tetrahydro-1H-azocin-2-one, thiomorpholine or tetrahydrofurane. The term "R3 and R5 or R4 and R6 together with the carbon atoms to which they are bonded can form a 4- to 8-membered ring, containing zero, 1, 2, 3 or 4 heteroatoms chosen from nitrogen, sulfur or oxygen" refers to structures of carbocycles or heterocycles which can be derived from compounds such as azetidine, azocane, azocane-2-one, cyclobutyl, cyloheptyl cyclohexyl, cyclooctane, cyclooctene, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane.

The term "oxo-residue" or "=0" refers to residues such as carbonyl (-C(O)-) or nitroso (-N=O).

The fact that many of the before-listed names of heterocycles are the chemical names of unsaturated or aromatic ring systems does not imply that the, the 4-15 membered mono- or polycyclic group could only be derived from the respective unsaturated ring system. The names here only serve to describe the ring system with respect to ring size and the number of the heteroatoms and their relative positions. As explained above, the 4-15 membered mono- or polycyclic group can be saturated or partially unsaturated or aromatic, and can thus be derived not only from the before-listed heterocycles themselves but also from all their partially or completely hydrogenated analogues and also from their more highly unsaturated analogues if applicable. As examples of completely or partially hydrogenated analogues of the before-listed heterocycles from which this group may be derived the following may be mentioned: pyrroline, pyrrolidine, tetrahydrofuran, tetrahydrothiophene, dihydropyridine, tetrahydropyridine, piperidine, 1,3-dioxolane, 2-imidazoline, imidazolidine, 4,5-dihydro-1,3-oxazol, 1,3-oxazolidine, 4,5-dihydro-1,3-thiazole, 1,3-thiazolidine, perhydro-1,4-dioxane, piperazine, perhydro-1,4-oxazine (= morpholine), perhydro-1,4-thiazine (= thiomorpholine), perhydroazepine, indoline, isoindoline, 1,2,3,4-tetrahydroquinoline, 1 ,2,3,4-tetrahydroisoquinoline, etc.

The 3-7 membered monocyclic group may be bonded via any ring carbon atom, and in the case of nitrogen heterocycles via any suitable ring nitrogen atom. Thus, for example, a pyrrolyl residue can be 1-pyrrolyl, 2-pyrrolyl or 3-pyrrolyl, a pyrrolidinyl residue can be pyrrolidin-1-yl (= pyrrolidino), pyrrolidin-2-yl or pyrrolidin-3-yl, a pyridinyl residue can be pyridin-2-yl, pyridin-3-yl or pyridin-4-yl, a piperidinyl residue can be piperidin-1-yl (= piperidino), piperidin-2-yl, piperidin-3-yl or piperidin-4-yl. Furyl can be 2-furyl or 3-furyl, thienyl can be 2-thienyl or 3-thienyl, imidazolyl can be imidazol-1-yl, imidazol-2-yl, imidazol-4-yl or imidazol-5-yl, 1,3-oxazolyl can be 1 ,3-oxazol-2-yl, 1,3-oxazol-4-yl or 1 , 3-oxazol-5-yl, 1 ,3-thiazolyl can be 1,3-thiazol-2-yl, 1,3-thiazol-4-yl or 1,3-thiazol-5-yl, pyrimidinyl can be pyrimidin-2-yl, pyrimidin-4-yl (= 6-pyrimidinyl) or 5-pyrimidinyl, piperazinyl can be piperazin-1-yl (= piperazin-4-yl = piperazino) or piperazin-2-yl. Unless stated otherwise, and irrespective of any specific substituents bonded to the 3-7 membered monocyclic group or any other heterocyclic groups which are indicated in the definition of the compounds of the formula

I can be unsubstituted or substituted on ring carbon atoms with one or more, for example one, two, three, four or five, identical or different substituents like $(C_1-C_8)$-alkyl, in particular $(C_1-C_4)$-alkyl, $(C_1-C_8)$-alkyloxy, in particular $(C_1-C_4)$-alkyloxy, $(C_1-C_4)$-alkylthio, halogen, nitro, amino, $((C_1-C_4)$-alkyl)carbonylamino like acetylamino, trifluoromethyl, trifluoromethoxy, hydroxy, oxo, hydroxy-$(C_1-C_4)$-alkyl such as, for example, hydroxymethyl or 1-hydroxyethyl or 2-hydroxyethyl, methylenedioxy, ethylenedioxy, formyl, acetyl, cyano, aminosulfonyl, methylsulfonyl, hydroxycarbonyl, aminocarbonyl, $(C_1-C_4)$-alkyloxycarbonyl, optionally substituted phenyl, optionally substituted phenoxy, benzyl optionally substituted in the phenyl group, benzyloxy optionally substituted in the phenyl group, etc. The substituents can be present in any desired position provided that a stable molecule results. Of course an oxo group cannot be present in an aromatic ring. Each suitable ring nitrogen atom in the 3-7 membered monocyclic group can independently of each other be unsubstituted, i. e. carry a hydrogen atom, or can be substituted, i. e. carry a substituent like $(C_1-C_8)$-alkyl, for example $(C_1-C_4)$-alkyl such as methyl or ethyl, optionally substituted phenyl, phenyl-$(C_1-C_4)$-alkyl, for example benzyl, optionally substituted in the phenyl group, hydroxy-$(C_2-C_4)$-alkyl such as, for example 2-hydroxyethyl, acetyl or another acyl group, methylsulfonyl or another sulfonyl group, aminocarbonyl, $(C_1-C_4)$-alkyloxycarbonyl, etc. In general, in the compounds of the formula I nitrogen heterocycles can also be present as N-oxides or as quaternary salts. Ring sulfur atoms can be oxidized to the sulfoxide or to the sulfone. Thus, for example a tetrahydrothienyl residue may be present as S, S-dioxotetrahydrothienyl residue or a thiomorpholinyl residue like thiomorpholin-4-yl may be present as 1-oxo-thiomorpholin-4-yl or 1,1-dioxo-thiomorpholin-4-yl. A substituted 3-7 membered monocyclic group that can be present in a specific position of the compounds of formula I can independently of other groups be substituted by substituents selected from any desired subgroup of the substituents listed before and/or in the definition of that group.

The term "-$(C_1-C_3)$-perfluoroalkyl" is a partial or totally fluorinated alkyl-residue, which can be derived from residues such as $-CF_3$, $-CHF_2$, $-CH_2F$, $-CHF-CF_3$, $-CHF-CHF_2$, $-CHF-CH_2F$, $-CH_2-CF_3$, $-CH_2-CHF_2$, $-CH_2-CH_2F$, $-CF_2-CF_3$, $-CF_2-CHF_2$, $-CF_2-CH_2F$, $-CH_2-CHF-CF_3$, $-CH_2-CHF-CHF_2$, $-CH_2-CHF-CH_2F$, $-CH_2-CH_2-CF_3$, $-CH_2-CH_2-CHF_2$, $-CH_2-CH_2-CH_2F$, $-CH_2-CF_2-CF_3$, $-CH_2-CF_2-CHF_2$, $-CH_2-CF_2-CH_2F$, $-CHF-CHF-CF_3$, $-CHF-CHF-CHF_2$, $-CHF-CHF-CH_2F$, $-CHF-CH_2-CF_3$, $-CHF-CH_2-CHF_2$, $-CHF-CH_2-CH_2F$, $-CHF-CF_2-CF_3$, $-CHF-CF_2-CHF_2$, $-CHF-CF_2-CH_2F$, $-CF_2-CHF-CF_3$, $-CF_2-CHF-CHF_2$, $-CF_2-CHF-CH_2F$, $-CF_2-CH_2-CF_3$, $-CF_2-CH_2-CHF_2$, $-CF_2-CH_2-CH_2F$, $-CF_2-CF_2-CF_3$, $-CF_2-CF_2-CHF_2$ or $-CF_2-CF_2-CH_2F$.

The term "-$(C_1-C_3)$-perfluoroalkylene" is a partial or totally fluorinated alkylene-residue, which can be derived from residues such as $-CF_2-$, $-CHF-$, $-CHF-CHF_2-$, $-CHF-CHF-$, $-CH_2-CF_2-$, $-CH_2-CHF-$, $-CF_2-CF_2-$, $-CF_2-CHF-$, $-CH_2-CHF-CF_2-$, $-CH_2-CHF-CHF-$, $-CH_2-CH_2-CF_2-$, $-CH_2-CH_2-CHF$, $-CH_2-CF_2-CF_2-$, $-CH_2-CF_2-CHF-$, $-CHF-CHF-CF_2-$, $-CHF-CHF-CHF-$, $-CHF-CH_2-CF_2-$, $-CHF-CH_2-CHF-$, $-CHF-CF_2-CF_2-$, $-CHF-CF_2-CHF-$, $-CF_2-CHF-CF_2-$, $-CF_2-CHF-CHF-$, $-CF_2-CH_2-CF_2-$, $-CF_2-CH_2-CHF-$, $-CF_2-CF_2-CF_2-$, or $-CF_2-CF_2-CHF$.

Halogen is fluorine, chlorine, bromine or iodine, preferably fluorine, chlorine or iodine, particularly preferably chlorine or fluorine.

Optically active carbon atoms present in the compounds of the formula I can independently of each other have R configuration or S configuration. The compounds of the formula I can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of the formula I and it comprises all ratios of the stereoisomers in the mixtures. In case the compounds of the formula I can be present as E isomers or Z isomers (or cis isomers or trans isomers) the invention relates both to pure E isomers and pure Z isomers and to E/Z mixtures in all ratios. The invention also comprises all tautomeric forms of the compounds of the formula I.

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds of the formula I can also be obtained by employing stereochemically uniform starting materials or by using stereoselective reactions.

Physiologically tolerable salts of the compounds of formula I are nontoxic salts that are physiologically acceptable, in particular pharmaceutically utilizable salts. Such salts of compounds of the formula I containing acidic groups, for example a carboxyl group COOH, are for example alkali metal salts or alkaline earth metal salts such as sodium salts, potassium salts, magnesium salts and calcium salts, and also salts with physiologically tolerable quaternary ammonium ions such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in the compounds of the formula I for example amino groups or guanidino groups, form acid addition salts, for example with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluenesulfonic acid. Compounds of the formula

I which simultaneously contain a basic group and an acidic group, for example a guanidino group and a carboxyl group, can also be present as zwitterions (betaines) which are likewise included in the present invention.

Salts of compounds of the formula I can be obtained by customary methods known to those skilled in the art, for example by combining a compound of the formula I I with an inorganic or organic acid or base in a solvent or dispersant, or from other salts by cation exchange or anion exchange. The present invention also includes all salts of the compounds of the formula I which, because of low physiologically tolerability, are not directly suitable for use in pharmaceuticals but are suitable, for example, as intermediates for carrying out further chemical modifications of the compounds of the formula I or as starting materials for the preparation of physiologically tolerable salts.

The present invention furthermore includes all solvates of compounds of the formula I for example hydrates or adducts with alcohols.

The invention also includes derivatives and modifications of the compounds of the formula I for example prodrugs, protected forms and other physiologically tolerable derivatives, as well as active metabolites of the compounds of the formula I. The invention relates in particular to prodrugs and protected forms of the compounds of the formula I, which can be converted into compounds of the formula I under physiological conditions. Suitable prodrugs for the compounds of the formula I, i. e. chemically modified derivatives of the compounds of the formula I having properties which are improved in a desired manner, for example with respect to solubility, bioavailability or duration of action, are known to those skilled in the art. More detailed information relating to prodrugs is found in standard literature like, for example, Design of Prodrugs, H. Bundgaard (ed.), Elsevier, 1985; Fleisher et al., Advanced Drug Delivery Reviews 19 (1996) 115-130; or H. Bundgaard, Drugs of the Future 16 (1991) 443 which are all incorporated herein by reference. Suitable prodrugs for the compounds of the formula I are especially acyl prodrugs and carbamate prodrugs of acylatable nitrogen-containing groups such as amino groups and the guanidino group and also ester prodrugs and amide prodrugs of carboxylic acid groups which may be present in compounds of the formula I. In the acyl prodrugs and carbamate prodrugs one or more, for example one or two, hydrogen atoms on nitrogen atoms in such groups are replaced with an acyl group or a carbamate, preferably a -($C_1$-$C_6$)-alkyloxycarbonyl group. Suitable acyl groups and carbamate groups for acyl prodrugs and carbamate prodrugs are, for example, the groups $R^{p1}$-CO- and $R^{p2}$O-CO-, in which $R^{p1}$ is hydrogen, ($C_1$-$C_{18}$)-alkyl, ($C_3$-$C_8$)-cycloalkyl, ($C_3$-$C_8$)-cycloalkyl-($C_1$-$C_4$)-alkyl-, ($C_6$-$C_{14}$)-aryl, Het-, ($C_6$-$C_{14}$)-aryl-($C_1$-$C_4$)-alkyl- or Het-($C_1$-$C_4$)-alkyl- and in which $R^{p2}$ has the meanings indicated for $R^{p1}$ with the exception of hydrogen.

Especially preferred compounds of the formula I are those wherein two or more residues are defined as indicated before for preferred compounds of the formula I, or residues can have one or some of the specific denotations of the residues given in their general definitions or in the definitions of preferred compounds before. All possible combinations of definitions given for preferred definitions and of specific denotations of residues explicitly are a subject of the present invention.

Also with respect to all preferred compounds of the formula I all their stereoisomeric forms and mixtures thereof in any ratio and their physiologically acceptable salts explicitly are a subject of the present invention, as well as are their prodrugs. Similarly, also in all preferred compounds of the formula I, all residues that are present more than one time in the molecule are independent of each other and can be identical or different.

The compounds of the formula I can be prepared by utilising procedures and techniques, which per se are well known and appreciated by one of ordinary skill in the art. Starting materials or building blocks for use in the general synthetic procedures that can be applied in the preparation of the compounds of formula I are readily available to one of ordinary skill in the art. In many cases they are commercially available or have been described in the literature. Otherwise they can be prepared from readily available precursor compounds analogously to procedures described in the literature, or by procedures or analogously to procedures described in this application.

In general, compounds of the formula I can be prepared, for example in the course of a convergent synthesis, by linking two or more fragments which can be derived retrosynthetically from the formula I. More specifically, suitably substituted starting β-aminoacid derivatives are employed as building blocks in the preparation of the compounds of formula I. If not commercially available, such β-aminoacid derivatives can be prepared according to the well-known standard procedures for the formation of the β-aminoacid. By choosing suitable precursor molecules, these β-aminoacid syntheses allow the introduction of a variety of substituents into the various positions of the β-aminoacid system, which can be chemically modified in order to finally arrive at the molecule of the formula I having the desired substituent pattern. As one of the comprehensive reviews in which numerous details and literature references on the chemistry of β-aminoacids and on synthetic procedures for their preparation can be found, Juaristi, E. (ed.), *Enantioselective Synthesis of β-Amino Acids.* 1st ed. Wiley-VCH: New York, 1997; Cole, D. C., *Recent Stereoselective Synthetic Approaches to β-Amino Acids. Tetrahedron* **1994,** *50,* 9517-9582; Abele, S. and Seebach, D., *Eur. J. Org. Chem.* **2000,** 1-15; Juaristi, E. and López-Ruiz, H., *Recent Advances in the Enantioselective Syntheses of β-Amino Acids. Curr. Med. Chem*. **1999,** *6*, 983-1004. If starting β-aminoacid derivatives are not commercially available and have to be synthesized this can be done, for example, according to the well-known β-aminoacid syntheses mentioned above. In the following procedures of particuluar interest for the embodiment of this invention are listed and referenced briefly, however, they are standard

procedures comprehensively discussed in the literature, and are well known to one skilled in the art. Although not always shown explicitly, in certain cases isomers will occur during the synthesis of the below mentioned reactions. Nevertheless such mixtures of isomers, can be separated by modern separation techniques like, for example, preparative HPLC.

1) Arndt-Eistert homologation of β-aminoacids (see for example Plucinska, K. and Liberek, B., *Tetrahedron* **1987**, *43*, 3509-3517; Cassal, J.-M., Fürst, A. and Meier, W., *Helv. Chim. Acta* **1976,** *59*, 1917-1924):

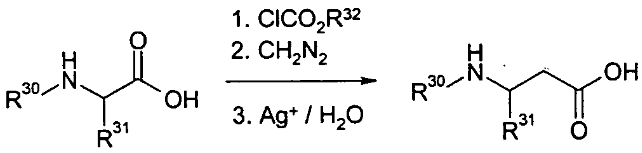


2) From aspartic acid, asparagines and derivatives, e.g. via transformation into homoserine derivatives and treatment whith a cuprate reagent "$R^{35}$Cu" (El Marino, A., Roumestant, M. L., Viallefont, P. Razafindramboa, D., Bonato, M. and Follet, M., *Synthesis* **1992,** 1104-1108)

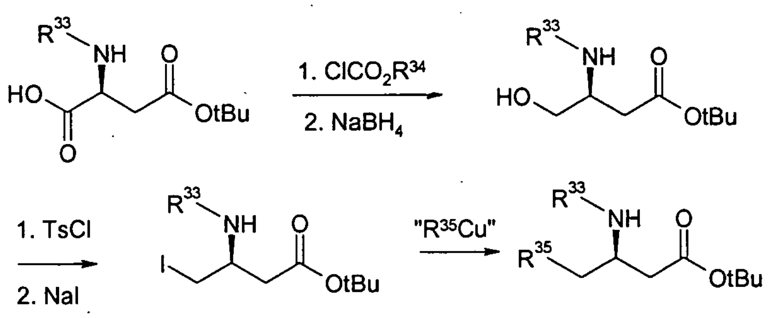


3) By Michael addition of amines to acrylates and their derivatives:
see for example Kwiatkowski, S., Jeganathan, A., Tobin, T. and Watt, D. S., *Synthesis* **1989,** 946-949 or Woster, P.M. & Murray, W J, *J.Med.Chem.*; 29; 5; 1986; 865-868 (cyclic acrylates):

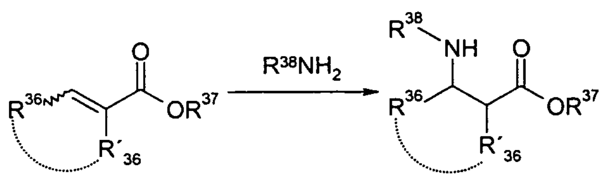


or, for example, by addition of lithium amide derivatives of chiral secondary amines to crotonate esters (Davies, S. G. and Ichihara, 0., *Tetrahedron: Asymmetry* **1991,** *2*, 183-186)

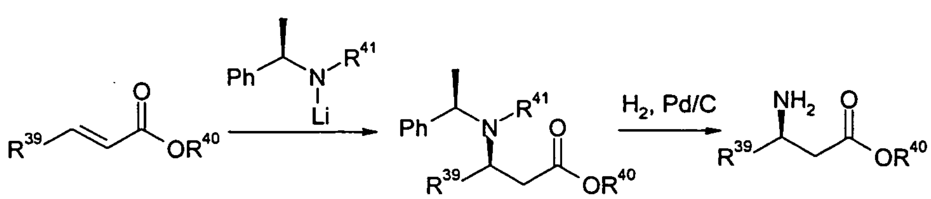


4) By hydrogenation of 3-amino acrylates (see for example Lubell, W. D., Kitamura, M. and Noyori, R., *Tetrahedron: Asymmetry* **1991,** *2*, 543-554; Achiwa, K. and Soga, T., *Tetradedron Lett.* **1978,**28,1119-1120)

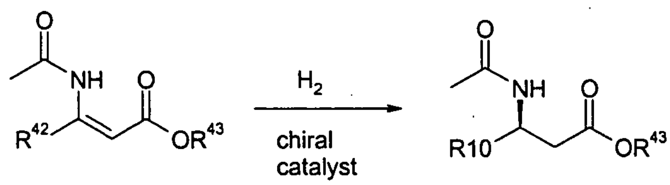


5) Nucleophilic addition to C-N double bond equivalents, e. g. addition of enolates to chiral sulfinimines (Tang, T. P. and Ellmann, J. A., *J. Org. Chem.* **2002,** *67,* 7819-7832, and references cited therein):

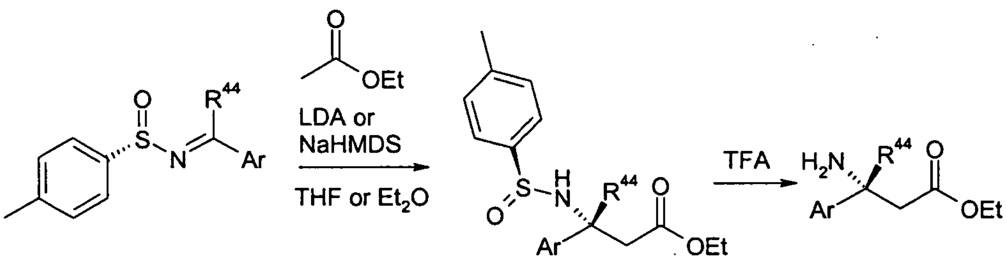


or

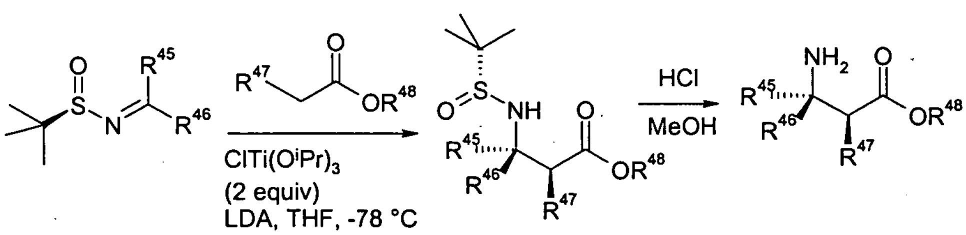


Further, in order to obtain the desired substituents at the β-aminoacid system in the formula I, the functional groups introduced into the ring system during the β-aminoacid synthesis can be chemically modified. Especially the groups present in the β-aminoacid system can be modified by a variety of reactions and thus the desired residues $R^{1a}$, $R^{1b}$ ,$R^{1c}$ ,$R^{1d}$ be obtained. Hydroxymethyl groups as well as formyl groups attached to the β-aminoacid system can be transformed to a variety of functional groups, for example, to the corresponding carboxylic acid or carboxylic ester by many oxidative reactions well known to those skilled in the art. Moreover a nitrile group attached to the β-aminoacid can, for example, easily be converted into the desired acid under acidic, basic or reductive conditions. In addition, carboxylic acid groups and acetic acid groups can be converted into their homologues by usual reactions for chain elongation of carboxylic acids. Halogen atoms can be introduced into aromatic side chains, for example according to procedures like the following described in the literature. For the fluorination N-fluoro-2,4,6-trimethylpyridinium triflate is the reagent of choice (T. Umemoto, S. Fukami, G. Tomizawa, K. Harasawa, K. Kawada, K. Tomita, J. Am. Chem. Soc. (1990) 112, 8563 see also K. Manko et al., J. Fluorine Chem. (1988) 39, 435; R. Storer et al. Nucleosides Nucleotides (1999) 18; 203) however, other suitable fluorinating reagents may also be employed where appropriate. The chlorination, bromination, or iodination of aromatic side chains can be accomplished by the reaction with elemental halogens or by the use of NCS, NBS or NIS and many other reagents well known to those skilled in the art. Depending on the reaction conditions, reagent, stochiometry and substitution pattern the halogen is introduced in the different positions of an aromatic side chain of the β-amino acid. By selective halogen/metal exchange or metalation by selective hydrogen/metal exchange and subsequent reaction with a wide range of electrophiles various substituents can be introduced at the aromatic nucleus. (M. R. Grimmett, Heterocycles (1994) 37, 2087; V. D. Gardner et al., J. Heterocycl. Chem. (1984) 21, 121; D. Butler et al., J. Org. Chem. (1971) 36, 2542). Halogens or hydroxy groups (via their triflates or nonaflates) - or primary amines (via their diazonium salts) present in the side chain of the β-amino acid - can be converted directly, or after interconversion to the corresponding stannane, or boronic acid, into a variety of other functional groups like for example -CN, $-CF_3$, $-C_2F_5$, ethers, acids, amides, amines, alkyl- or aryl- groups mediated by means of transition metals, namely palladium or nickel catalysts or copper salts and reagents for example referred to below (F. Diederich, P. Stang, Metal-catalyzed Cross-coupling Reactions, Wiley-VCH, 1998; or M. Beller, C. Bolm, Transition Metals for Organic Synthesis, Wiley-VCH, 1998; J. Tsuji, Palladium Reagents and Catalysts, Wiley, 1996;

J. Hartwig, Angew. Chem. (1998) 110, 2154; B. Yang, S. Buchwald, J. Organomet. Chem. (1999) 576, 125; T. Sakamoto, K. Ohsawa, J. Chem. Soc. Perkin Trans I (1999) 2323; D. Nichols, S. Frescas, D. Marona-Lewicka, X. Huang, B. Roth, G. Gudelsky, J. Nash, J. Med. Chem. (1994) 37, 4347; P. Lam, C. Clark, S. Saubern, J. Adams, M. Winters, D. Chan, A. Combs, Tetrahedron Lett. (1998) 39, 2941; D. Chan, K. Monaco, R. Wang, M. Winters, Tetrahedron Lett. (1998) 39, 2933; V. Farina, V. Krishnamurthy, W. Scott, The Stille Reaction, Wiley, 1994; F. Qing et al. J. Chem. Soc. Perkin Trans. I (1997) 3053; S. Buchwald et al. J. Am. Chem Soc. (2001) 123, 7727; S. Kang et al. Synlett (2002) 3, 427; S. Buchwald et al. Organic Lett. (2002) 4, 581; T. Fuchikami et al. Tetrahedron Lett. (1991) 32, 91; Q. Chen et al. Tetrahedron Lett. (1991) 32, 7689). For example, nitro groups can be reduced to amino groups by means of various reducing agents, such as sulfides, dithionites, complex hydrides or by catalytic hydrogenation. A reduction of a nitro group may also be carried out at a later stage of the synthesis of a compound of the formula I, and a reduction of a nitro group to an amino group may also occur simultaneously with a reaction performed on another functional group, for example when reacting a group like a cyano group with hydrogen sulfide or when hydrogenating a group. In order to introduce the residues $R^{1a}$, $R^{1b}$,$R^{1c}$ ,$R^{1d}$, amino groups can then be modified according to standard procedures for alkylation, for example by reaction with (substituted) alkyl halogenides or by reductive amination of carbonyl compounds, according to standard procedures for acylation, for example by reaction with activated carboxylic acid derivatives such as acid chlorides, anhydrides, activated esters or others or by reaction with carboxylic acids in the presence of an activating agent, or according to standard procedures for sulfonylation, for example by reaction with sulfonyl chlorides.

Ester groups present in the β-aminoacid can be hydrolyzed to the corresponding carboxylic acids, which after activation can then be reacted with amines or alcohols under standard conditions to give amides or alcohols, respectively. Ester groups present in the β-aminoacid can be converted to other esters by transesterification. Carboxylic acids attached to a suitable β-aminoacid can also be alkylated to give esters. Ether groups present at the β-aminoacid, for example benzyloxy groups or other easily cleavable ether groups, can be cleaved to give hydroxy groups which then can be reacted with a variety of agents, for example etherification agents or activating agents allowing replacement of the hydroxy group by other groups. Sulfur-containing groups can be reacted analogously.

During the course of the synthesis in order to modify the groups $R^{50}$ or $R^{8'}$ attached to the β-aminoacid system by application of parallel synthesis methodology, a variety of reactions can be extremely useful, including, for example, palladium, nickel or copper catalysis. Such reactions are described for example in F. Diederich, P. Stang, Metal-catalyzed Cross-coupling Reactions, Wiley-VCH (1998) ; or M. Beller, C. Bolm, Transition Metals for Organic Synthesis, Wiley-VCH (1998) ; J. Tsuji, Palladium Reagents and Catalysts, Wiley (1996) ; J. Hartwig, Angew. Chem. (1998) , 110, 2154; B. Yang, S. Buchwald, J. Organomet. Chem. (1999), 576, 125; P. Lam, C. Clark, S. Saubern, J. Adams, M. Winters, D. Chan, A. Combs, Tetrahedron Lett. (1998), 39, 2941; D. Chan, K. Monaco, R. Wang, M. Winters, Tetrahedron Lett. (1998), 39, 2933; J. Wolfe, H. Tomori, J. Sadight, J. Yin, S. Buchwald, J. Org. Chem. (2000), 65, 1158; V. Farina, V. Krishnamurthy, W. Scott, The Stille Reaction, Wiley, (1994) ; S. Buchwald et al., J. Am. Chem. Soc. (2001), 123, 7727; S. Kang et al., Synlett (2002), 3, 427; S. Buchwald et al., Org. Lett. (2002), 4, 581.

The previously-mentioned reactions for the conversion of functional groups are furthermore, in general, extensively described in textbooks of organic chemistry like M. Smith, J. March, March's Advanced Organic Chemistry, Wiley-VCH, 2001 and in treatises like Houben-Weyl, "Methoden der Organischen Chemie" (Methods of Organic Chemistry), Georg Thieme Verlag, Stuttgart, Germany, or "Organic Reactions", John Wiley & Sons, New York, or R. C. Larock, " Comprehensive Organic Transformations", Wiley-VCH, 2nd ed (1999), B. Trost, I. Fleming (eds.) Comprehensive Organic Synthesis, Pergamon, 1991; A. Katritzky, C. Rees, E. Scriven Comprehensive Heterocyclic Chemistry II, Elsevier Science, 1996) in which details on the reactions and primary source literature can be found. Due to the fact that in the present case the functional groups are attached to a β-aminoacid it may in certain cases become necessary to specifically adapt reaction conditions or to choose specific reagents from a variety of reagents that can in principle be employed in a conversion reaction, or otherwise to take specific measures for achieving a desired conversion, for example to use protection group techniques. However, finding out suitable reaction variants and reaction conditions in such cases does not cause any problems for one skilled in the art.

The structural elements present in the residues attached to the β-aminoacid in the compounds of the formula I and in the $COR^{8'}$ group present in the β-aminoacid can be introduced into the β-aminoacid derivative obtainable as outlined above by consecutive reaction steps using synthesis methodologies like those outlined below using procedures which per se are well known to one skilled in the art.

The residues $R^{8'}$ that can be introduced in formula 2, for example, by condensing a corresponding carboxylic acid of the formula 2 with a compound of the formula $HR^{8'}$, i. e. with an amine of the formula $HN(R^{1'})R^{2'}$-V-G-M to give a compound of the formula 3. The compound of the formula 3 thus obtained can already contain the desired final groups, i. e. the groups $R^{8'}$ and $R^{50}$ can be the groups $-N(R^1)-R^2$-V-G-M and $R^0$-Q- as defined in the formula I, or optionally in the compound of the formula 3 thus obtained subsequently the residue or the residues $R^{8'}$ and the residue $R^{50}$ are converted into the residues $-N(R^1)R^2$-V-G-M and $R^0$-Q-, respectively, to give the desired compound of the formula I.

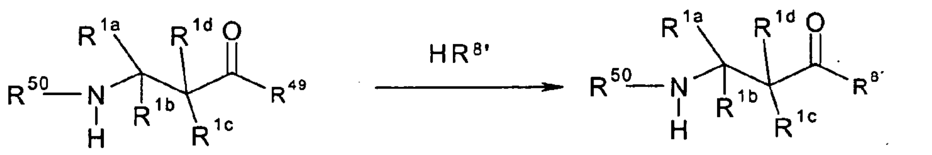


2 3

 formula I

Thus, the residues $R^{8'}$ and the residues $R^{1'}$ and $R^{2'}$-V-G-M contained therein can have the denotations of $R^1$ and $R^2$-V-G-M, respectively, given above or in addition in the residues $R^{1'}$ and $R^{2'}$-V-G-M functional groups can also be present in the form of groups that can subsequently be transformed into the final groups $R^1$ and $R^2$-V-G-M, i.e. functional groups can be present in the form of precursor groups or of derivatives, for example in protected form. In the course of the preparation of the compounds of the formula I, it can generally be advantageous or necessary to introduce functional groups which reduce or prevent undesired reactions or side reactions in the respective synthesis step, in the form of precursor groups which are later converted into the desired functional groups, or to temporarily block functional groups by a protective group strategy suited to the synthesis problem. Such strategies are well known to those skilled in the art (see, for example, Greene and Wuts, Protective Groups in Organic Synthesis, Wiley, 1991, or P. Kocienski, Protecting Groups, Thieme 1994). As examples of precursor groups cyano groups and nitro groups may be mentioned. The cyano group can in a later step be transformed into carboxylic acid derivatives or by reduction into aminomethyl groups, or the nitro groups may be transformed by reduction like catalytic hydrogenation into amino groups. Protective groups can also have the meaning of a solid phase, and cleavage from the solid phase stands for the removal of the protective group. The use of such techniques is known to those skilled in the art (Burgess K (Ed.) Solid Phase Organic Synthesis, New York, Wiley, 2000). For example, a phenolic hydroxy group can be attached to a trityl-polystyrene resin, which serves as a protecting group, and the molecule is cleaved from this resin by treatment with TFA at a later stage of the synthesis.

The residue $R^{50}$ in the compounds of the formulae 2 and 3 can denote the group -Q-$R^0$ as defined above which finally is to be present in the desired target molecule of the formula I, or it can denote a group which can subsequently be transformed into the group -Q-$R^0$, for example a precursor group or a derivative of the group -Q-$R^0$ in which functional groups are present in protected form, or $R^{50}$ can denote a hydrogen atom or a protective group for the nitrogen atom of the β-aminoacid. Similarly, the residues $R^{1a}$, $R^{1b}$ ,$R^{1c}$ ,$R^{1d}$ in the formulae 2 and 3 have the corresponding definitions of $R^3$; $R^4$; $R^5$, $R^6$ in formula I as defined above, however, for the synthesis of the compounds of the formula I these residues, too, can in principle be present at the stage of the condensation of a compound of the formula 2 with a compound of the formula $HR^{8'}$ giving a compound of the formula 3 in the form of precursor groups or in protected form.

The residues $R^{49}$ in the compounds of the formula 2 which can be identical or different, can be, for example, hydroxy or ($C_1$-$C_4$)-alkoxy, i. e., the groups $COR^{49}$ present in the compounds of the formula 2 can be, for example, the free carboxylic acids or esters thereof like alkyl esters as can be the groups $COR^{8'}$ in the compounds of the formula I. The groups $COR^{49}$ can also be any other activated derivative of a carboxylic acid which allows amide formation, ester formation or thioester formation with a compound of the formula $HR^{8'}$. The group $COR^{49}$ can be, for example, an acid chloride, an activated ester like a substituted phenyl ester or an N-hydroxysuccinimide or a hydroxybenzotriazole ester, an azolide like an imidazolide, an azide or a mixed anhydride, for example a mixed anhydride with a carbonic acid ester or with a sulfonic acid, which derivatives can all be prepared from the carboxylic acid by standard procedures and can be reacted with an amine, an alcohol or a mercaptan of the formula $HR^{8'}$ under standard conditions. A carboxylic acid group COOH representing $COR^{49}$ in a compound of the formula 2 can be obtained, for example, from an ester group of the β-aminoacid by standard hydrolysis procedures. It can also be obtained, for example, by hydrolysis of a nitrile group introduced into the β-aminoacid during a β-aminoacid synthesis.

Compounds of the formula I in which a group $COR^{8'}$ is an ester group can also be prepared from compounds of the formula 2 in which $COR^{49}$ is a carboxylic acid group by common esterification reactions like, for example, reacting the acid with an alcohol under acid catalysis, or alkylation of a salt of the carboxylic acid with an electrophile like an alkyl halogenide, or by transesterification from another ester. Compounds of the formula I in which a group $COR^{8'}$ is an amide group can be prepared from amines and compounds of the formula 2 in which $COR^{49}$ is a carboxylic acid group or an ester thereof by common amination reactions. Especially for the preparation of amides the compounds of the formula 2 in which $COR^{49}$ is a carboxylic acid group can be condensed under standard conditions with compounds of the formula $HR^{8'}$ which are amines by means of common coupling reagents used in peptide synthesis. Such coupling reagents are, for example, carbodiimides like dicyclohexylcarbodiimide (DCC) or diisopropylcarbodiimide, carbonyldiazoles like carbonyldiimidazole (CDI) and similar reagents, propylphosphonic anhydride, O-((cyano-(ethoxycarbonyl)-methylene)amino)-N,N,N',N'-tetramethyluronium tetrafluoroborate (TOTU), diethylphosphoryl cyanide (DEPC) or bis-(2-oxo-3-oxazolidinyl)-phosphoryl chloride (BOP-Cl) and many others.

If the residue $-Q-R^0$ present in an β-aminoacid of the formula I or the residue $R^{50}$ present in an β-aminoacid of the formula 2, or a residue in which functional groups within the residue $-Q-R^0$ or $R^{50}$ are present in protected form or in the form of a precursor group, have not already been introduced during a preceding step, for example during a synthesis of the β-aminoacid, these residues can, for example, be introduced into the β-aminoacid system by conventional literature procedures for N-alkylation, reductive amination, N-arylation, N-acylation or N-sulfonylation of ring nitrogen atoms of the β-aminoacid well known to one skilled in the art. N-Acylation of a nitrogen atom can, for example, be performed under standard conditions by means of common coupling reagents used in peptide synthesis. Such coupling reagents are, for example, carbodiimides like dicyclohexylcarbodiimide (DCC) or diisopropylcarbodiimide, carbonyldiazoles like carbonyldiimidazole (CDI) and similar reagents, propylphosphonic anhydride, O-((cyano-(ethoxycarbonyl)-methylene)amino)-N,N,N',N'-tetramethyluronium tetrafluoroborate (TOTU), diethylphosphoryl cyanide (DEPC) or bis-(2-oxo-3-oxazolidinyl)-phosphoryl chloride (BOP-Cl) and many others. N-Alkylation of a nitrogen atom can, for example, be performed under standard conditions, preferably in the presence of a base like $K_2CO_3$, $Cs_2CO_3$, NaH or $KO^tBu$, using an alkylating compound of the formula $LG-Q-R^0$ or of the formula $R^{50}-LG$, wherein the atom in the group Q or in the group $R^{50}$ bonded to the group LG in this case is an aliphatic carbon atom of an alkyl moiety and LG is a leaving group, for example halogen like chlorine, bromine or iodine, or a sulfonyloxy group like tosyloxy, mesyloxy or trifluormethylsulfonyloxy. The regioselectivity of the N-alkylation can be controlled by the choice of the base, solvent and reaction conditions. Nevertheless mixtures of positional isomers, can be separated by modern separation techniques like, for example, flash chromatography, crystallisation or preparative HPLC.

Preferred methods include, but are not limited to those described in the examples.

The compounds of the present invention are serine protease inhibitors, which inhibit the activity of the blood coagulation enzyme factors Xa and/or factor VIIa. In particular, they are highly active inhibitors of factor Xa. They are specific serine protease inhibitors inasmuch as they do not substantially inhibit the activity of other proteases whose inhibition is not desired. The activity of the compounds of the formula I can be determined, for example, in the assays described below or in other assays known to those skilled in the art. With respect to factor Xa inhibition, a preferred embodiment of the invention comprises compounds which have a $Ki < 1$ mM for factor Xa inhibition as determined in the assay described below, with or without concomitant factor VIIa inhibition, and which preferably do not substantially inhibit the activity of other proteases involved in coagulation and fibrinolysis whose inhibition is not desired (using the same concentration of the inhibitor). The compounds of the invention inhibit factor Xa catalytic activity either directly, within the prothrombinase complex or as a soluble subunit, or indirectly, by inhibiting the assembly of factor Xa into the prothrombinase complex.

As inhibitors of factor Xa and/or factor VIIa the compounds of the formula I and their physiologically tolerable salts and their prodrugs are generally suitable for the therapy and prophylaxis of conditions in which the activity of factor Xa and/or factor VIIa plays a role or has an undesired extent, or which can favorably be influenced by inhibiting factor Xa and/or factor VIIa or decreasing their activities, or for the prevention, alleviation or cure of which an inhibition of factor Xa and/or factor VIIa or a decrease in their activity is desired by the physician. As inhibition of factor Xa and/or factor VIIa influences blood coagulation and fibrinolysis, the compounds of the formula I and their physiologically tolerable salts and their prodrugs are generally suitable for reducing blood clotting, or for the therapy and prophylaxis of conditions in which the activity of the blood coagulation system plays a role or has an undesired extent, or which can favorably be influenced by reducing blood clotting, or for the prevention, alleviation or cure of which a decreased activity of the blood coagulation system is desired by the physician. A specific subject of the present invention thus are the reduction or inhibition of unwanted blood clotting, in particular in an individual, by administering an effective amount of a compound I or a physiologically tolerable salt or a prod rug thereof, as well as pharmaceutical preparations therefor.

The present invention also relates to the compounds of the formula I and/or their physiologically tolerable salts and/or their prodrugs for use as pharmaceuticals (or medicaments), to the use of the compounds of the formula I and/or their physiologically tolerable salts and/or their prodrugs for the production of pharmaceuticals for inhibition of factor Xa and/or factor VIIa or for influencing blood coagulation, inflammatory response or fibrinolysis or for the therapy or prophylaxis of the diseases mentioned above or below, for example for the production of pharmaceuticals for the therapy and prophylaxis of cardiovascular disorders, thromboembolic diseases or restenoses. The invention also relates to the use of the compounds of the formula I and/or their physiologically tolerable salts and/or their prodrugs for the inhibition of factor Xa and/or factor VIIa or for influencing blood coagulation or fibrinolysis or for the therapy or prophylaxis of the diseases mentioned above or below, for example for use in the therapy and prophylaxis of cardiovascular disorders, thromboembolic diseases or restenoses, and to methods of treatment aiming at such purposes including methods for said therapies and prophylaxis. The present invention also relates to pharmaceutical preparations (or pharmaceutical compositions) which contain an effective amount of at least one compound of the formula I and/or

its physiologically tolerable salts and/or its prodrugs in addition to a customary pharmaceutically acceptable carrier, i. e. one or more pharmaceutically acceptable carrier substances or excipients and/or auxiliary substances or additives.

The invention also relates to the treatment of disease states such as abnormal thrombus formation, acute myocardial infarction, unstable angina, thromboembolism, acute vessel closure associated with thrombolytic therapy or percutaneous transluminal coronary angioplasty (PTCA), transient ischemic attacks, stroke, intermittent claudication or bypass grafting of the coronary or peripheral arteries, vessel luminal narrowing, restenosis post coronary or venous angioplasty, maintenance of vascular access patency in long-term hemodialysis patients, pathologic thrombus formation occurring in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, certain viral infections or cancer.

The compounds of the present invention can also be used to reduce an inflammatory response. Examples of specific disorders for the treatment or prophylaxis of which the compounds of the formula I can be used are coronary heart disease, myocardial infarction, angina pectoris, vascular restenosis, for example restenosis following angioplasty like PTCA, adult respiratory distress syndrome, multi-organ failure and disseminated intravascular clotting disorder. Examples of related complications associated with surgery are thromboses like deep vein and proximal vein thrombosis, which can occur following surgery.

The compounds of the formula I and their physiologically tolerable salts and their prodrugs can be administered to animals, preferably to mammals, and in particular to humans as pharmaceuticals for therapy or prophylaxis. They can be administered on their own, or in mixtures with one another or in the form of pharmaceutical preparations, which permit enteral or parenteral administration.

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carriers being used in addition to the compound(s) of the formula I and/or its (their) physiologically tolerable salts and/or its (their) prodrugs. For the production of pills, tablets, coated tablets and hard gelatin capsules it is possible to use, for example, lactose, cornstarch or derivatives thereof, talc, stearic acid or its salts, etc. Carriers for soft gelatin capsules and suppositories are, for example, fats, waxes, semisolid and liquid polyols, natural or hardened oils, etc. Suitable carriers for the production of solutions, for example injection solutions, or of emulsions or syrups are, for example, water, saline, alcohols, glycerol, polyols, sucrose, invert sugar, glucose, vegetable oils, etc. Suitable carriers for microcapsules, implants or rods are, for example, copolymers of glycolic acid and lactic acid. The pharmaceutical preparations normally contain about 0.5 % to 90 % by weight of the compounds of the formula I and/or their physiologically tolerable salts and/or their prodrugs. The amount of the active ingredient of the formula I and/or its physiologically tolerable salts and/or its prodrugs in the pharmaceutical preparations normally is from about 0.5 mg to about 1000 mg, preferably from about 1 mg to about 500 mg.

In addition to the active ingredients of the formula I and/or their physiologically acceptable salts and/or prodrugs and to carrier substances, the pharmaceutical preparations can contain additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formula I, and/or their physiologically tolerable salts and/or their prodrugs. In case a pharmaceutical preparation contains two or more compounds of the formula I, the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the compounds of the formula I allows a great deal of control over the biological and physico-chemical properties of the compounds and thus allows the selection of such desired compounds. Furthermore, in addition to at least one compound of the formula I and/or a physiologically tolerable salt and/or its prodrug, the pharmaceutical preparations can also contain one or more other therapeutically or prophylactically active ingredients.

When using the compounds of the formula I the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily

dose for achieving the desired results in an adult weighing about 75 kg is from 0.01 mg/kg to 100 mg/kg, preferably from 0.1 mg/kg to 50 mg/kg, in particular from 0.1 mg/kg to 10 mg/kg, (in each case in mg per kg of body weight). The daily dose can be divided, in particular in the case of the administration of relatively large amounts, into several, for example 2, 3 or 4, part administrations. As usual, depending on individual behavior it may be necessary to deviate upwards or downwards from the daily dose indicated.

A compound of the formula I can also advantageously be used as an anticoagulant outside an individual. For example, an effective amount of a compound of the invention can be contacted with a freshly drawn blood sample to prevent coagulation of the blood sample. Further, a compound of the formula I or its salts can be used for diagnostic purposes, for example in in vitro diagnoses, and as an auxiliary in biochemical investigations. For example, a compound of the formula I can be used in an assay to identify the presence of factor Xa and/or factor VIIa or to isolate factor Xa and/or factor VIIa in a substantially purified form. A compound of the invention can be labeled with, for example, a radioisotope, and the labeled compound bound to factor Xa and/or factor VIIa is then detected using a routine method useful for detecting the particular label. Thus, a compound of the formula I or a salt thereof can be used as a probe to detect the location or amount of factor Xa and/or factor VIIa activity in vivo, in vitro or ex vivo.

Furthermore, the compounds of the formula I can be used as synthesis intermediates for the preparation of other compounds, in particular of other pharmaceutical active ingredients, which are obtainable from the compounds of the formula I, for example by introduction of substituents or modification of functional groups.

The general synthetic sequences for preparing the compounds useful in the present invention our outlined in the examples given below. Both an explanation of, and the actual procedure for, the various aspects of the present invention are described where appropriate. The following examples are intended to be merely illustrative of the present invention, and not limiting thereof in either scope or spirit. Those with skill in the art will readily understand that known variations of the conditions and processes described in the examples can be used to synthesize the compounds of the present invention.

It is understood that changes that do not substantially affect the activity of the various embodiments of this invention are included within the invention disclosed herein. Thus, the following examples are intended to illustrate but not limit the present invention.

Examples

When in the final step of the synthesis of a compound an acid such as trifluoroacetic acid or acetic acid was used, for example when trifluoroacetic acid was employed to remove a tBu group or when a compound was purified by chromatography using an eluent which contained such an acid, in some cases, depending on the work-up procedure, for example the details of a freeze-drying process, the compound was obtained partially or completely in the form of a salt of the acid used, for example in the form of the acetic acid salt or trifluoroacetic acid salt or hydrochloric acid salt.

Abbreviations used:

| | |
|---|---|
| tert-Butyl | tBu |
| 2,2'-bis(diphenylphoshino-1,1'-binaphthyl | Binap |
| Bis-(oxo-3-oxazolidinyl)-phosphoryl chloride | BOP-Cl |
| dibenzylidenacetone | dba |
| Dichloromethane | DCM |
| Dicyclohexyl-carbodiimide | DCC |
| Diethylphosphoryl cyanide | DEPC |
| Diisopropylethyl amine | DIPEA |
| 4-Dimethyaminopyridine | DMAP |
| N,N-Dimethylformamide | DMF |
| Dimethylsulfoxide | DMSO |
| 1,1'-Bis(diphenylphosphino)ferrocene | DPPF |
| O-(7-Azabenzotriazol-1-yl)-N,N,N',N'-tetramethyluronium-hexafluorophosphate | HATU |
| N-Bromosuccinimide | NBS |
| N-Chlorosuccinimide | NCS |
| N-Iodosuccinimide | NIS |
| N-Ethylmorpholine | NEM |
| Methanol | MeOH |

| | |
|---|---|
| Room temperature 20 °C to 25 °C | RT |
| Saturated | sat. |
| Tetrahydrofuran | THF |
| Trifluoroacetic acid | TFA |
| O-((Ethoxycarbonyl)cyanomethyleneamino)-N,N,N',N'-tetramethyluronium tetrafluoroborate | TOTU |

Example 1: 5-Chloro-thiophene-2-carboxylic acid {2-[4-(3-oxo-morpholin-4-yl)-phenylcarbamoyl]-ethyl}- amide

(i) 3-[(5-Chloro-thiophene-2-carbonyl)-amino]-propionic acid tert-butyl ester

A solution of 340 mg 5-Chloro-thiophene-2-carboxylic acid; 362 mg 3-Amino-propionic acid tert-butyl ester; 785 mg TOTU and 0.66 mL triethylamine in 10 mL DMF was stirred at room temperature for 12 h. 20 mL water were added and the organic phase was extracted twice with ethylacetate. The solvent was dried over $MgSO_4$ and the solvent was removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid.
Yield: 484 mg MS (ES+): m/e= 290, chloro pattern

(ii) 3-[(5-Chloro-thiophene-2-carbonyl)-amino]-propionic acid

A solution of 484 mg 3-[(5-Chloro-thiophene-2-carbonyl)-amino]-propionic acid tert-butyl ester in 50 ml $CH_2Cl_2$ and 15 mL trifluoroacetic acid was stirred for 12 h. The solvent was removed under reduced pressure. The product was recrystallized from diisopropylether.
Yield: 190 mg MS (ES+): m/e= 234, chloro pattern

(iii) 4-(4-Nitro-phenyl)-morpholine

A mixture of 24.5 g morpholine and 13.3 g 1-Fluoro-4-nitro-benzene in 30 ml DMSO was heated to 100°C for 4 h. This solution was poured on to 300 ml of water and the resulting precipitate was collected by filtration to yield a bright yellow crystalline product, which was dried in vacuo.
Yield: 19.7g.

(iv) 4-(4-Nitro-phenyl)-morpholin-3-one

To a solution of 10 g 4-(4-Nitro-phenyl)-morpholine in 200 ml DCM, 32 g Benzyl-triethylammonium chloride and 22.7 g potassium permanganate (325 mesh) were cautiously added at RT. After stirring for 1 h at RT the reaction mixture was heated to reflux for 10 h. Then a solution of 95 g $Na_2SO_3$ in 450 ml water were added under ice cooling and vigourous stirring. The mixture was filtered trough a pad of celite and the filtrate was concentrated under reduced pressure. The yellow solid was stirred with 250 ml water and the precipitated product was collected by filtration. This crude product was purified by chromatography on silica gel eluting with a gradient of DCM/MeOH 100%->50%. The fractions containing the product were combined and
the solvent evaporated under reduced pressure. Yield: 2.6 g.

(v) 4-(4-Amino-phenyl)-morpholin-3-one

To a solution of 2.6 g 4-(4-Nitro-phenyl)-morpholin-3-one in 350 ml ethyl acetate and 17 ml ethanol, 13.2 g $SnCl_2$ dihydrate were added and the reaction mixture was heated to reflux for 2 h. Then, after cooling to RT the mixture was stirred for 16 h. The precipitated product was collected by filtration and was pure enough for the next reaction step. Yield: 2.07 g.

(vi) 5-Chloro-thiophene-2-carboxylic acid {2-[4-(3-oxo-morpholin-4-yl)-phenylcarbamoyl]-ethyl}-amide

A solution of 185 mg 3-[(5-Chloro-thiophene-2-carbonyl)-amino]-propionic acid; 160 mg 4-(4-Amino-phenyl)-morpholin-3-one, 312 mg TOTU and 0.26 mL triethylamine in 10 mL DMF was stirred for 4h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 202 mg MS (ES+): m/e= 408, chloro pattern

Example 2: 5-Chloro-thiophene-2-carboxylic acid {2-[4-(2-oxo-2H-pyrazin-1-yl)-phenylcarba moyl]- ethyl}-amide

(i) 1-(4-Nitro-phenyl)-1H-pyrazin-2-one

A mixture of 720 mg 1-Fluoro-4-nitro-benzene, 632 mg sodium salt of 1H-Pyrazin-2-one and 3.3 g $Cs_2CO_3$ in 13 mL DMF was stirred at 35 °C for 6 h. Water was added and the mixture was stirred for 1 h. The precipitate was collected by filtration. The product was pure enough to use without further purification. Yield: 545 mg MS (ES+): m/e= 218.

(ii) 1-(4-Amino-phenyl)-1H-pyrazin-2-one

To a solution of 520 mg 1-(4-Nitro-phenyl)-1H-pyrazin-2-one in 26 mL ethylacetate and 13 mL ethanol was added 2.7 g $SnCl_2 \cdot (H_2O)_2$. The mixture was refluxed for 6 h. The precipitate was collected by filtration and the product was pure enough to use without further purification. Yield: 450 mg MS (ES+): m/e= 188.

(iii) 5-Chloro-thiophene-2-carboxylic acid {2-[4-(2-oxo-2H-pyrazin-1-yl)-phenylcarbamoyl]- ethyl}-amide

A solution of 218 mg 3-[(5-Chloro-thiophene-2-carbonyl)-amino]-propionic acid; 262 mg 1-(4-Amino-phenyl)-1H-pyrazin-2-one, 367 mg TOTU and 0.31 mL triethylamine in 10 mL DMF was stirred for 6h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 212 mg MS (ES+): m/e= 403, chloro pattern.

Example 3: 4-Chloro-N-{2-[4-(3-oxo-morpholin-4-yl)-phenylcarbamoyl]-ethyl}-benzamide

(i) 3-(4-Chloro-benzoylamino)-propionic acid tert-butyl ester

A solution of 3-amino-propionic acid tert-butyl ester in 5 mL $CH_2Cl_2$ was slowly added during 5 minutes to an ice-cold solution of 384 mg 4-Chloro-benzoyl chloride and 0.6 mL triethylamine in 25 mL $CH_2Cl_2$. After 1.h hour the reaction solution was washed 2x with water and the organic solvent was dried over $MgSO_4$. After removal of the solvent under reduced pressure the residue was purified by preparative HPLC (C18 reverse phase column, elution with a $H_2O$/MeCN gradient with 0.1% TFA). The fractions containing the product were evaporated and lyophilized to yield a colourless oil. Yield : 451 mg MS (ES+) : m/e= 284, chloro pattern.

(ii) 3-(4-Chloro-benzoylamino)-propionic acid

450 mg 3-(4-Chloro-benzoylamino)-propionic acid tert-butyl ester was dissolved in a mixture of 70 mL $CH_2Cl_2$ and 30 mL trifluoroacid acid. After 12 h the solvent was removed under reduced pressure and the residue was purified by preparative HPLC (C18 reverse phase column, elution with a $H_2O$/MeCN gradient with 0.1% TFA). The fractions containing the product were evaporated and lyophilized to yield a white solid. Yield: 360 mg MS (ES+): m/e= 228, chloro pattern

(iii) 4-Chloro-N-{2-[4-(3-oxo-morpholin-4-yl)-phenylcarbamoyl]-ethyl}-benzamide

A solution of 180 mg 3-(4-Chloro-benzoylamino)-propionic acid, 311 mg TOTU, 160 mg 4-(4-Amino-phenyl)-morpholin-3-one and 0,26 mL triethylamine in 10 mL DMF was stirred for 3h at room temperature. 5 mL water were added and the precipitate was collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 302 mg MS (ES+): m/e= 402, chloro pattern.

Example 4: 5-Chloro-thiophene-2-carboxylic acid {3-oxo-3-[5-(2-oxo-2H-pyrazin-1-yl)-2,3-dihydro-indol-1-yl]-propyl}-amide

(i) 5-Bromo-2,3-dihydro-indole-1-carboxylic acid tert-butyl ester

To a solution of 600 mg 5-Bromo-2,3-dihydro-1H-indole and 37 mg 4-dimethylaminopyridine in 30 ml acetonitrile was slowly added 992 mg di-tert-butyldicarbonate. After 2 h at 50 °C the solvent was removed under reduced pressure. The residue was dissolved in 50 mL ethylacetate and washed with water. The organic solvent was dried over

$MgSO_4$ and the solvent was removed under reduced pressure. The product was used without further purification.
Yield: 712 mg MS (ES+): m/e= 299.

(ii) 5-(2-Oxo-2H-pyrazin-1-yl)-2,3-dihydro-indole-1-carboxylic acid tert-butyl ester

A mixture of 712 mg 5-Bromo-2,3-dihydro-indole-1-carboxylic acid tert-butyl ester, 338 mg sodium salt of 1H-Pyrazin-2-one, 363 mg potassium carbonate, 173 mg 8-hydroxyquinoline and 227 mg copper(I) iodide in 10 ml DMF was carefully degassed under an atmosphere of argon. The reaction mixture was heated to 120 °C for 6h. 4 mL of 14% $NH_3$-solution was added and the mixture was stirred for 1 h. 20 mL water were added and the mixture was extracted with ethylacetate. The organic phase was dried over $MgSO_4$ and the solvent was removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid.
Yield: 237 mg MS (ES+): m/e= 314.

(iii) 1-(2,3-Dihydro-1H-indol-5-yl)-1H-pyrazin-2-one

237 mg 5-(2-Oxo-2H-pyrazin-1-yl)-2,3-dihydro-indole-1-carboxylic acid tert-butyl ester was dissolved in a mixture of 60 mL $CH_2Cl_2$ and 40 mL trifluoroacid acid. After 12 h the solvent was removed under reduced pressure and the residue was purified by preparative HPLC (C18 reverse phase column, elution with a $H_2O$/MeCN gradient with 0.1% TFA). The fractions containing the product were evaporated and lyophilized to yield a white solid.
Yield: 150 mg MS (ES+): m/e= 214.

(iv) 5-Chloro-thiophene-2-carboxylic acid {3-oxo-3-[5-(2-oxo-2H-pyrazin-1-yl)-2,3-dihydro- indol-1-yl]-propyl}-amide

A solution of 150 mg 3-[(5-Chloro-thiophene-2-carbonyl)-amino]-propionic acid; 270 mg 1-(2,3-Dihydro-1H-indol-5-yl)-1H-pyrazin-2-one, 253 mgTOTU and 0.32 mL triethylamine in 3 mL DMF was stirred for 5h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 100 mg MS (ES+): m/e= 429, chloro pattern.

Example 5: 5-Chloro-thiophene-2-carboxylic acid {3-oxo-3-[5-(2-oxo-2H-pyrazin-1-yl)-indol-1-yl]-propyl}-amide

(i) 1-(1H-Indol-5-yl)-1H-pyrazin-2-one

A mixture of 700 mg 5-Bromo-1H-indole, 506 mg sodium salt of 1H-Pyrazin-2-one, 543 mg potassium carbonate, 259 mg 8-hydroxyquinoline and 340 mg copper(I) iodide in 9 ml DMF was carefully degassed under an atmosphere of argon. The reaction mixture was heated to 120 °C for 6h. 4 mL of 14% $NH_3$-solution was added and the mixture was stirred for 1 h. 20 mL water were added and the mixture was extracted with ethylacetate. The organic phase was dried over MgS04 and the solvent was removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid.
Yield: 259 mg MS (ES+): m/e= 212.

(ii) 5-Chloro-thiophene-2-carboxylic acid {3-oxo-3-[5-(2-oxo-2H-pyrazin-1-yl)-indol-1-yl]-propyl}-amide

A solution of 60 mg 3-[(5-Chloro-thiophene-2-carbonyl)-amino]-propionic acid; 84 mg 1-(1H-Indol-5-yl)-1H-pyrazin-2-one, 101 mg TOTU, 35 mg hydroxybenzotriazole and 0.13 mL triethylamine in 3 mL DMF was stirred for 5h. Water was added and the water phase was extracted with ethylacetate. The organic phase was dried over $MgSO_4$ and the solvent removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid.
Yield: 10 mg MS (ES+): m/e= 427, chloro pattern.

Example 6: 5-Chloro-thiophene-2-carboxylic acid {2-[2-fluoro-4-(2-oxo-2H-pyrazin-1-yl)-phenylcarbamoyl]-ethyl}-amide

(i) 1-(4-Amino-3-fluoro-phenyl)-1H-pyrazin-2-one

A mixture of 2 g 2-Fluoro-4-iodo-phenylamine, 1 g sodium salt of 1 H-Pyrazin-2-one, 1,3 g potassium carbonate, 612 mg 8-hydroxyquinoline and 804 mg copper(I) iodide in 20 ml DMF was carefully degassed under an atmosphere of argon. The reaction mixture was heated to 130 °C in a microwave reactor for 120 min. 10 mL of 14% $NH_3$-solution were added and the mixture was stirred for 1 h. 20 mL water were added and the mixture was extracted with ethylacetate. The organic phase was dried over $MgSO_4$ and the solvent was removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid. Yield: 180 mg MS (ES+): m/e= 206.

(ii) 5-Chloro-thiophene-2-carboxylic acid {2-[2-fluoro-4-(2-oxo-2H-pyrazin-1-yl)-phenylcarbamoyl]-ethyl}-amide

A solution of 120 mg 3-[(5-Chloro-thiophene-2-carbonyl)-amino]-propionic acid; 164 mg 1-(4-Amino-3-fluoro-phenyl)-1H-pyrazin-2-one, 202 mg TOTU, and 0.26 mL triethylamine in 3 mL DMF was stirred for 12h. Water was added and the water phase was extracted with ethylacetate. The organic phase was dried over $MgSO_4$ and the solvent removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid. Yield: 9 mg MS (ES+): m/e= 421, chloro pattern.

Example 7: 5-Chloro-thiophene-2-carboxylic acid {2-[4-(2-dimethylaminomethyl-imidazol-1-yl)-2-fluoro-phenylcarbamoyl]-ethyl}-amide

(i) (1H-imidazol-2-ylmethyl)-dimethyl-amine

A solution of 710 mg dimethylamine hydrochloride and 1 g 1 H-Imidazole-2-carbaldehyde in 30 mL methanol was stirred for 5 min at room temperature. 657 mg sodium cyanoborhydride were added and the mixture was heated under reflux. The solvent was removed under reduced pressure, 20 ml of sat. $NaHCO_3$-solution were added and the mixture was extracted with ethylacetate. The organic phase was dried over $MgSO_4$ and the solvent was removed under reduced pressure. The product was used without further purification.
Yield: 911 mg MS (ES+): m/e= 126.

(ii) 4-(2-Dimethylaminomethyl-imidazol-1-yl)-2-fluoro-phenylamine

A mixture of 1,22 g 2-Fluoro-4-iodo-phenylamine, 773 mg (1H-Imidazol-2-ylmethyl)-dimethylamine, 783 mg potassium carbonate, 112 mg 8-hydroxyquinoline and 147 mg copper(I) iodide in 25 ml DMSO was carefully degassed under an atmosphere of argon. The reaction mixture was heated to 120 °C for 6h. 6 mL of 14% $NH_3$-solution were added and the mixture was stirred for 1 h. 20 mL water were added and the mixture was extracted with ethylacetate. The organic phase was dried over MgS04 and the solvent was removed under reduced pressure. The crude product was purified by preparative HPLC (C18 reverse phase column, elution with a $H_2O$/MeCN gradient with 0.1% TFA). The fractions containing the product were evaporated and lyophilized to yield a white solid. Yield: 713 mg MS (ES+): m/e= 235.

(iii) 5-Chloro-thiophene-2-carboxylic acid {2-[4-(2-dimethylaminomethyl-imidazol-1-yl)-2-fluoro-phenylcarbamoyl]-ethyl}-amide

A solution of 150 mg 3-[(5-Chloro-thiophene-2-carbonyl)-amino]-propionic acid; 235 mg 4-(2-Dimethylaminomethyl-imidazol-1-yl)-2-fluoro-phenylamine, 253 mg TOTU and 0.32 mL triethylamine in 3 mL DMF was stirred for 5h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid.
Yield: 110 mg MS (ES+): m/e= 450, chloro pattern.

Example 8: 3-(5-Chloro-thiophene-2-sulfonylamino)-N-[4-(3-oxo-morpholin-4-yl)-phenyl]-propionamide

(i) 3-(5-Chloro-thiophene-2-sulfonylamino)-propionic acid tert-butyl ester

To a solution of 1,33 g 3-Amino-propionic acid tert-butyl ester and 2,8 g diisopropylethylamine in 33 mL $CH_2Cl_2$ was slowly added at 0 °C the solution of 1,6 g 5-Chloro-thiophene-2-sulfonyl chloride in 5 mL $CH_2Cl_2$. The reaction mixture was allowed to reach room temperature and was stirred for 3h. The organic phase was washed with water, dried over $MgSO_4$ and the solvent was evaporated under reduced pressure. The crude product was purified by column chromatography (silica gel elution with a ethylacetate/heptane gradient). The fractions containing the product were evaporated to yield a colourless oil. Yield: 2,37 g MS (ES+): m/e= 326, chloro pattern.

(ii) 3-(5-Chloro-thiophene-2-sulfonylamino)-propionic acid

2,37 g 3-(5-Chloro-thiophene-2-sulfonylamino)-propionic acid butyl ester was dissolved in a mixture of 120 mL $CH_2Cl_2$ and 40 mL trifluoroacid acid. After 12 h the solvent was removed under reduced pressure and the residue was purified by preparative HPLC (C18 reverse phase column, elution with a $H_2O$/MeCN gradient with 0.1% TFA). The fractions containing the product were evaporated and lyophilized to yield a white solid.
Yield: 1,96 g MS (ES+): m/e= 270, chloro pattern.

(iii) 3-(5-Chloro-thiophene-2-sulfonylamino)-N-[4-(3-oxo-morpholin-4-yl)-phenyl]- propionamide

A solution of 160 mg 3-(5-Chloro-thiophene-2-sulfonylamino)-propionic acid; 120 mg 4-(4-Aminophenyl)-morpholin-3-one, 234 mg TOTU and 0.20 mL triethylamine in 3 mL DMF was stirred for 12h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 60 mg MS (ES+): m/e= 444, chloro pattern.

Example 9: 5-Chloro-thiophene-2-carboxylic acid {2-[4-(3,3-dioxo-3$16-[1,3,4]oxathiazinan-4-yl)-phenylcarbamoyl]-ethyl}-amide

(i) N-Benzyl-C-chloro-N-(2-hydroxy-ethyl)-methanesulfonamide

A solution of 4,9 g Chloro-methanesulfonyl chloride in 50 mL THF was slowly added to an ice cold solution of 5 g 2-Benzylamino-ethanol and 11.2 mL diisopropopylethylamine in 180 mL THF. The reaction mixture was allowed to slowly warm to room temperature. The solvent was removed under reduced pressure. The residue was dissolved in 100 mL ethylacetate and washed with water. The organic phase was dried over $MgSO_4$ and the solvent removed under reduced pressure. The crude product was pure enough to use without further purification.
Yield: 8.7 g MS (ES+): m/e= 264

(ii) 4-Benzyl-[1,3,4]oxathiazinane 3,3-dioxide

A mixture of 8.7 g N-Benzyl-C-chloro-N-(2-hydroxy-ethyl)-methanesulfonamide and 21.5 g $Cs_2CO_3$ in 100 mL DMF was heated to 80 °C for 15 h. Water was added and the solution was extracted with ethylacetate. The organic phase was dried over $MgSO_4$ and the solvent was removed under reduced pressure. The product was purified by column chromatography (silica gel elution with a ethylacetate/heptane gradient). The fractions containing the product were evaporated to yield a colourless oil. Yield: 7.5 g MS (ES+): m/e= 228

(iii) [1,3,4]Oxathiazinane 3,3-dioxide

A mixture of 6,3 g 4-Benzyl-[1,3,4]oxathiazinane 3,3-dioxide and 0,6 g Pd/C in 150 mL THF and 1,5 mL acetic acid was stirred under an atmosphere of hydrogen (6 bar/ 45 °C). After 6 h the reaction vessel was carefully purged with nitrogen, the catalyst was removed by filtration and the solvent was removed under reduced pressure. The product was pure enough to use without further purification. Yield: 3.8 g MS (ES+): m/e= 138

(iv) 4-(4-Nitro-phenyl)-[1,3,4]oxathiazinane 3,3-dioxide

A mixture of 2,2 g 1-Fluoro-4-nitro-benzene, 2.1 g [1,3,4]Oxathiazinane 3,3-dioxide and 10,0 g $Cs_2CO_3$ in 15 mL DMF was heated to 50 °C for 7 h. Water was added and the precipitate was collected by filtration. The crude product

was purified by column chromatography (silica gel elution with a ethylacetate/heptane gradient). The fractions containing the product were evaporated to yield a slightly yellow oil. Yield: 3,0 g MS (ES+): m/e= 259

(v) 4-(3,3-Dioxo-3$I6-[1,3,4]oxathiazinan-4-yl)-phenylamine

A mixture of 600 mg 4-(4-Nitro-phenyl)-[1,3,4]oxathiazinane 3,3-dioxide and 8 g Raney-Ni in 100 mL $NH_3$/methanol was stirred under an atmosphere of hydrogen (1 bar/ RT). After 4 h the reaction vessel was carefully purged with nitrogen, the catalyst was removed by filtration and the solvent was removed under reduced pressure. The crude product was purified by column chromatography (silica gel elution with a ethylacetate/heptane gradient). The fractions containing the product were evaporated to yield a slightly yellow oil.
Yield: 404 mg MS (ES+): m/e= 229

(vi) 5-Chloro-thiophene-2-carboxylic acid {2-[4-(3,3-dioxo-3$I6-[1,3,4]oxathiazinan-4-yl)-phenylcarbamoyl]-ethyl}-amide

A solution of 200 mg 3-[(5-Chloro-thiophene-2-carbonyl)-amino]-propionic acid; 195 mg 4-(3,3-Dioxo-3$16-[1,3,4]oxathiazinan-4-yl)-phenylamine, 337 mg TOTU and 0.29 mL triethylamine in 3 mL DMF was stirred for 4h. Water was added and the precipitate collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 318 mg MS (ES+): m/e= 444, chloro pattern.

Example 10: 5-Chloro-thiophene-2-carboxylic acid {2,2-difluoro-2-[4-(2-oxo-2H-pyrazin-1-yl)-phenykarbamoyl]-ethyl}-amide

(i) 3-[(5-Chloro-thiophene-2-carbonyl)-amino]-2,2-difluoro-propionic acid ethyl ester

To a solution of 650 mg 3-Amino-2,2-difluoro-propionic acid ethyl ester (prepared according to Cheguillaume et al, Tetrahedron Letters 44 (2003) 2375-2377), 415 mg 5-Chloro-thiophene-2-carboxylic acid, 958 mg TOTU and 1.2 mL triethylamine in 10 mL DMF was stirred for 6 h at room temperature. 100 mL ethylacetate and 50 ml water were added, the phases were separated and the organic phase was dried over $MgSO_4$. The crude product was purified by preparative HPLC (C18 reverse phase column, elution with a $H_2O$/MeCN gradient with 0.1% TFA). The fractions containing the product were evaporated and lyophilized to yield a white solid.
Yield: 445 mg MS (ES+): m/e= 298, chloro pattern.

(ii) 3-[(5-Chloro-thiophene-2-carbonyl)-amino]-2,2-difluoro-propionic acid

A solution of 445 mg 3-[(5-Chloro-thiophene-2-carbonyl)-amino]-2,2-difluoro-propionic acid ethyl ester and 71 mg LiOH in 7.5 mL THF and 7.5 mL water was stirred for 4 h. The organic phase was removed under reduced pressure and the 1 N hydrochloric acid was added. The water phase was extracted twice with ethylacetate and the organic phase was dried over $MgSO_4$. The solvent was removed under reduced pressure. The product was pure enough to use without further purification. Yield: 330 mg MS (ES+): m/e= 270, chloro pattern.

(iii) 5-Chloro-thiophene-2-carboxylic acid {2,2-difluoro-2-[4-(2-oxo-2H-pyrazin-1-yl)-phenylcarbamoyl]-ethyl}-amide

A solution of 160 mg 3-[(5-Chloro-thiophene-2-carbonyl)-amino]-2,2-difluoro-propionic acid, 166 mg 1-(4-Amino-phenyl)-1H-pyrazin-2-one, 233 mg TOTU and 0.20 mL triethylamine in 5 mL DMF was stirred for 3 h. Water was added and the precipitate was collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 210 mg MS (ES+): m/e= 439, chloro pattern.

Example 11: 5-Chloro-thiophene-2-carboxylic acid {2,2-difluoro-2-[4-(3-oxo-morpholin-4-yl)-phenylcarbamoyl]-ethyl}-amide

(i) A solution of 160 mg 3-[(5-Chloro-thiophene-2-carbonyl)-amino]-2,2-difluoro-propionic acid, 137 mg 4-(4-amino-phenyl)-morpholin-3-one, 234 mg TOTU and 0,20 mL triethylamine in 5 mL DMF was stirred for 3h. Water was added and the precipitate was collected by filtration. The crude product was purified by preparative HPLC (C18 reverse phase column, elution with a $H_2O$/MeCN gradient with 0.1% TFA). The fractions containing the product were evaporated and lyophilized to yield a white solid. Yield: 239 mg MS (ES+): m/e= 444, chloro pattern.

Pharmacological testing

The ability of the compounds of the formula I to inhibit factor Xa or factor VIIa or other enzymes like thrombin, plasmin, or trypsin can be assessed by determining the concentration of the compound of the formula I that inhibits enzyme activity by 50 %, i. e. the IC50 value, which was related to the inhibition constant Ki. Purified enzymes were used in chromogenic assays. The concentration of inhibitor that causes a 50 % decrease in the rate of substrate hydrolysis was determined by linear regression after plotting the relative rates of hydrolysis (compared to the uninhibited control) versus the log of the concentration of the compound of formula I. For calculating the inhibition constant Ki, the IC50 value was corrected for competition with substrate using the formula

$$Ki = IC50 / \{1 + (\text{substrate concentration} / Km)\}$$

wherein Km is the Michaelis-Menten constant (Chen and Prusoff, Biochem. Pharmacol. 22 (1973) 3099-3108; I. H. Segal, Enzyme Kinetics, 1975, John Wiley & Sons, New York, 100-125; which were incorporated herein by reference).

a) Factor Xa Assay

In the assay for determining the inhibition of factor Xa activity TBS-PEG buffer (50 mM Tris-HCl, pH 7.8, 200 mM NaCl, 0.05 % (w/v) PEG-8000, 0.02 % (w/v) NaN3) was used. The IC50 was determined by combining in appropriate wells of a Costar half-area microtiter plate 25 µl human factor Xa (Enzyme Research Laboratories, Inc.; South Bend, Indiana) in TBS-PEG; 40 µl 10 % (v/v) DMSO in TBS-PEG (uninhibited control) or various concentrations of the compound to be tested diluted in 10 % (v/v) DMSO in TBS-PEG; and substrate S-2765 (N(α)-benzyloxycarbonyl-D-Arg-Gly-L-Arg-p-nitroanilide; Kabi Pharmacia, Inc.; Franklin, Ohio) in TBS-PEG. The assay was performed by pre-incubating the compound of formula I plus enzyme for 10 min. Then the assay was initiated by adding substrate to obtain a final volume of 100 µl. The initial velocity of chromogenic substrate hydrolysis was measured by the change in absorbance at 405 nm using a Bio-tek Instruments kinetic plate reader (Ceres UV900HDi) at 25 °C during the linear portion of the time course (usually 1.5 min after addition of substrate). The enzyme concentration was 0.5 nM and substrate concentration was 140 µM.

b) Factor VIIa Assay

The inhibitory activity towards factor VIIa/tissue factor activity was determined using a chromogenic assay essentially as described previously (J. A. Ostrem et al., Biochemistry 37 (1998) 1053-1059 which was incorporated herein by reference). Kinetic assays were conducted at 25 °C in half-area microtiter plates (Costar Corp., Cambridge, Massachusetts) using a kinetic plate reader (Molecular Devices Spectramax 250). A typical assay consisted of 25 µl human factor VIIa and TF (5 nM and 10 nM, respective final concentration) combined with 40 µl of inhibitor dilutions in 10% DMSO/TBS-PEG buffer (50 mM Tris, 15 mM NaCl, 5 mM $CaCl_2$, 0.05 % PEG 8000, pH 8.15). Following a 15 minutes preincubation period, the assay was initiated by the addition of 35 µl of the chromogenic substrate S-2288 (D-Ile-Pro-Arg-p-nitroanilide, Pharmacia Hepar Inc., 500 µM final concentration). The results (inhibition constants Ki (FXa) for inhibition of factor Xa) are shown in Table 1.

Table1:

| Example | Ki(FXa) [nM] | Example | Ki(FXa) [nM] |
|---|---|---|---|
| 1 | 30 | 7 | 32 |
| 2 | 7 | 8 | 375 |
| 3 | 1395 | 9 | 30 |
| 4 | 40 | 10 | 8 |
| 5 | 8 | 11 | 18 |
| 6 | 4 | | |

## Claims

**1.** A compound of the formula I,

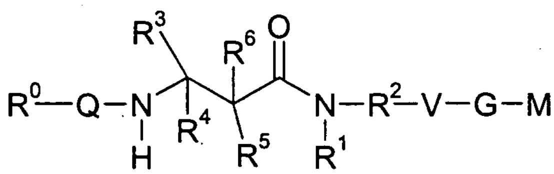

(I)

wherein

$R^0$ is

1) a monocyclic or bicyclic 4- to 15-membered heterocyclyl, selected out of the group benzimidazolyl, 1,3-benzodioxolyl, benzofuranyl, benzoxazolyl, benzothiazolyl, benzothienyl, cinnolinyl, chromanyl, indazolyl, indolyl, imidazolyl, isochromanyl, isoindolyl, isoquinolyl, phthalazinyl, pteridinyl, purinyl, pyridyl, pyrimidinyl, pyridazinyl, pyrazolyl, pyrrolyl, pyrazinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, thiazolyl, thiadiazolyl, thienyl or triazolyl, wherein said heterocyclyl is unsubstituted or mono-, di- or trisubstituted independently of one another by R8, or

2) a monocyclic or bicyclic 4- to 15-membered heterocyclyl, containing one, two, three or four heteroatoms chosen from nitrogen, sulfur or oxygen,
wherein said heterocyclyl is unsubstituted or mono-, di- or trisubstituted independently of one another by R8,

3) a monocyclic or bicyclic 6- to 14-membered aryl, wherein aryl is mono-, di- or trisubstituted independently of one another by R8,

R8 is

1) halogen,
2) $-NO_2$,
3) -CN,
4) $-C(O)-NH_2$,
5) -OH,
6) $-NH_2$,
7) $-O-CF_3$,
8) $-O-(C_1-C_8)$-alkyl, wherein alkyl is unsubstituted or mono-, di- or trisubstituted independently of one another by halogen, $NH_2$, -OH or a methoxy residue,
9) $-(C_1-C_8)$-alkyl, wherein alkyl is unsubstituted or mono-, di- or trisubstituted independently of one another by halogen, $NH_2$, -OH or a methoxy residue,
10) $-SO_2-CH_3$ or
11) $-SO_2-CF_3$,

provided that R8 is at least one halogen, $-C(O)-NH_2$ or $-O-(C_1-C_8)$-alkyl residue,

Q is a direct bond, $-(C_0-C_2)$-alkylene-C(O)-$(C_0-C_2)$-alkyl, $-(C_1-C_6)$-alkylene, $-(C_0-C_3)$-alkylene-$S(O)_2$- or $-(C_0-C_2)$-alkylene-$NR^{10}$-C(O)-O-$(C_0-C_2)$-alkylene, wherein the alkylene residues are unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH,

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen; $-(C_1-C_3)$-perfluoroalkylene, $-(C_1-C_3)$-alkylene-S(O) $-(C_1-C_4)$-alkyl, $-(C_1-C_3)$-alkylene$S(O)_2$-$(C_1-C_3)$-alkyl, $-(C_1-C_3)$-alkylene-$S(O)_2$-$N(R^{4'})$-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a 3- to 7-membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or -($C_1$-$C_4$)-alkylene, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =O, -($C_1$-$C_8$)-alkyl, -($C_1$-$C_4$)-alkoxy, -$NO_2$, -($C_0$-$C_4$)-alkyl-C(O)-O-$R^{18}$, -CN, -($C_0$-$C_4$)-alkyl-N($R^{18}$)-$R^{21}$, -($C_0$-$C_4$)-alkyl-O-$R^{18}$, -($C_0$-$C_4$)-alkyl-het, -($C_0$-$C_8$)-alkyl-$SO_2$-($C_1$-$C_4$)-alkyl, -($C_0$-$C_8$)-alkyl-$SO_2$-($C_1$-$C_3$)-perfluoroalkyl, -($C_0$-$C_8$)-alkyl-$SO_2$-N($R^{18}$)-$R^{21}$, -C(O)-NH-($C_1$-$C_8$)-alkyl, -C(O)-N-[($C_1$-$C_8$)-alkyl]$_2$, -N$R^{18}$-C(O)-NH-($C_1$-$C_8$)-alkyl, -C(O)-$NH_2$, -S-$R^{18}$, or -N$R^{18}$-C(O)-NH-[($C_1$-$C_8$)-alkyl]$_2$, wherein $R^{18}$ and $R^{21}$ are independently from each other hydrogen atom, -($C_1$-$C_3$)-perfluoroalkyl or -($C_1$-$C_6$)-alkyl,

V is
1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, -$(CH_2)_m$-N$R^{10}$-$SO_2$-N$R^{10}$-$(CH_2)_n$-, -$(CH_2)_m$-CH(OH)-$(CH_2)_n$-, -$(CH_2)_m$-, -$(CH_2)_m$-O-$(CH_2)_n$-, -$(CH_2)_m$-C(O)-N$R^{10}$ -$(CH_2)_n$-, -$(CH_2)$-$SO_2$-$(CH_2)_n$-, -$(CH_2)_m$-N$R^{10}$-C(O)-N$R^{10}$-$(CH_2)_n$-, -$(CH_2)_m$-NR-$^{10}$-C(O)-$(CH_2)_n$-, -$(CH_2)_m$-C(O)-$(CH_2)_n$-, -$(CH_2)$-S-$(CH_2)_n$-, -$(CH_2)_m$-$SO_2$-N$R^{10}$-$(CH_2)_n$-, -$(CH_2)_m$-N$R^{10}$-$SO_2$-$(CH_2)_n$-, -$(CH_2)_m$-N$R^{10}$-, -$(CH_2)_m$-O-C(O)-N$R^{10}$-$(CH_2)_n$- or -$(CH_2)_m$-N$R^{10}$-C(O)-O-$(CH_2)_n$-, n and m are independently of one another identical or different and are the integers zero, 1, 2, 3, 4, 5 or 6,

M is
1) a 6- to14-membered aryl selected out of the group phenyl, naphthyl, biphenylyl, 2-biphenylyl, 3-biphenylyl, 4-biphenylyl, anthryl or fluorenyl, wherein aryl is unsubstituted or mono-, di- or trisubstituted independently of one another by R14,
2) a monocyclic or bicyclic 4- to 15-membered heterocyclyl selected out of the group acridinyl, azaindole (1H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1 ,3-dioxolenyl, 3,3-dioxo-(1,3,4)oxathiazine, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1 ,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1 ,3,4-thiadiazolyl, thianthrenyl, 1 ,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1 ,2,3-triazinyl, 1 ,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl, wherein heterocyclyl is unsubstituted or mono-, di- or trisubstituted independently of one another by R14,
3) a 3- to 7-membered cyclic residue, containing 1, 2, 3 or 4 heteroatoms chosen from nitrogen, sulfur or oxygen, selected out of the group azepine, azetidine, aziridine, azirine, 1,4 diazepane, 1,2-diazepine, 1,3-diazepine, 1,4-diazepine, diaziridine, diazirine, dihydroimidazolone, dioxazole, dioxazine, dioxole, 1,3-dioxolene, 1,3-dioxolane, furan, imidazole, imidazoline, imidazolidine, imidazolidinone, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazolone, oxazole, [1,3,4]oxathiazinane 3,3-dioxide, oxaziridine, oxazolidinone, oxetan, oxirane, piperazine, piperidine, pyran, pyrazine, pyrazole, pyrazin-2-one, piperazin-2-one, pyrazoline, pyrazolidine, pyridazine, pyridine, pyridinone, pyrimidine, pyrimidine-2,4-dione, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiomorpholine 1,1-dioxide thiopyran, 1,2,3-triazine, 1,2,4-triazine, 1,3,5-triazine, 1,2,3-triazole or 1,2,4-triazole, wherein said cyclic residue is unsubstituted or mono-, di- or trisubstituted independently of one another by R14, wherein R14 is defined above,

$R^3$, $R^4$, $R^5$ or $R^6$ are independent of one another and are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

    a) hydrogen atom,
    b) -($C_1$-$C_4$)-alkyl, wherein alkyl is unsubstituted or mono-, di- or trisubstituted independently of one another by R13, or
    c) phenyl, wherein phenyl is unsubstituted or mono-, di- or trisubstituted independently of one another by R13,
    d) $-CF_3$, or
    e) $-CHF_2$,

7) $-NO_2$,
8) -CN,
9) $-SO_s-R^{11}$, wherein s is 1 or 2,
10) $-SO_t-N(R^{11})-R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$ ,
14) -($C_0$-$C_4$)-alkylene-N($R^{11}$)-$R^{12}$,
15) $-NR^{10}-SO_2-R^{10}$,
16) $-S-R^{10}$,
17) -($C_0$-$C_2$)alkylene-C(O)-O-($C_2$-$C_4$)-alkylene-O-C(O)-($C_1$-$C_4$)-alkyl,
18) -C(0)-0-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(0)-0- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_4$)-alkylene-O-$CH_2$-($C_1$-$C_3$)-perfluoroalkylene-$CH_2$-O-($C_0$-$C_4$)-alkyl,

26) $-SO_w-N(R^{11})-R^{13}$, wherein w is 1 or 2,
27) $-(C_0-C_4)$-alkylene-C(O)-$N(R^{11})-R^{13}$ ,
28) $-(C_0-C_4)$-alkylene-$N(R^{11})-R^{13}$, or
29) a residue from the following list

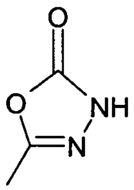
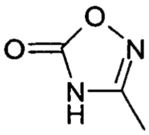
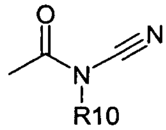
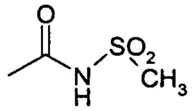
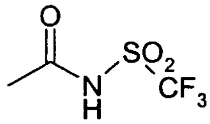
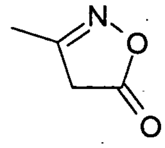
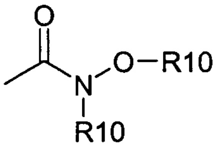
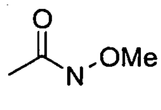
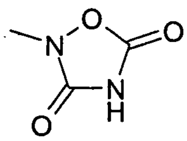
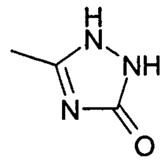
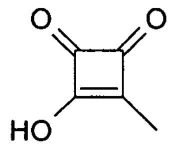
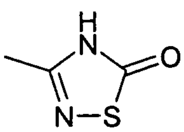
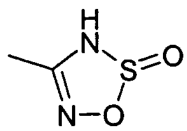
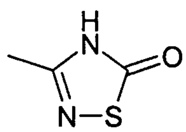
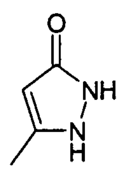
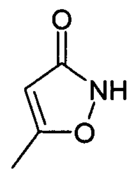
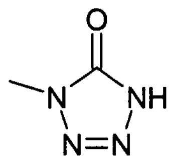
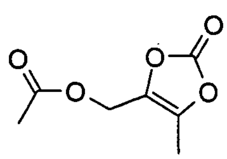
and
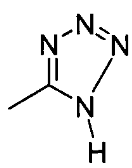

wherein Me is methyl, or if two -OR19 residues are attached to adjacent atoms they can form together with the atoms which they are attached to a 5- or 6- membered ring, which is unsubstituted or substituted one, two, three or four times by R13,
provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or
R3 and R4, or R5 and R6, or R3 and R5 or R4 and R6 together with the carbon atom to which they are bonded can form a 3- to 8-membered ring, containing zero, 1,2,3 or 4 heteroatoms chosen from nitrogen, sulfur or oxygen, wherein said ring is unsubstituted or mono-, di- or trisubstituted independently of one another by R13, wherein R13 is defined below, or
R3 and R5 or R4 and R6 together with the carbon atoms to which they are bonded can form a 4- to 8-membered ring, containing zero, 1, 2, 3 or 4 heteroatoms chosen from nitrogen, sulfur or oxygen, wherein said ring is unsubstituted or mono-, di- or trisubstituted independently of one another by R13, wherein R13 is defined below,
R11 and R12 are independently of one another identical or different and are

1) hydrogen atom,
2) $-(C_1-C_6)$-alkyl, wherein alkyl is unsubstituted or mono-, di- or trisubstituted independently of one another by R13,
3) $-(C_0-C_6)$-alkyl-$(C_3-C_8)$-cycloalkyl,
4) $-SO_t-R^{10}$, wherein t is 1 or 2,
5) $-(C_0-C_6)$-alkyl-$(C_6-C_{14})$-aryl, wherein alkyl and aryl independently from one another are unsubstituted or mono-, di- or trisubstituted by R13,

6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 — together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 — is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-($CH_3$)$_3$, -N($R^{10}$)-S(O)$_u$-$R^{10}$, wherein u is 1 or 2, -S-$R^{10}$, -$SO_r$-$R^{10}$, wherein r is 1 or 2, -S(O)$_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -($C_1$-$C_3$)-perfluoroalkyl, -($C_0$-$C_4$)-alkyl-C (O)-O-C(R15, R16)-O-C(O)-R17, -0-R15, -NH-C(O)-NH-$R^{10}$, -O-$CF_3$, -($C_0$-$C_4$)-alkyl-C(O) -O-C(R15, R16)-O-C(O)-O-R17, -NH-C(O)-O-$R^{10}$, or a residue from the following list

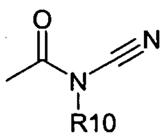 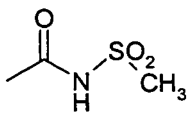 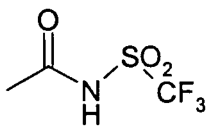 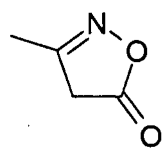 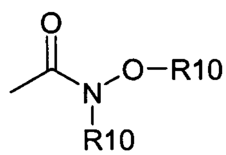

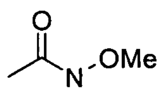 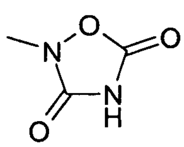 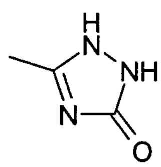 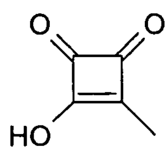 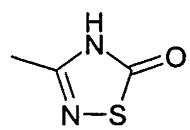

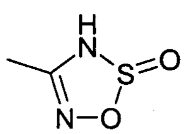 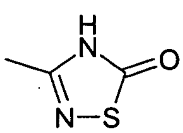 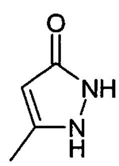 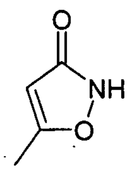 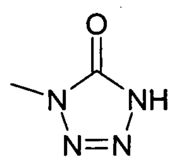

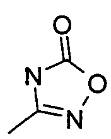 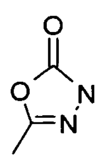 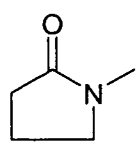 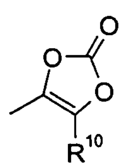 and 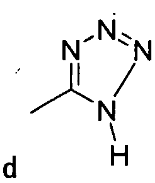 ,

$R^{10}$ and $R^{20}$ are — independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are — independently of one another hydrogen, -($C_1$-$C_6$)-alkyl, or together with the carbon atom to which they are bonded they can form a 3- to 6 membered carbocyclic ring which is unsubstituted or substituted one to three times by $R^{10}$, and

R17is — -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts.

**2.** A compound of formula I as claimed in claim 1, wherein

$R^0$ is 1) a monocyclic or bicyclic 5- to 15-membered heterocyclyl out of the group benzothienyl, indazolyl, indolyl, imidazolyl, isoindolyl, isoquinolyl, phthalazinyl, pyridyl, pyrimidinyl, pyridazinyl, pyrazolyl, pyrazinyl, quinazolinyl, quinolyl, thiazolyl, thiadiazolyl, thienyl or triazolyl, wherein said heterocyclyl is un-substituted or mono-, di- or trisubstituted independently of one another by R8, or
2) a 6- to14-membered aryl selected out of the group phenyl, naphthyl, biphenylyl, 2-biphenylyl, 3-biphenylyl, 4-biphenylyl, anthryl or fluorenyl, wherein aryl is mono-, di- or trisubstituted independently of one another by R8,

R8 is 1) halogen,
2) $-NO_2$,
3) -CN,
4) $-C(O)-NH_2$,
5) -OH,
6) $-NH_2$,
7) $-O-CF_3$
8) $-(C_1-C_8)$-alkyl, wherein alkyl is unsubstituted or mono-, di- or tri-substituted independently of one another by halogen, $NH_2$, -OH or a methoxy residue, or
9) $-O-(C_1-C_8)$-alkyl, wherein alkyl is unsubstituted or mono-, di- or tri-substituted independently of one another by halogen, $NH_2$, -OH or a methoxy residue,
10) $-SO_2-CH_3$ or
11) $-SO_2-CF_3$,

provided that R8 is at least one halogen, $-C(O)-NH_2$ or $-O-(C_1-C_8)$-alkyl residue,

Q is a direct bond, $-(C_0-C_2)$-alkylene-C(O)-$(C_0-C_2)$-alkyl, $-(C_1-C_6)$-alkylene, $-(C_0-C_3)$-alkylene-$SO_2$-, $-(C_0-C_2)$-alkylene-$NR^{10}$-C(O)-O-$(C_0-C_2)$-alkylene, wherein $R^{10}$ is as defined below, and wherein the alkylene residues are unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen;
$-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-$N(R^{4'})$-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V can form a 4- to 8-membered cyclic group, containing 1, 2, 3 or 4 heteroatoms chosen from nitrogen, sulfur or oxygen, wherein said cyclic group is un-substituted or mono-, di- or trisubstituted independently of one another by R14,

R14 is halogen, -OH, =O, $-(C_1-C_8)$-alkyl, $-(C_1-C_4)$-alkoxy, $-NO_2$, $-(C_0-C_4)$-alkyl-C(O)-O-$R^{18}$, -CN, $-(C_0-C_4)$-alkyl-$N(R^{18})$-$R^{21}$, $-(C_0-C_4)$-alkyl-O-$R^{18}$, $-(C_0-C_4)$-alkyl-het, $-(C_0-C_8)$-alkyl-$SO_2$-$(C_1-C_4)$-alkyl, $-(C_0-C_8)$-alkyl-$SO_2$-$(C_1-C_3)$-perfluoroalkyl, $-(C_0-C_8)$-alkyl-$SO_2$-$N(R^{18})$-$R^{21}$, -C(O)-NH-$(C_1-C_8)$-alkyl, -C(O)-N-$[(C_1-C_8)\text{-alkyl}]_2$, $-NR^{18}$-C(O)-NH-$(C_1-C_8)$-alkyl, $-C(O)-NH_2$, $-S-R^{18}$, or $-NR^{18}$-C(O)-NH-$[(C_1-C_8)\text{-alkyl}]_2$,

wherein $R^{18}$ and $R^{21}$ are independently from each other hydrogen atom, $-(C_1-C_3)$-perfluoroalkyl or $-(C_1-C_6)$-alkyl,

V is 1) a 3- to 7-membered cyclic residue, containing 1, 2, 3 or 4 heteroatoms chosen from nitrogen, sulfur or oxygen, wherein said cyclic residue is un-substituted or mono-, di- or trisubstituted independently of one another by R14,
2) a 6- to14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$,$-(CH_2)_m-NR^{10}-C(O)-NR^{10}-(CH_2)_n-$, $-(CH_2)_m-NR^{10}-C(O)-(CH_2)_n-$, $-(CH_2)_m-C(O)-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$,

n and m are independently of one another identical or different and are the integers zero, 1, 2, 3, 4, 5 or 6,

M is 1) a 6- to 14-membered aryl selected out of the group phenyl, naphthyl, biphenylyl, 2-biphenylyl, 3-biphenylyl, 4-biphenylyl, anthryl or fluorenyl, wherein aryl is unsubstituted or mono-, di- or trisubstituted independently of one another by R14,
2) a monocyclic or bicyclic 4- to 15-membered heterocyclyl selected out of the group acridinyl, azaindole (1H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1 ,3-dioxolanyl, 1 ,3-dioxolenyl, 3,3-dioxo-(1,3,4)oxathiazine, 6H- 1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H- indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5- oxadiazolyl, 1,3,4-oxadiazolyl, 1 ,2-oxa-thiepanyl, 1,2-oxathiolanyl, 1,4-oxazepanyl, 1,4- oxazepinyl, 1,2-oxazinyl, 1,3-oxazinyl, 1 , 4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1 ,2,4-thiadiazolyl, 1,2,5-thiadiazolyl, 1 ,3,4-thiadiazolyl, thianthrenyl, 1 ,2-thiazinyl, 1,3- thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3- triazolyl, 1,2,3-triazolyl, 1,2,4-triazolyl, 1,2,5-triazolyl, 1,3,4-triazolyl and xanthenyl, wherein heterocyclyl is unsubstituted or mono-, di- or trisubstituted independently of one another by R14,
3) a 3- to 7-membered cyclic residue, containing 1, 2, 3 or 4 heteroatoms chosen from nitrogen, sulfur or oxygen, selected out of the group azepine, azetidine, aziridine, azirine, 1,4 diazepane, 1,2-diazepine, 1,3-diazepine, 1,4-diazepine, diaziridine, diazirine, dihydroimidazolone, dioxazole, dioxazine, dioxole, 1 ,3-dioxolene, 1,3- dioxolane, furan, imidazole, imidazoline, imidazolidine, imidazolidinone, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4- oxazepane, 1,2-oxazine, 1,3-oxazine, 1,4-oxazine, oxazolone, oxazole, [1,3,4]oxathiazinane 3,3-dioxide, oxaziridine, oxazolidinone, oxetan, oxirane, piperazine, piperidine, pyran, pyrazine, pyrazole, pyrazin-2-one, piperazin-2-one, pyrazoline, pyrazolidine, pyridazine, pyridine, pyridinone, pyrimidine, pyrimidine-2,4-dione, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3- thiazine, 1,4-thiazine, 1 ,3-thiazole, thiazole, thiazolidine, thiazoline, thienyl, thietan, thiomorpholine, thiomorpholine 1,1-dioxide thiopyran, 1,2,3-triazine, 1,2,4-triazine, 1,3,5-triazine, 1,2,3-triazole or 1,2,4-triazole, wherein said cyclic residue is unsubstituted or mono-, di- or trisubstituted independently of one another by R14, wherein R14 is defined above,

$R^3$, $R^4$, $R^5$ or $R^6$ are independent of one another and are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

a) hydrogen atom,
b) -($C_1$-$C_4$)-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$,
e) -$CHF_2$,

7) -$NO_2$,
8) -CN,
9) -$SO_S$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(0)-0-C(R15, R16)-0-C(0)-R17,
19) -($C_0$-$C_2$)akylene-C(O)-O-($C_2$-$C_4$)-alkylene-O-C(O)-O-($C_1$-$C_6$)-alkyl,
20) -C(0)-0- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_3$)-alkylene-O-$CH_2$-($C_1$-$C_3$)-perfluoroalkylene-$CH_2$-O-($C_0$-$C_3$)-alkyl, or

26) $-SO_w-N(R^{11})-R^{13}$, wherein w is 1 or 2,
27) $-(C_0-C_4)$-alkylene-C(O)-$N(R^{11})-R^{13}$,
28) $-(C_0-C_4)$-alkylene-$N(R^{11})-R^{13}$, or
29) a residue from the following list

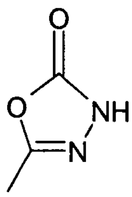 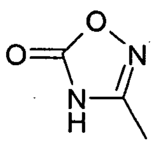 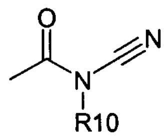 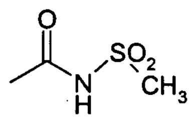 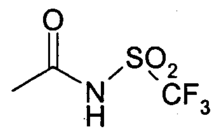

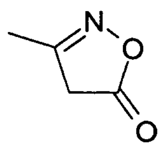 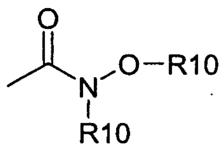 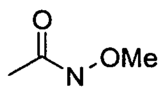 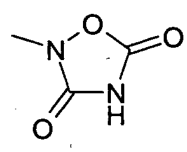

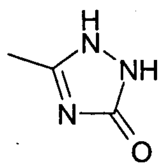 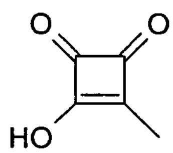 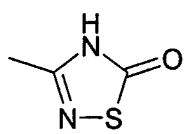 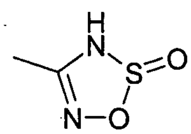 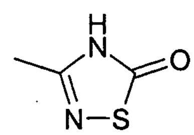

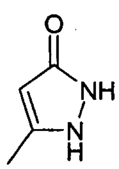 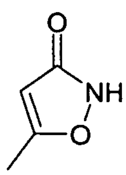 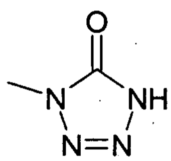 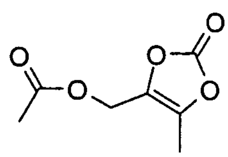

and

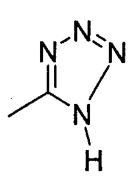

wherein Me is methyl,
if two -OR19 residues are attached to adjacent atoms they can form together with the atoms which they are attached to a 5- or 6- membered ring, which is unsubstituted or substituted one, two, three or four times by R13,
provided that the residue $-NH-CH_2(R^4)-R^3$, which is part of formula I, is not one of the following linkage residues $-NH-CH_2(R^4)-N-$ or $-N-CH_2(R^4)-O-$, wherein R4 is as defined above, or

R3 and R4 or R5 and R6 together with the carbon atom to which they are bonded can form a 3- to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine,

1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or

R3 and R5 or R4 and R6 together with the carbon atoms to which they are bonded can form a 4- to 8-membered ring selected out of the group azetidine, azocane, azocane-2-one, cyclobutyl, cyloheptyl cyclohexyl, cyclooctane, cycloodene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 together with the nitrogen atom to which they are bonded can form a 4-to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -($C_1$-$C_3$)-perfluoroalkyl, -($C_0$-$C_4$)-alkyl-C(O)-O-C(R15, R16)-0-C(0)-R17, -O-R15, -NH-C(O)-NH-$R^{10}$, -O-$CF_3$, -($C_0$-$C_4$)-alkyl-C(O)-O-C(R15, R16)-O-C(O)-O-R17, -NH-C(O)-O-$R^{10}$, or a residue from the following list

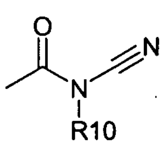 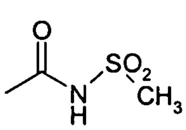 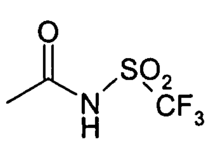 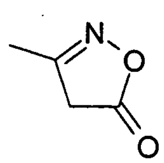 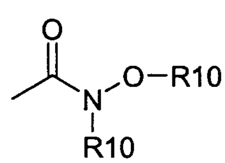

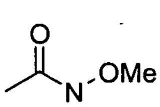 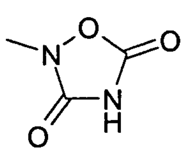 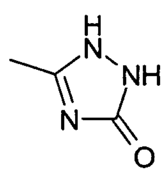 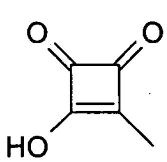 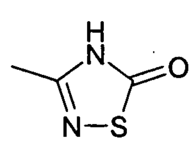 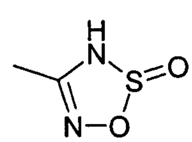

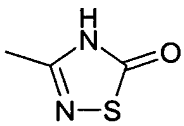 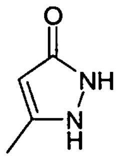 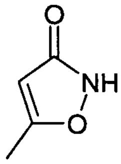 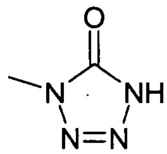 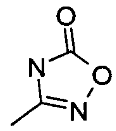 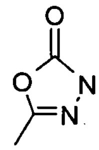 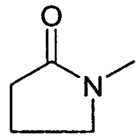

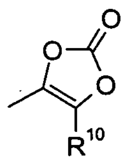 and 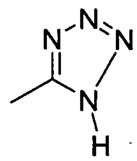 ,

$R^{10}$ and $R^{20}$ are independently of one another hydrogen, $-(C_1-C_6)$-alkyl or $-(C_1-C_3)$-perfluoroalkyl,

R15 and R16 are independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$.

**3.** A compound of the formula I as claimed in claims 1 or 2, wherein

$R^0$ is 1) phenyl or naphthyl, wherein phenyl or naphthyl is mono-, di- or trisubstituted independently of one another by R8,

2) a monocyclic or bicyclic 4- to 15-membered heterocyclyl out of the group benzimidazolyl, 1,3-benzodioxolyl, benzofuranyl, benzoxazolyl, benzothiazolyl, benzothienyl, cinnolinyl, chromanyl, indazolyl, indolyl, isochromanyl, isoindolyl, isoquinolinyl, imidazolyl phenylpyridyl, phthalazinyl, pteridinyl, purinyl, pyridinyl, pyridoimidazolyl, pyridopyrimidinyl, pyridothienyl, pyrimidinyl, quinazolinyl, quinolyl, quinoxalinyl or 1,4,5,6-tetrahydro-pyridazinyl, thiadiazolyl, thiazolyl, thienyl, wherein said heterocyclyl is unsubstituted or mono-, di- or trisubstituted independently of one another by R8, or

3) a heterocyclyl, wherein heterocyclyl is selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1 ,3-dioxolanyl, 1,3-dioxolenyl, 6H-1,5,2-dithiazinyl, dihydrofuro [2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3- oxadiazolyl, 1 , 2,4-oxadiazolyl, 1,2,5-oxadiazolyl, 1,3,4-oxadiazolyl, 1,2-oxa-thiepanyl, 1,2- oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H- quinolizinyl, quinoxalinyl, quinuclidinyl, tetrahydrofuranyl, tetrahydroisoquinolinyl, tetrahydroquinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1 ,2,4-thiadiazolyl, 1,2,5-thiadiazolyl, 1 ,3,4-thiadiazolyl, thianthrenyl, 1 ,2-thiazinyl, 1,3- thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3- triazolyl, 1 ,2,4-triazolyl, 1,2,5-triazolyl, 1 ,3,4-triazolyl and xanthenyl,

wherein said heterocyclyl is unsubstituted or mono-, di- or trisubstituted independently of one another by R8

R8 is
1) halogen,
2) $-NO_2$,
3) -CN,
4) $-C(O)-NH_2$,
5) -OH,
6) $-NH_2$,
7) $-O-CF_3$,
8) $-(C_1-C_8)$-alkyl, wherein alkyl is unsubstituted or mono-, di- or trisubstituted independently of one another by halogen, $NH_2$, -OH or a methoxy residue,
9) $-O-(C_1-C_8)$-alkyl, wherein alkyl is unsubstituted or mono-, di- or trisubstituted independently of one another by halogen, $NH_2$, -OH or a methoxy residue,
10) $-SO_2-CH_3$ or
11) $-SO_2-CF_3$,

provided that R8 is at least one halogen, $-C(O)-NH_2$ or $-O-(C_1-C_8)$-alkyl residue,

Q is $-(C_0-C_2)$-alkylene-C(O)-$(C_0-C_2)$-alkylene- or $-SO_2-$, wherein the alkylene residue is unsubstituted or mono-, di- or trisubstituted independently of one another by halogen, $-NH_2$, -OH or $-(C_3-C_6)$-cycloalkylen, wherein cycloalkylen is unsubstituted or mono-, di- or trisubstituted independently of one another by halogen, $-NH_2$ or -OH;

$R^1$ is hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, an aryl out of the group phenyl, naphthyl, biphenylyl, anthryl or fluorenyl, wherein aryl is mono-, di- or trisubstituted independently of one another by R8,
a monocyclic or bicyclic 4- to 15-membered heterocyclyl,which is as defined above; $-(C_1-C_3)$-perfluoroalkylene, $-(C_1-C_3)$-alkylene-S(O)-$(C_{1-4})$-alkyl, $-(C_1-C_3)$-alkylene-S$(O)_2$-$(C_1-C_3)$-alkyl, $-(C_1-C_3)$-alkylene-S$(O)_2$-N($R^{4'}$)-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a residue selected out of the group azepine, azetidine, aziridine, azirine, 1,4-diazapane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,4- oxazepane, 1,2-oxa-thiepane, 1,2-oxathiolane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, 2,3 dihydroindole, imidazole, imidazoline, imidazolidine, indole, isothiazole, isothiazolidine, isothiazoline, isoxazoline, isoxazolidine, 2-isoxazoline, ketopiperazine, morpholine, 1,4-oxazepane, 1,2-oxathiepane, 1,2-oxathiolane, 1,2-oxazine, 1,3-oxazine, 1,4-oxazine, oxazole, piperazine, piperidine, pyrazine, pyrazole, pyrazoline, pyrazolidine, pyrrole, pyrrolidine, pyrrolidinone, pyrroline, tetrahydropyridine, thiazolidine, thiazoline or thiomorpholine, wherein said cyclic group is unsubstituted or mono-, di- or trisubstituted independently of one another by R14,

R14 is halogen, -OH, =O, $-(C_1-C_8)$-alkyl, $-(C_1-C_4)$-alkoxy, $-NO_2$, $-(C_0-C_4)$-alkyl-C(O)-O-$R^{18}$, -CN,

$-(C_0-C_4)$-alkyl-N($R^{18}$)-$R^{21}$, $-(C_0-C_4)$-alkyl-O-$R^{18}$, $-(C_0-C_4)$-alkyl-het, wherein het is a residue selected from azetidine, azetidinone, piperidine, piperazine, pyridine, pyrimidine, pyrrolidine, pyrrolidinone, 1,2,3-triazine, 1,2,4-triazine, 1,3,5-triazine, 1,2,3- triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, $-(C_0-C_8)$-alkyl-$SO_2$-$(C_{1-4})$-alkyl, $-(C_0-C_8)$-alkyl-$SO_2$-$(C_1-C_3)$-perfluoroalkyl, $-(C_0-C_8)$-alkyl-$SO_2$-N($R^{18}$)-$R^{21}$, -C(O)-NH-$(C_1-C_8)$-alkyl, -C(O)-N-[$(C_1-C_8)$-alkyl]$_2$, -$NR^{18}$-C(O)-NH-$(C_1-C_8)$-alkyl, -C(O)-$NH_2$, -S-$R^{18}$, or -$NR^{18}$-C(O)-NH-[$(C_1-C_8)$-alkyl]$_2$,

wherein $R^{18}$ and $R^{21}$ are independently from each other hydrogen atom, $-(C_1-C_3)$-perfluoroalkyl or $-(C_1-C_6)$-alkyl,

V is
1) an aryl out of the group phenyl or naphthyl, wherein aryl is mono-, di- or trisubstituted independently of one another by R14, or
2) a heterocyclyl out of the group acridinyl, azaindole (1H-pyrrolopyridine), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH- carbazolyl, carbolinyl, chromanyl, chromenyl, cinnolinyl, decahydrochinolinyl, 1,4- diazepane, 4,5-dihydrooxa-zolinyl, dioxazolyl, dioxazinyl, 1 ,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H- indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, odahydroisoquinolinyl, oxadiazolyl, 1,2,3-oxadiazolyl, 1 ,2,4-oxadiazolyl, 1,2,5- oxadiazolyl, 1,3,4-oxadiazolyl, 1,2-oxa-thiepanyl, 1,2-oxathiolanyl, 1,4-oxazepanyl, 1,4- oxazepinyl, 1,2-oxazinyl, 1,3-oxazinyl, 1 ,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1 , 2,5-thiadiazolyl, 1 ,3,4-thiadiazolyl, thianthrenyl, 1,2-thiazinyl, 1,3- thiazinyl, 1 ,4-thiazinyl, 1,3-thiazotyt, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1 , 2,3-triazinyl, 1,2,3-triazolyl, 1,2,3-triazolyl, 1,2,4-triazolyl, 1 ,2,5-triazolyl, 1,3,4-triazolyl and xanthenyl,

wherein said heterocyclyl is unsubstituted or mono-, di- or trisubstituted independently of one another by R14,

G is a direct bond, $-(CH_2)_m$-$NR^{10}$-$SO_2$-$NR^{10}$-$(CH_2)_n$-, $-(CH_2)_m$-, $-(CH_2)_m$-C(O)-$NR^{10}$-$(CH_2)_n$-, $-(CH_2)$-$SO_2$-$(CH_2)_n$-,$-(CH_2)_m$-$NR^{10}$-C(O)-$NR^{10}$-$(CH_2)_n$-, $-(CH_2)_m$-$NR^{10}$-C(O)-$(CH_2)_n$-, $-(CH_2)_m$-C(O)-$(CH_2)_n$-, $-(CH_2)_m$-$SO_2$-$NR^{10}$-$(CH_2)_n$-, $-(CH_2)_m$-$NR^{10}$-$SO_2$-$(CH_2)_n$-,

n and m are independently of one another identical or different and are the integers zero, 1, 2, 3, 4, 5 or 6,

M is heterocyclyl, wherein heterocyclyl is a residue out of the group which can be derived from azepane, azepine, 1,4-diazepane, 1,2-diazepine, 1,3-diazepine, 1,4- diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3- triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

$R^3$,$R^4$, $R^5$ or $R^6$ are independent of one another are identical or different and are

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

   a) hydrogen atom,
   b) -($C_1$-$C_4$)-alkyl, wherein alkyl is unsubstituted or mono-, di- or trisubstituted independently of one another by R13, or
   c) phenyl, wherein phenyl is unsubstituted or mono-, di- or trisubstituted independently of one another by R13,
   d) -$CF_3$,
   e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(O)-O-C(R15, R16)-O-C(O)-R17,
19) -($C_0$-$C_2$)alkylene-C(O)-O-($C_2$-$C_4$)-alkylene-O-C(O)-O-($C_1$-$C_6$)-alkyl,
20) -C(O)-O- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or mono-, di- or trisubstituted independently of one another by R13,
24) -($C_1$-$C_4$)-alkylene-het, wherein het is unsubstituted or mono-, di- or trisubstituted independently of one another by R13,
25) -($C_0$-$C_3$)-alkylene-O-$CH_2$-($C_1$-$C_3$)-perfluoroalkylene-$CH_2$-O-($C_0$-$C_3$)-alkyl,
26) -$SO_w$-N($R^{11}$)-$R^{13}$, wherein w is 1 or 2,
27) -($C_0$-$C_4$)-alkylene-C(O)-N($R^{11}$)-$R^{13}$,
28) -($C_0$-$C_4$)-alkylene-N($R^{11}$)-$R^{13}$, or
29) a residue from the following list

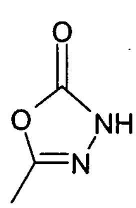 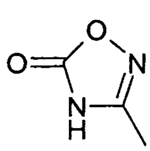 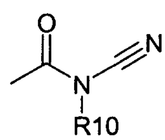 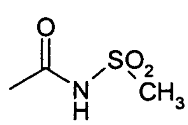 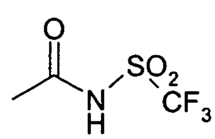

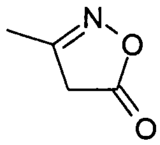 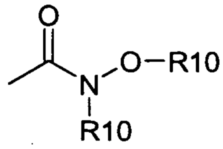 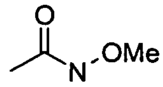 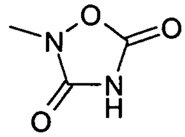 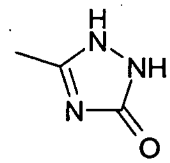

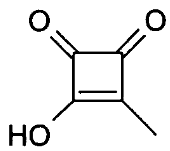 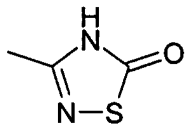 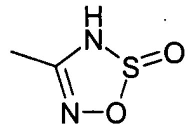 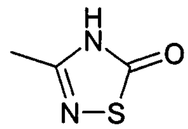 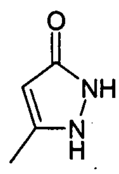 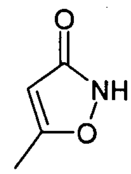

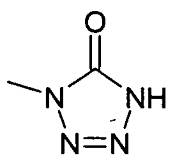 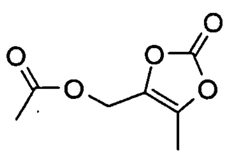 and 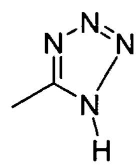

wherein Me is methyl, if two -OR19 residues are attached to adjacent atoms they can form together with the atoms which they are attached to a 5- or 6- membered ring, which is unsubstituted or substituted one, two, three or four times by R13,

provided that the residue -NH-$CH_2(R^4)$-$R^3$, which is part of formula I, is not one of the following linkage residues -NH-$CH_2(R^4)$-N- or -N-$CH_2(R^4)$-O-, wherein R4 is as defined above, or

R3 and R4 or R5 and R6 together with the carbon atom to which they are bonded can form a 3- to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or
R3 and R5 or R4 and R6 together with the carbon atoms to which they are bonded can form a 4- to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,

6) $-(C_1-C_3)$-perfluoroalkyl,
7) $-O-R^{17}$, or
8) $-(C_0-C_6)$-alkyl-$(C_4-C_{15})$-heterocyclyl, wherein alkyl and heterocyclyl are as defined above and are independently from one another unsubstituted or mono-, di- or trisubstituted by R13, or

R11 and R12 together with the nitrogen atom to which they are bonded form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R13 is halogen, $-NO_2$, -CN, =O, -OH, $-CF_3$, $-C(O)-O-R^{10}$, $-C(O)-N(R^{10})-R^{20}$, $-N(R^{10})-R^{20}$, $-(C_3-C_8)$-cycloalkyl, $-(C_0-C_3)$-alkylene-$O-R^{10}$, $-Si-(CH_3)_3$, $-N(R^{10})-S(O)_u-R^{10}$, wherein u is 1 or 2, $-S-R^{10}$, $-SO_r-R^{10}$, wherein r is 1 or 2, $-S(O)_v-N(R^{10})-R^{20}$, wherein v is 1 or 2, $-C(O)-R^{10}$, $-(C_1-C_8)$-alkyl, $-(C_1-C_8)$-alkoxy, phenyl, phenyloxy-, $-O-CF_3$, $-(C_0-C_4)$-alkyl-C(O)-O-C(R15, R16)-O-C(O)-R17, $-(C_1-C_4)$-alkoxy-phenyl, $-(C_0-C_4)$-alkyl-C(O)-O-C(R15, R16)-O-C(O) -O-R17, $-(C_1-C_3)$-perfluoroalkyl, -0-R15, $-NH-C(O)-NH-R^{10}$, $-NH-C(O)-O-R^{10}$, or a residue from the following list

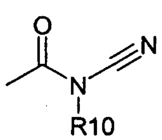 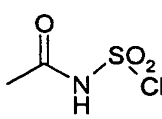 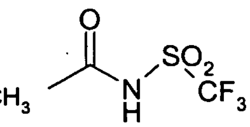 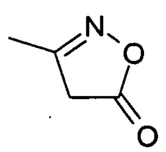 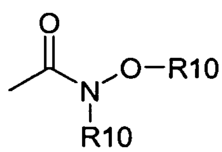

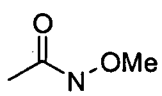 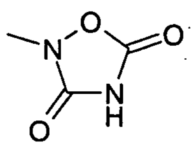 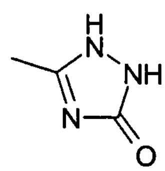 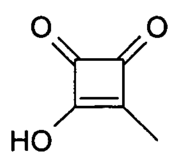 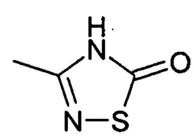 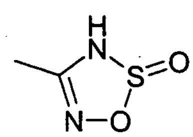

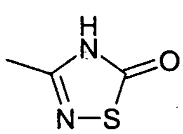 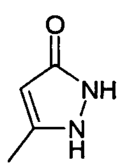 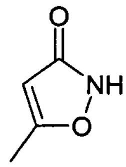 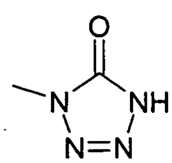 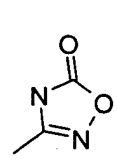 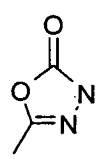 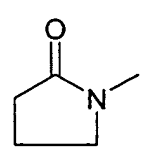

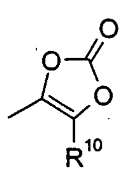 and 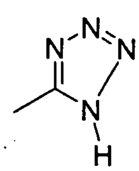 ,

wherein Me is methyl,

$R^{10}$ and $R^{20}$ are independently of one another hydrogen, $-(C_1-C_6)$-alkyl, $-(C_0-C_4)$-alkyl-OH, $-(C_0-C_4)$-alkyl-$O-(C_1-C_4)$-akyl or $-(C_1-C_3)$-perfluoroalkyl, R15 and R16 are independently of one another hydrogen, $-(C_1-C_6)$-alkyl, cyclopropyl, cyclobutyl, cyclopentyl or cyclohexyl, wherein each ring is

unsubstituted or substituted one to three times by $R^{10}$, and

R17 is -$(C_1-C_6)$-alkyl, -$(C_1-C_6)$-alkyl-OH, -$(C_1-C_6)$-alkyl-O-$(C_1-C_6)$-alkyl, -$(C_3-C_8)$-cycloalkyl, -$(C_1-C_6)$-alkyl-O-$(C_1-C_8)$-alkyl-$(C_3-C_8)$-cycloalkyl, -$(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$.

**4.** A compound of formula I as claimed in claims 1 to 3, wherein

$R^0$ is
1) a monocyclic or bicyclic 6- to 14-membered aryl out of the group naphthyl or phenyl, wherein aryl is mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, imidazolyl phenylpyridyl, phthalazinyl, pteridinyl, purinyl, pyridinyl, pyridoimidazolyl, pyridopyrimidinyl, pyridothienyl, pyrimidinyl, quinazolinyl, quinolyl, quinoxalinyl or 1,4,5,6-tetrahydropyridazinyl, thienyl, thiazolyl, thiadiazolyl wherein said heterocyclyl is unsubstituted or mono-, di- or trisubstituted independently of one another by R8, or
3) a heterocyclyl out of the group azabenzimidazolyl, benzimidazolyl, 1,3-benzodioxolyl, benzofuranyl, benzothiazolyl, benzothiophenyl, benzoxazolyl, chromanyl, cinnolinyl, 2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, thiadiazolyl, 2-thienyl or 3-thienyl, wherein said heterocyclyl is unsubstituted or mono-, di- or trisubstituted independently of one another by R8,

R8 is
1. fluorine, chlorine or bromine,
2. -$NO_2$,
3. -CN,
4. -C(O)-$NH_2$,
5. -OH,
6. -$NH_2$,
7. -$OCF_3$,
8. -$(C_1-C_8)$-alkyl, wherein alkyl is unsubstituted or mono-, di- or trisubstituted independently of one another by halogen, $NH_2$, -OH or a methoxy residue, or
9. -O-$(C_1-C_8)$-alkyl, wherein alkyl is unsubstituted or mono-, di- or trisubstituted independently of one another by halogen, $NH_2$, -OH or a methoxy residue,
10. -$SO_2CH_3$ or
11. -$SO_2CF_3$,

provided that R8 is at least one halogen, -C(O)-$NH_2$ or -O-$(C_1-C_8)$-alkyl residue,

Q is a -$(C_0-C_2)$-alkylene-C(O)-$(C_0-C_2)$-alkylene-, -$SO_2$-, wherein the alkylene residue is unsubstituted or mono-, di- or trisubstituted independently of one another by halogen, -$NH_2$ or -OH; or -$(C_3-C_6)$-cycloalkylen, wherein cycloalkylen is unsubstituted or mono-, di- or trisubstituted independently of one another by halogen, -$NH_2$ or -OH;

$R^1$ is a hydrogen atom, -$(C_1-C_4)$-alkyl, wherein alkyl is unsubstituted or substituted one to three times by R13; -$(C_1-C_3)$-alkylene-C(O)-NH-$R^0$, -$(C_1-C_3)$-alkylene-C(O)-O-R15, -$(C_1-C_3)$-perfluoroalkylene, -$(C_1-C_3)$-alkylene-S(O)-$(C_1-C_4)$-alkyl, -$(C_1-C_3)$-alkylene-$S(O)_2$-$(C_1-C_3)$-alkyl, -$(C_1-C_3)$-alkylene-$S(O)_2$-N($R^{4'}$)-$R^{5'}$, -$(C_1-C_3)$-alkylene-O-$(C_1-C_4)$-alkyl, -$(C_0-C_3)$-alkylene-$(C_3-C_8)$-cycloalkyl, or -$(C_0-C_3)$-alkylene-het, wherein het is a residue selected out of the group azepine, azetidine, aziridine, azirine, 1,4-diazepane, 1,2-diazepine, 1,3-diazepine, 1,4-diazepine, diaziridine, diazirine, dioxazole, dioxazine, dioxole, 1 ,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or -($C_1$-$C_4$)-alkyl,

$R^2$ is a direct bond or -($C_{1-4}$)-alkylene,

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, 2,3 dihydroindole, imidazole, imidazoline, imidazolidine, indole, isothiazole, isothiazolidine, isothiazoline, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, piperazine, piperidine, pyrazine, pyrazole, pyrazoline, pyrazolidine, pyrrole, pyrrolidine, pyrrolidinone, pyrroline, tetrahydropyridine, thiazolidine, thiazoline or thiomorpholine, wherein said cyclic group is unsubstituted or mono-, di- or trisubstituted independently of one another by R14,

$R^3$, $R^4$, $R^5$ or $R^6$, are independent of one another are identical or different and are

1) hydrogen atom,
2) fluorine, chlorine or bromine,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

    a) hydrogen atom,
    b) -($C_1$-$C_4$)-alkyl, wherein alkyl is unsubstituted or mono-, di- or trisubstituted independently of one another by R13, or
    c) phenyl; wherein phenyl is unsubstituted or mono-, di- or trisubstituted independently of one another by R13,
    d) -$CF_3$,
    e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(0)-0-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(0)-0- C(R15, R16)-O-C(O)-O-R17,
21) -($C_1$-$C_4$)-alkylene-($C_6$-$C_{14}$)-aryl, wherein aryl is mono-, di- or trisubstituted independently of one another by R13,
22) -($C_1$-$C_4$)-alkylene-($C_4$-$C_{15}$)-heterocyclyl, wherein heterocyclyl is unsubstituted or mono-, di- or trisubstituted independently of one another by R13
23) -($C_1$-$C_4$)-alkylene-($C_3$-$C_8$)-cycloalkyl, wherein cycloalkyl is unsubstituted or

mono-, di- or trisubstituted independently of one another by R13,

24) $-(C_1-C_4)$-alkylene-het, wherein het is unsubstituted or mono-, di- or trisubstituted independently of one another by R13,

25) $-(C_0-C_3)$-alkylene-O-$CH_2$-$(C_1-C_3)$-perfluoroalkylene-$CH_2$-O-$(C_0-C_3)$-alkyl, or

26) a residue from the following list

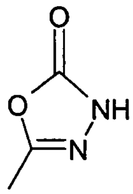 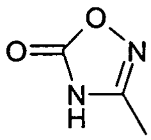 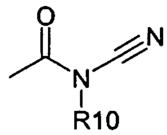 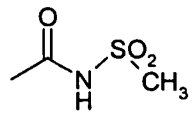 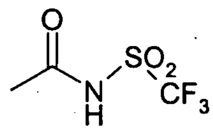

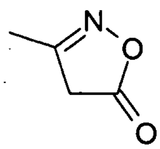 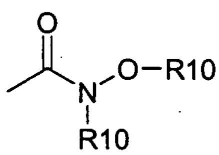 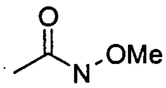 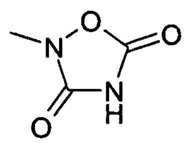 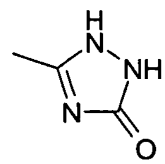

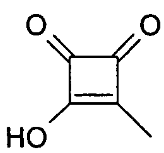 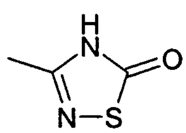 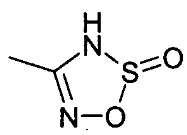 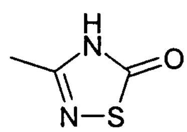 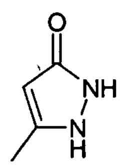 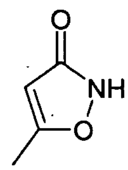

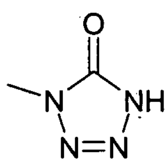 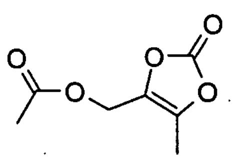 and 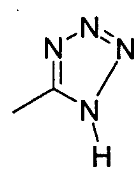

wherein Me is methyl, if two -OR19 residues are attached to adjacent atoms they can form together with the atoms which they are attached to a 1,3-dioxole ring or a 2,3-dihydro-[1,4]dioxine ring, which is substituted one, two, three or four times by R13, provided that the residue -NH-$CH_2(R^4)$-$R^3$, which is part of formula I, is not one of the following linkage residues -NH-$CH_2(R^4)$-N- or -N-$CH_2(R^4)$-O-, wherein R4 is as defined above, or

R3 and R4 or R5 and R6 together with the carbon atom to which they are bonded can form a 3- to 8-membered ring selected out of the group azetidine, azocane, azocane-2-one, cyclobutyl, cyloheptyl cyclohexyl, cycloodane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetane, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below, or

R3 and R5 or R4 and R6 together with the carbon atoms to which they are bonded can form a 4- to 8-membered ring selected out of the group azetidine, azocane, azocane-2-one, cyclobutyl, cyloheptyl cyclohexyl, cyclooctane, cyclooctene, cyclopropyl, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,4-oxazepane, 1,2-oxazine, 1,3-oxazine, 1,4-oxazine, [1,4]oxazocane, [1,3]oxazocan-2-one, oxetarie, oxocane, oxocan-2-one, piperazine, piperidine, pyran, 5,6,7,8-tetrahydro-1H-azocin-2-one, thiomorpholine or tetrahydrofurane, wherein said ring is unsubstituted or mono-, di- or trisubstituted independently of one another by R13, wherein R13 is defined below,

R11 and R12 together with the nitrogen atom to which they are bonded can form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R14 is fluorine, chlorine, bromine, -OH, =O, $-(C_1-C_8)$-alkyl, $-(C_1-C_4)$-alkoxy, $-NO_2$, -C(O)-OH, -CN, $-NH_2$, $-C(O)-O-(C_1-C_4)$-alkyl, $-(C_0-C_8)$-alkyl-$SO_2$-$(C_1-C_4)$-alkyl, $-(C_0-C_8)$-alkyl-$SO_2$-$(C_1-C_3)$-perfluoroalkyl, $-(C_0-C_8)$-alkyl-$SO_2$-$N(R^{18})-R^{21}$, $-C(O)-NH-(C_1-C_8)$-alkyl, $-C(O)-N-[(C_1-C_8)\text{-alkyl}]_2$, $-NR^{18}-C(O)-NH-(C_1-C_8)$-alkyl, $-C(O)-NH_2$, $-S-R^{18}$, or $-NR^{18}-C(O)-NH-[(C_1-C_8)\text{-alkyl}]_2$,

wherein $R^{18}$ and $R^{21}$ are independently from each other hydrogen atom, $-(C_1-C_3)$-perfluoroalkyl or $-(C_1-C_6)$-alkyl,

V is

1) a het residue out of the group azaindole (1H-pyrrolopyridine), azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine, thiadiazole, 1,2-thiazine, of 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently one another by R14, or

2) phenyl, wherein phenyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_n-$, $-(CH_2)_m-NR^{10}-C(O)-(CH_2)_n-$, $-(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$,

n and m are independently of one another identical or different and are the integers zero, 1, 2, 3, 4, 5 or 6,

M is

1) phenyl or naphthyl, wherein phenyl or naphthyl are unsubstituted or mono-, di- or trisubstituted independently of one another by R14,

2) heterocyclyl, wherein heterocyclyl is a residue out of the group which can be derived from azepane, azepine, 1,4-diazepane, 1,2-diazepine, 1,3-diazepine, 1,4- diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3- triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

R11 and R12 are independently of one another identical or different and are

1) hydrogen atom,
2) $-(C_1-C_6)$-alkyl, wherein alkyl is unsubstituted or mono-, di- or trisubstituted independently of one another by R13,
3) $-(C_0-C_6)$-alkyl-$(C_6-C_{14})$-aryl, wherein aryl is as defined above and wherein alkyl and aryl are independently from one another unsubstituted or mono-, di- or trisubstituted by R13,
4) $-O-R^{17}$, or
5) $-(C_0-C_6)$-alkyl-$(C_4-C_{15})$-heterocyclyl, wherein alkyl and heterocyclyl is as defined above and independently from one another are unsubstituted or mono-, di- or trisubstituted by R13, or

R11 and R12 together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2- isoxazoline, ketopiperazine, morpholine, [1,4] oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, which is unsubstituted or mono-, di- or trisubstituted independently of one another by R13,

R13 isfluorine, chlorine, bromine, iodine, $-NO_2$, -CN, =O, -OH, $-CF_3$, $-C(O)-O-R^{10}$, $-C(O)-N(R^{10})-R^{20}$, $-N(R^{10})-R^{20}$, $-(C_0-C_3)$-alkylene-$O-R^{10}$, $-Si-(CH_3)_3$, $-N(R^{10})-S(O)_2-R^{10}$, $-S-R^{10}$, $-SO_2-R^{10}$, $-S(O)_2-N(R^{10})-R^{20}$, $-C(O)-R^{10}$, $-(C_1-C_8)$-alkyl, $-(C_1-C_8)$-alkoxy, phenyl, phenyloxy-, $-O-CF_3$, $-(C_1-C_3)$-perfluoroalkyl, $-(C_0-C_4)$-alkyl-C(O)-O-C(R15, R16)-0-C(0) -R17, $-(C_1-C_4)$-alkoxy-phenyl, $-(C_0-C_4)$-alkyl-C(O)-O-C(R15, R16)-O-C(O)-O-R17, -O-R15, $-NH-C(O)-NH-R^{10}$, -NH-C (O)-$O-R^{10}$, or a residue from the following list

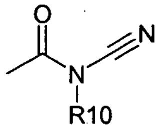 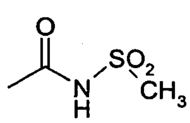 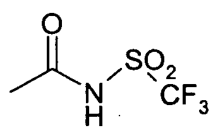 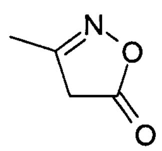 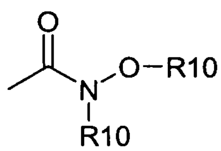

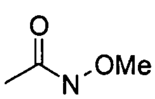 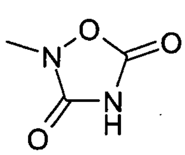 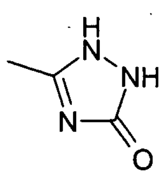 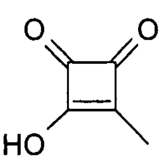 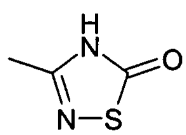 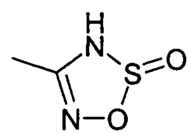

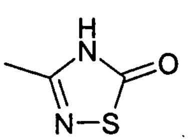 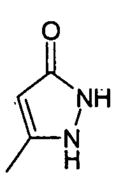 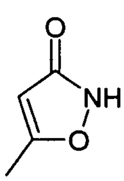 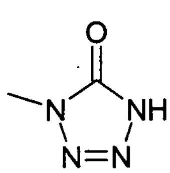 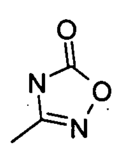 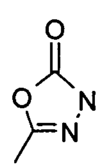 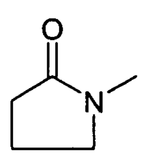

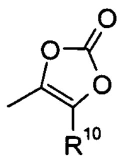

and

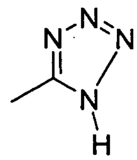

,

wherein Me is methyl,

$R^{10}$ and $R^{20}$ are independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$.

**5.** A compound of formula I as claimed in claims 1 to 4, wherein

R0 is 1) phenyl, wherein phenyl is unsubstituted or mono-, di- or trisubstituted independently of one another by R8,

2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or

3) a heterocyclyl out of the group imidazolyl, pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, pyrazolyl, pyrrolyl, wherein said heterocyclyl is unsubstituted or mono-, di- or trisubstituted independently of one another by R8,

R8 is 1. F, Cl, Br or J,

2. -C(O)-$NH_2$,

3. -($C_1$-$C_4$)-alkyl, wherein alkyl is unsubstituted or mono-, di- or trisubstituted independently of one another by halogen, -OH or a methoxy residue, or

4. -O-($C_1$-$C_4$)-alkyl, wherein alkyl is unsubstituted or mono-, di- or trisubstituted independently of one another by halogen or a methoxy residue,

provided that R8 is at least one F, Cl, Br, J, -C(O)-$NH_2$ or -O-($C_1$-$C_4$)-alkyl residue,

Q is a -($C_0$-$C_2$)-alkylene-C(O)-($C_0$-$C_2$)-alkylene-, -$SO_2$-,

$R^1$ is hydrogen atom, -($C_1$-$C_2$)-alkyl, -($C_1$-$C_3$)-alkylene-C(O)-NH-R0, -($C_1$-$C_3$)-perfluoroalkylene, -($C_1$-$C_3$)-alkylene-C(O)-O-$R^{15}$, -($C_1$-$C_3$)-alkylene-S(O)$_2$-($C_1$-$C_3$)-alkyl or -($C_1$-$C_3$)-alkylene-S(O)$_2$-N($R^{4'}$)-$R^{5'}$,

wherein $R^{4'}$ and $R^{5'}$ are independent of one another are identical or different and are hydrogen atom or -($C_1$-$C_4$)-alkyl,

$R^2$ is a direct bond or -($C_1$-$C_2$)-alkylene,

$R^1$-N-$R^2$-V can form a 4- to 7- membered cyclic group out of the group azetidine, azetidinone, 2,3 dihydroindole, indole, piperidine, piperazine, pyridine, pyrrole, pyrazole, pyrimidine, pyrrolidine, pyrrolidinone, 1,2,3-triazine, 1,2,4-triazine, 1,3,5- triazine, 1,2,3-triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14,

R14 is fluorine, chlorine, -OH, =O, $-(C_1-C_8)$-alkyl, -C(O)-OH, -CN, $-NH_2$, $-C(O)-O-(C_1-C_4)$-alkyl, $-C(O)-NH-(C_1-C_8)$-alkyl, $-C(O)-N-[(C_1-C_8)\text{-alkyl}]_2$ or $-C(O)-NH_2$ or $-N(R^{18})-R^{21}$, wherein $R^{18}$ and $R^{21}$ are independently from each other hydrogen atom, $-(C_1-C_3)$-perfluoroalkyl or $-(C_1-C_4)$-alkyl,

V is 1) a cyclic residue out of the group selected from compounds which are derived from azaindole (1H-pyrrolopyridine), aziridine, azirine, azetidine, azetidinone, 1,4-diazepane, pyrrole, pyrrolidine, pyridonyl, imidazole, pyrazole, 1,2,3-triazole, 1,2,4- triazole, tetrazole, pyridine, pyrimidine, pyridazine, pyrazine, 1,2,3-triazine, 1,2,4- triazine, 1,3,5-triazine, tetrazine, tetrazole, azepine, diazirine, 1,2-diazepine, 1,3- diazepine, 1,4-diazepine, pyridine, pyrazine, pyrimidine, pyridazine, piperidine,

piperazine, pyrrolidinone, ketopiperazine, furan, pyran, dioxole, 1,4-oxazepane, oxazole, isoxazole, 2-isoxazoline, isoxazolidine, morpholine, oxirane, oxaziridine, 1,3-dioxolene, 1,3-dioxolane, 1,2-oxazine, 1,3-oxazine, 1,4-oxazine, oxaziridine, thiophene, thiopyran, thietan, thiazole, isothiazole, isothiazoline, isothiazolidine, 1,2-okathiolan, thiadiazole, thiopyran, 1,2-thiazine, 1,3-thiazole, 1,3-thiazine, 1,4-thiazine, thiadiazine or thiomorpholine,

wherein said cyclic residue is unsubstituted or mono-, di- or trisubstituted independently of one another by R14, or

2) phenyl, wherein phenyl is unsubstituted or mono-, di- or trisubstituted independently of one another by R14,

G is a direct bond, $-(CH_2)_m-$, $-(CH_2)_m-C(O)-NR^{10}$ $-(CH_2)_n-$, $-(CH_2)_m-C(O)-(CH_2)_n-$ or $-(CH_2)_m-NR^{10}-$

n and m are independently of one another identical or different and are the integers zero, 1, 2, 3 or 4,

M is heterocyclyl, wherein heterocyclyl is a residue out of the group which can be derived from azepane, azepine, 1,4-diazepane, 1,2-diazepine, 1,3-diazepine, 1,4- diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3- triazine, 1,2,4-triazine, 1,3,5-triazine, 1,2,3-triazole or 1,2,4-triazole, wherein said heterocyclyl is unsubstituted or mono-, di- or trisubstituted independently of one another by R14,
provided that M contains or is substituted with at least with one oxo-residue,

$R^3$, $R^4$, $R^5$ or $R^6$ are independent of one another are identical or different and are

1) hydrogen atom,
2) fluorine, chlorine or bromine,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

    a) hydrogen atom,
    b) $-(C_1-C_4)$-alkyl, wherein alkyl is unsubstituted or mono-, di- or trisubstituted independently of one another by R13, or
    c) phenyl, wherein phenyl is unsubstituted or mono-, di- or trisubstituted independently of one another by R13,
    d) $-CF_3$ or
    e) $-CHF_2$,

7) $-NO_2$,

8) -CN,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-NR^{10}-SO_2-R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(0)-0-C(R15, R16)-0-C(0)-R17,
19) $-(C_0-C_2)$alkylene-C(O)-O-$(C_2-C_4)$-alkylene-O-C(O)-O-$(C_1-C_6)$-alkyl,
20) -C(O)-O- C(R15, R16)-O-C(O)-O-R17,
21) $-(C_1-C_4)$-alkylene-$(C_6-C_{14})$-aryl, wherein aryl is mono-, di- or trisubstituted independently of one another by R13,
22) $-(C_1-C_4)$-alkylene-$(C_4-C_{15})$-heterocyclyl, wherein heterocyclyl is unsubstituted or mono-, di- or trisubstituted independently of one another by R13
23) $-(C_1-C_4)$-alkylene-$(C_3-C_8)$-cycloalkyl, wherein cycloalkyl is unsubstituted or mono-, di- or trisubstituted independently of one another by R13,
24) $-(C_1-C_4)$-alkylene-het, wherein het is unsubstituted or mono-, di- or trisubstituted independently of one another by R13,
25) $-(C_0-C_3)$-alkylene-O-$CH_2$-$(C_1-C_3)$-perfluoroalkylene-$CH_2$-O-$(C_0-C_3)$-alkyl, or
26) a residue from the following list

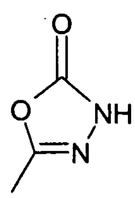 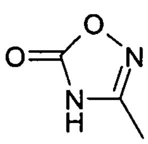 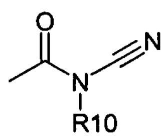 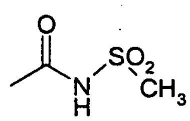 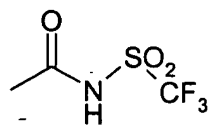

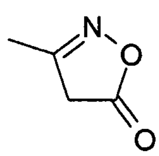 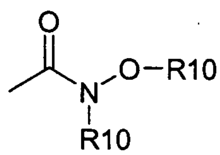 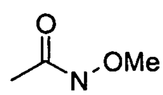 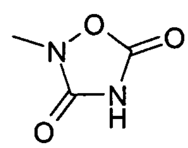 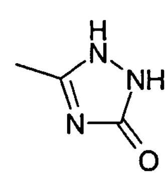

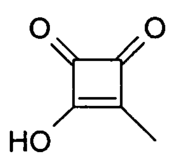 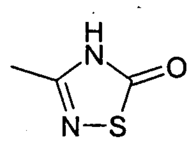 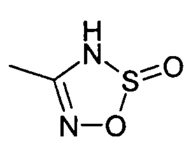 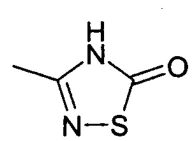 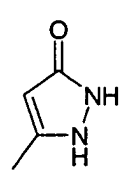

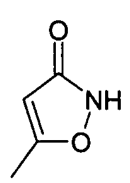 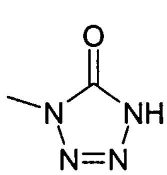 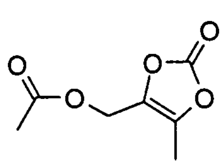 and 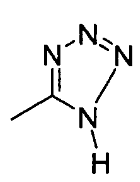

wherein Me is methyl, or
if two -OR19 residues are attached to adjacent atoms they can form together with the atoms which they are attached to a 1,3-dioxole ring or a 2,3-dihydro-[1,4]dioxine ring, which is substituted one, two, three or four times by R13,

provided that the residue -NH-$CH_2(R^4)$-$R^3$, which is part of formula I, is not one of the following linkage residues -NH-$CH_2(R^4)$-N- or -N-$CH_2(R^4)$-O-, wherein R4 is as defined above, or

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,
4) -O-$R^{17}$, or
5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

$R^{11}$ and $R^{12}$ together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]-oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_0$-$C_3$)-alkylene-O-$R^{10}$, -Si-$(CH_3)_3$, -N($R^{10}$)-S$(O)_2$-$R^{10}$, -S-$R^{10}$, -$SO_2$-$R^{10}$, -S$(O)_2$-N($R^{10}$)-$R^{20}$, -C(O)-$R^{10}$, -($C_1$-$C_8$)-alkyl, -($C_1$-$C_8$)-alkoxy, phenyl, phenyloxy-, -O-$CF_3$, -($C_1$-$C_3$)-perfluoroalkyl, -NH-C(O)-NH-$R^{10}$, -($C_0$-$C_4$)-alkyl-C(O)-O-C(R15, R16)-0-C(0)-R17, -($C_1$-$C_4$)-alkoxy-phenyl, -($C_0$-$C_4$)-alkyl-C(O)-O-C(R15, R16)-O-C(O)-O-R17, -O-R15, -NH-C(O)-O-$R^{10}$, or a residue from the following list

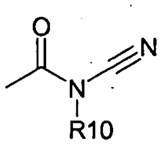 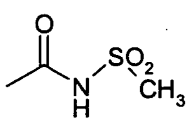 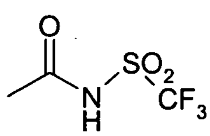 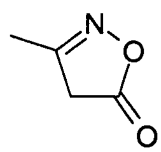 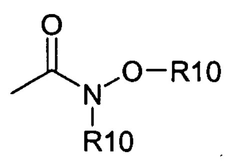

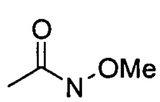 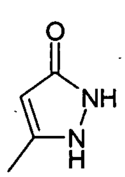 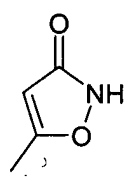 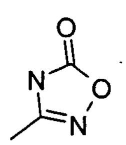 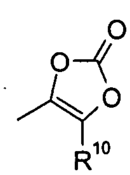 and, 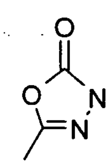

wherein Me is methyl,

$R^{10}$ and $R^{20}$ are independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl,

R15 and R16 are independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$.

**6.** A compound of formula I as claimed in claims 1 to 5, wherein

R0 is a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8,

R8 is
1. F, Cl, Br, J,
2. $-C(O)-NH_2$,
3. $-(C_1-C_4)$-alkyl, wherein alkyl is unsubstituted or mono-, di- or trisubstituted independently of one another by halogen, -OH or a methoxy residue, or
4. $-O-(C_1-C_4)$-alkyl, wherein alkyl is unsubstituted or mono-, di- or trisubstituted independently of one another by halogen or a methoxy residue,

provided that R8 is at least one F, Cl, Br, J, $-C(O)-NH_2$ or $-O-(C_1-C_4)$-alkyl residue,

Q is a $-(C_0-C_2)$-alkylene-C(O)-$(C_0-C_2)$-alkylene-, $-SO_2-$,

$R^1$ is hydrogen atom or $-(C_1-C_2)$-alkyl,

$R^2$ is a direct bond or $-(C_1-C_2)$-alkylen, or

$R^1$-N-$R^2$-V can form a 4- to 7- membered cyclic group out of the group azepine, 1,2- diazepine, 1,3-diazepine, 1,4-diazepine, 2,3 dihydroindole, indole, indazole, isothiazole, isoxazole, isoxazolidine, 2-isoxazoline, ketopiperazine, morpholine, oxazole, oxepane, piperidine, piperazine, pyridine, pyrimidine, pyrrole, pyrazole, pyrrolidine, pyrrolidinone, 1,2,3-triazine, 1,2,4-triazine, 1,3,5-triazine, 1,2,3-triazole, 1,2,4-triazole, tetrazine, tetrazole, thiadiazole, thiazole or thiomorpholine, wherein said cyclic group is unsubstituted or mono-, di- or trisubstituted independently of one another by R14,

R14 is fluoro, chlorine, =O, $-(C_1-C_4)$-alkyl or $-NH_2$,

V is
1. a cyclic residue out of the group containing compounds, which are derived from azaindolyl (1H-pyrrolopyridyl), azetidine, azepine, aziridine, azirine, 1,4-diazepane, 1,2-diazepine, 1,3-diazepine, 1,4-diazepine, diazirine, 1,3-dioxolane, dioxazole, furan, imidazole, isoquinoline, isothiazole, isothiazolidine, isothiazoline, isoxazole, 2-isoxazoline, isoxazolidine, ketopiperazine, morpholine, 1,2-oxazine, 1,3-oxazine, 1,4-oxazine, oxazole, 1,2-oxathiolan, piperidine, pyran, pyrazine, pyrazole, pyridazine, piperazine, pyridine, pyridone, pyrimidine, pyrrole, pyrrolidine, pyrrolidinone, quinazoline, quinoline, tetrazine, tetrazole, thiadiazine, thiadiazole, 1,2-thiazine, 1,3- thiazine, 1,4-thiazine, 1,3-thiazole, thietan, thiomorpholine, thiophene, thiopyran, 1,2,3-triazine, 1,2,4-triazine, 1,3,5-triazine, 1,2,3-triazole or 1,2,4-triazole,
wherein said cyclic residue is unsubstituted or mono-, di- or trisubstituted independently of one another by R14, or
2. phenyl, wherein phenyl is unsubstituted or mono-, di- or trisubstituted independently of one another by R14,

G is a direct bond, $-(CH_2)_m-$, $-(CH_2)_m-C(O)-NR^{10}-(CH_2)_n-$ or $-(CH_2)_m-C(O)-(CH_2)_n-$,

n and m are independently of one another identical or different and are the integers zero, 1, 2, 3 or 4,

M is heterocyclyl, wherein heterocyclyl is a residue out of the group which can be derived from azepane, azepine, 1,4-diazepane, 1,2-diazepine, 1,3-diazepine, 1,4- diazepine, dihydropyrimidinone, dihydroimidazolone, 3,3-dioxo-(1,3,4)oxathiazine, imidazole, imidazolidinone, isothiazole, isoxazole, isoxazolidine, 2-isoxazoline, ketomorpholine, ketopiperazine, morpholine, morpholinone, oxazole, oxazolone, oxazolidinone, [1,4]-oxazepane, piperazine, piperazinone, piperidine, piperidinone, pyrazine, pyridazine, pyridazinone, pyridine, pyridone, pyrimidine-2,4-dione, pyrimidine, pyrimidinone, pyrimidinedione, pyrrolidine, pyrrolidinone, tetrahydropyran, 1,4,5,6-tetrahydro-pyridazinyl, tetrahydro-pyrimidinone, tetrazine, tetrazole, thiadiazole, thiazole, thiophene, thiomorpholine, thiomorpholine 1,1-dioxide, pyrazinone, 1,2,3- triazine, 1,2,4-triazine, 1,3,5-triazine, 1,2,3-triazole or 1,2,4-triazole, wherein said heterocyclyl is unsubstituted or mono-, di- or trisubstituted independently of one another by R14,
provided that M contains or is substituted with at least with one oxo-residue,

$R^3$, $R^4$, $R^5$ or $R^6$, are independent of one another are identical or different and are

1) hydrogen atom,
2) fluorine, chlorine or bromine,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

   a) hydrogen atom,
   b) -($C_1$-$C_4$)-alkyl, wherein alkyl is unsubstituted or mono-, di- or trisubstituted independently of one another by R13, or
   c) phenyl, wherein phenyl is unsubstituted or mono-, di- or trisubstituted independently of one another by R13,
   d) -$CF_3$ or
   e) $CHF_2$,

7) -CN,
8) -($C_1$-$C_4$)-alkylene-($C_4$-$C_{15}$)-heterocyclyl, wherein heterocyclyl is as defined above and is unsubstituted or mono-, di- or trisubstituted independently of one another by R13,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -($C_1$-$C_4$)-alkylene-het, wherein het is as defined above and is unsubstituted or mono-, di- or trisubstituted independently of one another by R13,
17) -($C_0$-$C_2$)alkylene-C(O)-($C_2$-$C_4$)-alkylene-O-C(O)-($C_1$-$C_4$)-alkyl,
18) -C(O)-O-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(O)-O- C(R15, R16)-O-C(O)-O-R17,
21) -($C_1$-$C_4$)-alkylene-($C_6$-$C_{14}$)-aryl, wherein aryl is as defined above and is mono-, di- or trisubstituted independently of one another by R13,
22) -($C_1$-$C_4$)-alkylene-($C_3$-$C_8$)-cycloalkyl, wherein cycloalkyl is unsubstituted or mono-, di- or trisubstituted independently of one another by R13,
23) -($C_0$-$C_3$)-alkylene-O-$CH_2$-$CF_2$-$CH_2$-O-($C_0$-$C_3$)-alkyl,
24) -($C_0$-$C_3$)-alkylene-O-$CH_2$-$CF_2$-$CF_2$-$CH_2$-O-($C_0$-$C_3$)-alkyl,
25) -($C_0$-$C_3$)-alkylene-O-$CH_2$-($C_1$-$C_3$)-perfluoroalkylene-$CH_2$-OH, or
26) a residue from the following list

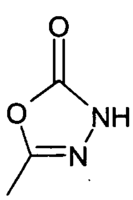 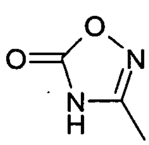 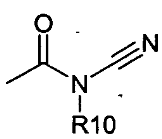 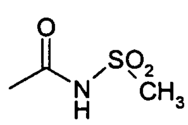 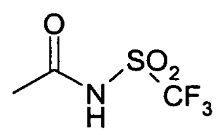

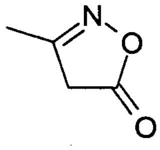 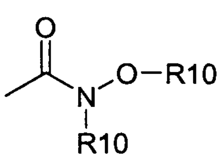 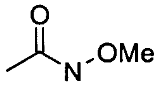 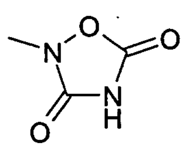 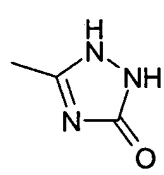

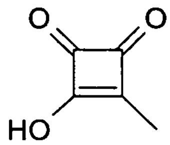 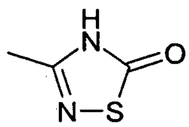 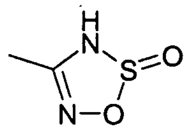 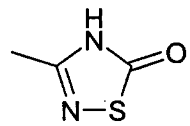 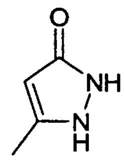 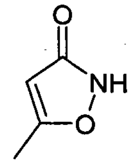

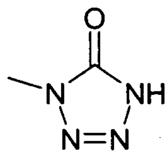 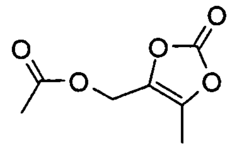 and 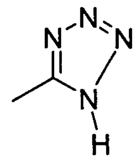 ,

wherein Me is methyl, if two -OR19 residues are attached to adjacent atoms they can form together with the atoms which they are attached to a 1,3-dioxole ring or a 2,3-dihydro-[1,4]dioxine ring, which is substituted one, two, three or four times by R13, provided that the residue -NH-$CH_2(R^4)$-$R^3$, which is part of formula I, is not one of the following linkage residues -NH-$CH_2(R^4)$-N- or -N-$CH_2(R^4)$-O-, wherein R4 is as defined above, or

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,
4) -O-$R^{17}$, or
5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

$R^{11}$ and $R^{12}$ together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]-oxazepane, 1,4-oxazepine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, $-NO_2$, -CN, =O, -OH, $-CF_3$, -C(O)-O-$R^{10}$, -C(O)-N($R^{10}$)-$R^{20}$, -N($R^{10}$)-$R^{20}$, -($C_0$-$C_3$)-alkylene-O-$R^{10}$, -Si-$(CH_3)_3$, -N($R^{10}$)-$S(O)_2$-$R^{10}$, -S-$R^{10}$, $-SO_2$-$R^{10}$, -S$(O)_2$-N($R^{10}$)-$R^{20}$, -C(O)-$R^{10}$, -($C_1$-$C_8$)-alkyl, -($C_1$-$C_8$)-alkoxy, phenyl, phenyloxy-, -O-$CF_3$, -($C_1$-$C_3$)-perfluoroalkyl, -NH-C(O)-NH-$R^{10}$, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -O-R15, -NH-C(O)-O-$R^{10}$, or a residue from the following list

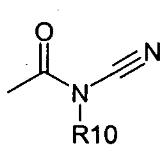 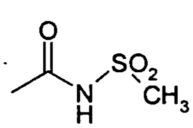 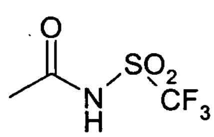 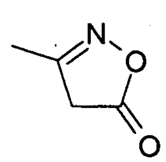 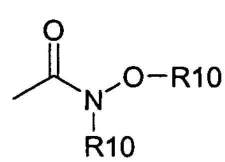

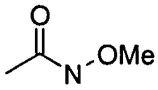 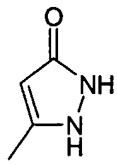 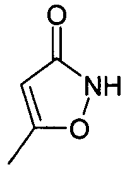 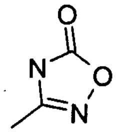 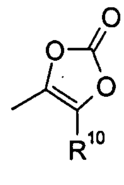 and, 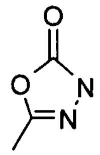

| | |
|---|---|
| | wherein Me is methyl, |
| $R^{10}$ and $R^{20}$ are | independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl; |
| R15 and R16 are | independently of one another hydrogen, $-(C_1-C_6)$-alkyl, or together form a ring out of the droup cyclopropyl, cyclobutyl, cyclopentyl or cyclohexyl, wherein each ring is unsubstituted or substituted one to three times by $R^{10}$, and |
| R17 is | $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$. |

**7.** A compound of formula I as claimed in claims 1 to 6, wherein the compound of the formula I is

5-Chloro-thiophene-2-carboxylic acid {2-[4-(3-oxo-morpholin-4-yl)-phenylcarbamoyl]-ethyl}- amide,
5-Chloro-thiophene-2-carboxylic acid {2-[4-(2-oxo-2H-pyrazin-1-yl)-phenylcarbamoyl]-ethyl}-amide,
4-Chloro-N-{2-[4-(3-oxo-morpholin-4-yl)-phenylcarbamoyl]-ethyl}-benzamide, 5-Chloro-thiophene-2-carboxylic acid {3-oxo-3-[5-(2-oxo-2H-pyrazin-1-yl)-2,3-dihydro-indol-1-yl]-propyl}-amide,
5-Chloro-thiophene-2-carboxylic acid {3-oxo-3-[5-(2-oxo-2H-pyrazin-1-yl)-indol-1-yl]-propyl}-amide,
5-Chloro-thiophene-2-carboxylic acid {2-[2-fluoro-4-(2-oxo-2H-pyrazin-1-yl)-phenylcarbamoyl]-ethyl}-amide,
5-Chloro-thiophene-2-carboxylic acid {2-[4-(2-dimethylaminomethyl-imidazol-1-yl)-2-fluoro- phenylcarbamoyl]-ethyl}-amide,
3-(5-Chloro-thiophene-2-sulfonylamino)-N-[4-(3-oxo-morpholin-4-yl)-phenyl]-propionamide,
5-Chloro-thiophene-2-carboxylic acid {2-[4-(3,3-dioxo-3$l6-[1,3,4]oxathiazinan-4-yl)-phenylcarbamoyl]-ethyl}-amide,
5-Chloro-thiophene-2-carboxylic acid {2,2-difluoro-2-[4-(2-oxo-2H-pyrazin-1-yl)-phenylcarbamoyl]-ethyl}-amide,
5-Chloro-thiophene-2-carboxylic acid {2,2-difluoro-2-[4-(3-oxo-morpholin-4-yl)-phenylcarbamoyl]-ethyl}-amide.

**8.** A process for the preparation of a compound of the formula I as claimed in claims 1 to 7, which comprises condensing a compound of the formula 2 with a compound of the formula $HR^{8'}$ to give a compound of the formula 3 and optionally converting the compound of the formula 3 into a compound of the formulae I and Ia,

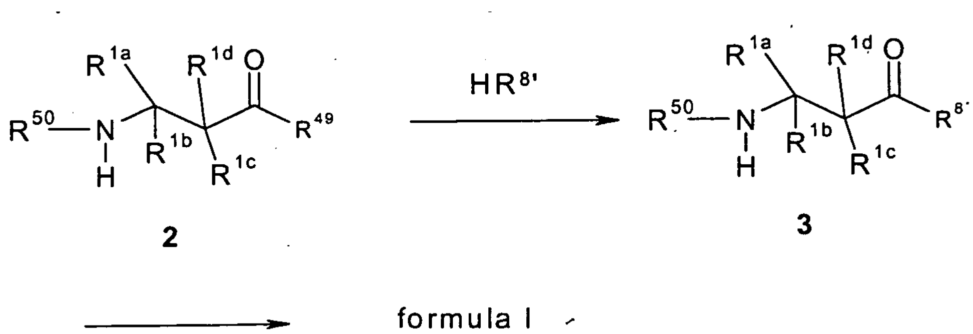


wherein the residue $R^{8'}$ has the donation of $-N(R^1)-R^2$-V-G-M as indicated in claims 1 to 7, but where in $R^{8'}$ functional groups can also be present in the form of groups that are subsequently transformed into the final functional groups present in $-N(R^1)-R^2$-V-G-M, and where the residue $R^{50}$ denotes the group $-Q-R^o$ or can denote a group which is subsequently transformed into the group $-Q-R^o$, and where the group $-C(O)-R^{49}$ can be a carboxylic acid group or derivatives thereof, and where the groups $R^{1a}$, $R^{1b}$, $R^{1c}$ and $R^{1d}$ in the formulae 2 and 3 have the corresponding

definitions of R3, R4, R5 and R6 in formula I as defined in claims 1 to 7 or functional groups in them can also be present in protected form or in the form of precursor groups.

**9.** A pharmaceutical preparation, comprising at least one compound of the formula I as claimed in one or more of claims 1 to 7 in all its stereoisomeric forms and mixtures thereof in any ratio and/or its physiologically tolerable salts and a pharmaceutically acceptable carrier.

**10.** The use of a compound of the formula I as claimed in one or more of claims 1 to 7 in all its stereoisomeric forms and mixtures thereof in any ratio and/or their physiologically tolerable salts for the production of pharmaceuticals for inhibition of factor Xa and/or factor VIIa or for influencing blood coagulation or fibrinolysis.

**11.** The use as claimed in claim10 for abnormal thrombus formation, acute myocardial infarction, cardiovascular disorders, unstable angina, thromboembolism, acute vessel closure associated with thrombolytic therapy or percutaneous transluminal coronary angioplasty (PTCA), transient ischemic attacks, stroke, intermittent claudication, bypass grafting of the coronary or peripheral arteries, vessel luminal narrowing, restenosis post coronary or venous angioplasty, maintenance of vascular access patency in long-term hemodialysis patients, pathologic thrombus formation occurring in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, viral infections or cancer, or reducing an inflammatory response, fibrinolysis, or treatment of coronary heart disease, myocardial infarction, angina pectoris, vascular restenosis, for example restenosis following angioplasty like PTCA, adult respiratory distress syndrome, multi-organ failure and disseminated intravascular clotting disorder, deep vein or proximal vein thrombosis, which can occur following surgery.

European Patent Office

# PARTIAL EUROPEAN SEARCH REPORT

which under Rule 45 of the European Patent Convention shall be considered, for the purposes of subsequent proceedings, as the European search report

Application Number: EP 04 00 4904

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01/38309 A (FAN JINGMEI ;HUANG WENRONG (US); SU TING (US); WANG LINGYAN (US);) 31 May 2001 (2001-05-31)<br>* claims 9,14-21 * | 1-6,8-11 | C07K5/02<br>C07D413/12<br>C07D409/12<br>C07D295/104<br>C07D419/12<br>A61K31/381<br>A61K31/5377<br>A61K31/4965<br>A61K31/4178<br>A61K31/54 |
| X | WO 02/30880 A (MERCK PATENT GMBH ;DORSCH DIETER (DE); GLEITZ JOHANNES (DE); JURAS) 18 April 2002 (2002-04-18)<br>* page 35, line 10 - page 44, line 26 *<br>* claims 1,5-8 * | 1-6,8-11 | |
| X | WO 00/71493 A (COR THERAPEUTICS INC) 30 November 2000 (2000-11-30)<br>* claims 1-4,7-20 *<br>* tables 33-48 * | 1-6,8-11 | |
| A | WO 00/71507 A (COR THERAPEUTICS INC) 30 November 2000 (2000-11-30)<br>* the whole document * | 1-11 | |
| A | WO 00/71508 A (COR THERAPEUTICS INC) 30 November 2000 (2000-11-30)<br>* the whole document * | 1-11 | TECHNICAL FIELDS SEARCHED (Int.Cl.7)<br>C07K<br>C07D<br>A61K |
| | -/-- | | |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 4 June 2004 | Döpfer, K-P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C07)

European Patent Office

# PARTIAL EUROPEAN SEARCH REPORT

Application Number
EP 04 00 4904

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 94/12181 A (MERCK & CO INC ;EGBERTSON MELISSA S (US); TURCHI LAURA M (US); HAR) 9 June 1994 (1994-06-09)<br>* claims; examples *<br>----- | 1-11 | |
| | | | TECHNICAL FIELDS SEARCHED (Int.Cl.7) |

2

EPO FORM 1503 03.82 (P04C10)

European Patent Office

**INCOMPLETE SEARCH SHEET C**

Application Number
EP 04 00 4904

Claim(s) searched completely:
7

Claim(s) searched incompletely:
1-6,8-11

Reason for the limitation of the search:

Present claims 1-6 and 8-11 relate to an extremely large number of possible compounds or products. Support within the meaning of Article 84 EPC and/or disclosure within the meaning of Article 83 EPC is to be found, however, for only a very small proportion of the compounds/products/apparatus/methods claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds, products and methods of present claim 7 and the examples. Nevertheless, a generic search has been performed for compounds of the genral formula Het-CO-beta- alanyl-NH-Het/Ar-Het

European Patent Office

Application Number
EP 04 00 4904

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11 (all partially)

European Patent Office

# LACK OF UNITY OF INVENTION SHEET B

Application Number
EP 04 00 4904

The Search Division considers that the present European patent application does not comply with the requirements of unity of invention and relates to several inventions or groups of inventions, namely:

1. claims: Claims 1-11 (all partially)

   (a) Compounds of the general formula Het-Q-NH-CH2-CH2-CO-NH-Ar-Het, their preparation, pharmaceutical compositions comprising said compounds, and their use in the manufacture of medicaments for diseases influenced by Fxa/FVIIa. ((a) and (b) comprise the examples and have been searched completely).

1.1. claims: 1-11 (all partially)

   (b) Compounds of the general formula Het-Q-NH-CH2-CH2-CO-NH-Het-Het, their preparation, pharmaceutical compositions comprising said compounds, and their use in the manufacture of medicaments for diseases influenced by Fxa/FVIIa. ((a) and (b) comprise the examples and have been searched completely).

---

2. claims: Claims 1-11 (all partially)

   Compounds of the general formula Ar-Q-NH-CH2-CH2-CO-NH-Ar-Het, their preparation, pharmaceutical compositions comprising said compounds, and their use in the manufacture of medicaments for diseases influenced by Fxa/FVIIa.

---

3. claims: Claims 1-11 (all partially)

   Compounds of the general formula Ar-Q-NH-CH2-CH2-CO-NH-Het-Het, their preparation, pharmaceutical compositions comprising said compounds, and their use in the manufacture of medicaments for diseases influenced by FXa/FVIIa.

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO. EP 04 00 4904

This annex lists the patent family membersrelating to the patent documents cited in the above-mentioned European search report.
The members are as contained in the European Patent Office EDP file on
The European Patent Office is in no way liable for these particulars which are merely given for the purpose of information.

04-06-2004

| Patent document cited in search report | Publication date | Patent family member(s) | Publication date |
|---|---|---|---|
| WO 0138309 A | 31-05-2001 | AU 1770001 A | 04-06-2001 |
| | | CA 2392576 A1 | 31-05-2001 |
| | | EP 1235807 A1 | 04-09-2002 |
| | | JP 2003514897 T | 22-04-2003 |
| | | WO 0138309 A1 | 31-05-2001 |
| WO 0230880 A | 18-04-2002 | DE 10050723 A1 | 25-04-2002 |
| | | AU 1823302 A | 22-04-2002 |
| | | WO 0230880 A2 | 18-04-2002 |
| WO 0071493 A | 30-11-2000 | AU 5155500 A | 12-12-2000 |
| | | AU 5283900 A | 12-12-2000 |
| | | CA 2374641 A1 | 30-11-2000 |
| | | CA 2374646 A1 | 30-11-2000 |
| | | EP 1196379 A2 | 17-04-2002 |
| | | EP 1181270 A2 | 27-02-2002 |
| | | JP 2003500376 T | 07-01-2003 |
| | | JP 2003500382 T | 07-01-2003 |
| | | WO 0071493 A2 | 30-11-2000 |
| | | WO 0071507 A2 | 30-11-2000 |
| | | US 6545055 B1 | 08-04-2003 |
| WO 0071507 A | 30-11-2000 | AU 5155500 A | 12-12-2000 |
| | | AU 5283900 A | 12-12-2000 |
| | | CA 2374641 A1 | 30-11-2000 |
| | | CA 2374646 A1 | 30-11-2000 |
| | | EP 1196379 A2 | 17-04-2002 |
| | | EP 1181270 A2 | 27-02-2002 |
| | | JP 2003500376 T | 07-01-2003 |
| | | JP 2003500382 T | 07-01-2003 |
| | | WO 0071493 A2 | 30-11-2000 |
| | | WO 0071507 A2 | 30-11-2000 |
| | | US 6545055 B1 | 08-04-2003 |
| WO 0071508 A | 30-11-2000 | AU 5041300 A | 12-12-2000 |
| | | AU 5283800 A | 12-12-2000 |
| | | AU 5723500 A | 12-12-2000 |
| | | CA 2374650 A1 | 30-11-2000 |
| | | CA 2374793 A1 | 30-11-2000 |
| | | CA 2374947 A1 | 30-11-2000 |
| | | EP 1185508 A2 | 13-03-2002 |
| | | EP 1183235 A2 | 06-03-2002 |
| | | EP 1185509 A2 | 13-03-2002 |
| | | JP 2003500383 T | 07-01-2003 |
| | | JP 2003500385 T | 07-01-2003 |
| | | JP 2003500386 T | 07-01-2003 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 00 4904

This annex lists the patent family membersrelating to the patent documents cited in the above-mentioned European search report.
The members are as contained in the European Patent Office EDP file on
The European Patent Office is in no way liable for these particulars which are merely given for the purpose of information.

04-06-2004

| Patent document cited in search report | | Publication date | Patent family member(s) | | Publication date |
|---|---|---|---|---|---|
| WO 0071508 | A | | WO | 0071510 A2 | 30-11-2000 |
| | | | WO | 0071511 A2 | 30-11-2000 |
| | | | WO | 0071508 A2 | 30-11-2000 |
| | | | US | 6638980 B1 | 28-10-2003 |
| WO 9412181 | A | 09-06-1994 | AU | 675689 B2 | 13-02-1997 |
| | | | AU | 5826894 A | 22-06-1994 |
| | | | CA | 2150550 A1 | 09-06-1994 |
| | | | EP | 0673247 A1 | 27-09-1995 |
| | | | JP | 8504194 T | 07-05-1996 |
| | | | WO | 9412181 A1 | 09-06-1994 |
| | | | US | 5648368 A | 15-07-1997 |

EPO FORM P0459

For more details about this annex : see Official Journal of the European Patent Office, No. 12/82